# EUROPEAN PATENT APPLICATION

(11) **EP 3 513 808 A1**
(43) Date of publication of application: **24.07.2019**
(21) Application number: 19151519.6
(22) Date of filing: 17.06.2014
(51) Int. Cl.: A61K 39/395, A61K 31/713, A61P 19/08, C07H 21/00, C12Q 1/68, G01N 33/48, G01N 33/53

(54) **A METHOD OF INCREASING GIPCR SIGNALIZATION IN THE CELLS OF A SCOLIOTIC SUBJECT**

(30) Priority: 17.06.2013 US 201361835926 P
(62) Divisional of application: 14814185.6
(71) Applicant: CHU Sainte-Justine, Montréal, Québec H3T 1C5 (CA)
(72) Inventor: MOREAU, Alain, Montreal Québec H1A 5T5 (CA); AKOUME NDONG, Marie-Yvonne, Montreal Québec H3G 1B1 (CA)
(74) Representative: Grosset-Fournier, Chantal Catherine

(57) **Abstract**

There is provided a method of increasing GiPCR signalization in the cells of a subject in need thereof comprising administering to the subject an effective amount of an inhibitor of integrin α₅β₁ expression and/or activity, whereby GiPCR signalization is increased in the cells of the subject.

## Description

### BACKGROUND OF THE INVENTION

Idiopathic Scoliosis is a spine deformity of unknown cause generally defined as a lateral curvature greater than 10 degrees accompanied by a vertebral rotation 1. Adolescent Idiopathic Scoliosis (AIS) is one of the most frequent childhood deformities worldwide, characterized by a 3D spinal deformity with unknown cause, and represents both an immediate medical challenge and a chronic condition affecting individuals throughout their lives. It is the most common orthopedic condition requiring surgery in adolescents and affects 4% of this population. This condition is most commonly diagnosed between the ages of 9 to 13 years 2, 3, 4. The diagnosis is primarily of exclusion and is made only after ruling out other causes of spinal deformity such as vertebral malformation, neuromuscular or syndromic disorders. Traditionally, the truncal asymmetry is revealed by Adams forward bending test and measured with scoliometer during physical examination. The diagnosis can then be confirmed by radiographic observation of the curve and the angle measurement using the Cobb method.

Once diagnosed, the primary concern for physicians in managing scoliotic children is whether the curve will progress. Indeed, the curve progression is often unpredictable and is more frequently observed among girls than in boys. If untreated, the curve can progress dramatically, creating significant physical deformity and even cardiopulmonary problems. These manifestations become life threatening when the curve exceeds 70 degrees. The current treatment options to prevent or stop curve progression include bracing and surgery. In general, bracing is recommended for curves between 25 and 40 degrees, while surgery is reserved for curves greater than 45 degrees or curves that are unresponsive to bracing. Today in the United States there are approximately one million children between ages 10 and 16 with some degree of idiopathic scoliosis (IS). Approximately, 10 % of children diagnosed with idiopathic scoliosis have curve progression requiring corrective surgery. About 29,000 scoliosis surgeries are done every year in North America, resulting in significant psychological and physical morbidity. (Goldberg MS, Mayo NE, Poitras B et al. The Ste-Justine Adolescent Idiopathic Scoliosis Cohort Study. Part I: Description of the study. Spine 1994;19:1551-61; Poitras B, Mayo NE, Goldberg MS et al. The Ste-Justine Adolescent Idiopathic Scoliosis Cohort Study. Part IV: Surgical correction and back pain. Spine 1994;19:1582-8).

Currently, there is no proven method or test available to identify subjects at risk of developing IS to predict which affected individuals require treatment to prevent or stop progression of the disease so that appropriate treatment can be early provided and prevent surgical complications and cardiac and/or respiratory problems. (Weinstein SL, Dolan LA, Cheng JC et al. Adolescent idiopathic scoliosis. Lancet 2008;371:1527-37).

Therefore, the application of current treatments, such as bracing or surgical correction, is delayed until a significant deformity is detected or until a significant progression is clearly demonstrated, resulting in a delayed, less than optimal treatment and often important psychological sequels (Society SR. Morbidity & Mortality Committee annual Report 1997).

Currently, in order to detect the deformity, diagnosed children are subjected to multiple radiographs over several years, usually until they reach skeletal maturity. It is estimated that the typical patients with scoliosis will have approximately 22 radiological examinations over a 3-year period. There are potential risks in multiple radiographic examinations. For this reason also, alternative approaches that could allow performing the prognosis of idiopathic scoliosis are strongly desirable.

The major limitation in developing prognostic tests that could facilitate treatment choices for patients is the heterogeneous nature of AIS. At the clinical level, the heterogeneity of AIS is clearly illustrated by the variability of curve patterns, localisations and curve magnitude even in families with multiple affected members.

In absence of reliable AIS phenotypes, there is a need to understand better the molecular changes associated with disease onset and spinal deformity progression. Molecular definition of disease is rapidly replacing traditional pathology-based disease descriptions in part because of its utility in identifying the optimal treatment regimen for patients.

To this effect, the existence of a differential melatonin signaling dysfunction was reported among AIS patients leading to their stratification into three functional groups or biological endophenotypes (Moreau et al., 2004); (Azeddine et al., 2007); (Letellier et al., 2008) and WO2003/073102 to Moreau. More particularly, AIS patients were stratified into three functional groups (FG1, FG2 and FG3) representing distinct biological endophenotypes. According to this stratification, scoliotic patients and children more at risk of developing scoliosis are less responsive to Gi protein stimulation when compared with healthy control subjects, and the classification is based on the percentage of degree of reduction relative to the control group. The classification ranges were fixed between about 10 and 40% of reduction of response relative to control group for FG3, between about 40 and 60% for FG2 and between about 60 and 90% of reduction of response relative to control group for FG1.

More recently, using the cellular dielectric spectrometry (CDS) technique, which is a label-free method for the functional evaluation of G proteins and endogenous receptors coupled to those proteins (Verdonk et al., 2006), it was found that the cellular response following melatonin receptor stimulation by melatonin was mainly Gi-dependent in normal osteoblasts and was reduced to different extents in osteoblasts derived from AIS patients (Akoume et al., 2010). Approximately 33% of asymptomatic children diagnosed with a defective Gi protein function have developed scoliosis many years later (Akoume et al., 2010).

Early detection/prognosis of scoliosis is not only critical to successful and less invasive clinical outcomes but broadens the range of treatment options for clinicians. Indeed, improving patients' stratification and disease staging represent key steps to select AIS patients for minimally invasive surgeries before their spinal deformity is too advanced. OPN, a multifunctional cytokine, has been identified as a potentially key pathophysiologic contributor in the development of idiopathic scoliosis. Particularly, increased plasma OPN levels in patients with idiopathic scoliosis and in bipedal mice, a well-established animal model of this disease, were correlated with the disease (see WO 2008/119170 to Moreau).

It is commonly accepted that the development of scoliosis is influenced by a postural mechanism. The bipedal condition, naturally present in humans or experimentally induced in animals seems to play an important role in the manifestation of scoliotic deformities (Machida et al., 1999). Importantly, it has been reported that mice on a C57BI/6 or C3HHe background develop scoliosis closely similar to human idiopathic scoliosis when they gain bipedal posture for 40 weeks following amputation of their forelimbs and tails (Machida et al., 2006); (Oyama et al., 2006). Hence, to address the question of whether OPN contributes to the development of idiopathic scoliosis we took advantage of the availability of OPN-deficient mice on a C57BI/6 background.

### SUMMARY OF THE INVENTION

The present invention shows that OPN reduces GiPCR signaling *in vitro* mainly via α₅β₁ integrin engagement. Without being bound by such hypothesis, the mechanism by which OPN interferes with signaling of GiPCR is believed to be a depletion of functional Gi proteins necessary for these receptors through the sequestration of a part of Gi proteins by integrin β₁ subunit and the inactivation of the remaining Gi proteins following their phosphorylation by various kinases engaged in the signaling cascade of α₅β₁ integrin. Furthermore, CD44 levels and/or bioavailability to bind to OPN can modulate the inhibitory effect of OPN on GiPCR signaling. The present invention shows that depletion of CD44 exacerbates the inhibitory effect of OPN and that hyaluronic acid (HA), a known CD44 ligand, further potentiates OPN's effect in a dose-dependent manner. HA exhibits a higher binding affinity than OPN toward CD44. Therefore, HA is competing against OPN for the liaison of CD44; thereby increasing OPN's bioavailability to bind α₅β₁ integrin and to inhibit GiPCR signaling. Finally, the present invention shows that the presence of a mutation in CD44 further decreases GiPCR signaling in osteoblasts of scoliotic subjects.

Accordingly, the present invention concerns a method of increasing GiPCR signalization in the cells of a subject in need thereof comprising administering to a subject in need thereof an effective amount of: (a) an inhibitor of OPN expression and/or activity; (b) an inhibitor of integrin α₅β₁; or (c) a combination of (a) and (b), whereby GiPCR signalization is increased in the cells of a subject in need thereof.

In accordance with a related aspect, the present invention concerns a method of treating or preventing scoliosis in a subject in need thereof comprising reducing α5β1 expression or activity (e.g., binding to OPN) in the cells of the subject. In an embodiment, the method comprises administering to the subject an effective amount of an inhibitor of integrin α5β1 expression or activity.

In a specific embodiment, the inhibitor of OPN activity is an OPN antibody. In another specific embodiment, the inhibitor of OPN expression is a siRNA specific to OPN. In another specific embodiment, the inhibitor of α₅β₁ is (a) an antibody that binds specifically to integrin subunit α₅ ; (b) an antibody that binds specifically to integrin subunits β₁; or (c) a combination of (a) and (b). In a specific embodiment, the inhibitor of α₅β₁ is a siRNA specific to α₅ or β₁.

In accordance with another aspect of the present invention, there is provided a method of selecting an agent as a potential candidate for the reduction or prevention of GiPCR signaling inhibition induced by OPN comprising contacting a candidate agent with a cell expressing (i) OPN; (ii) integrin α₅β₁; and/or (iii) CD44/sCD44, wherein (a) when OPN expression or activity is decreased in the presence of the candidate agent as compared to in the absence thereof; (b) when integrin α₅β₁ expression or activity (e.g., binding to OPN) is decreased in the presence of the candidate agent as compared to in the absence thereof, (c) when sCD44 and/or CD44 expression (level) or binding to OPN is increased in the presence of the candidate agent as compared to in the absence thereof; or (d) any combination of at least two of (a), (b) and (c), the candidate agent is selected.

In accordance with another aspect of the present invention, there is provided a method of selecting an agent as a potential candidate for the reduction or prevention of GiPCR signalization comprising contacting a candidate agent with a cell expressing (a) OPN; and/or (b) integrin α₅β₁, wherein (a) when OPN expression or activity; and/or (b) when integrin α_{5/}β₁ is increased in the presence of the candidate agent as compared to in the absence thereof, the candidate agent is selected.

In accordance with another aspect of the present invention, there is provided (i) an inhibitor of OPN expression or activity; ii) an inhibitor of integrin α₅β₁ expression or activity; iii) sCD44 or a stimulator of sCD44/CD44 expression; or iv) any combination of at least two of (i) to (iii) for use in increasing GiPCR signalization in cells of a subject in need thereof.

In accordance with another aspect of the present invention, there is provided a use of (i) an inhibitor of OPN expression or activity; ii) an inhibitor of integrin α₅β₁ expression or activity; iii) sCD44 or a stimulator of sCD44/CD44 expression; or iv) any combination of at least two of (i) to (iii), for the preparation of a medicament for increasing GiPCR signalization in cells of a subject in need thereof.

In accordance with another aspect of the present invention, there is provided a composition for increasing GiPCR signalization in the cells of a subject in need thereof comprising (i) an inhibitor of OPN expression or activity; (ii) an inhibitor of α₅β₁ expression or activity; (iii) sCD44 or a stimulator of sCD44/CD44 expression; or (iv) any combination of at least two of (i) to (iii).

In a specific embodiment, the subject in need thereof is a subject diagnosed with a scoliosis or at risk of developing a scoliosis.

In accordance with another aspect of the present invention, there is provided a method of increasing GiPCR signalization in the cells of a subject in need thereof comprising administering to the subject an effective amount of an inhibitor of integrin α₅β₁ expression and/or activity, whereby GiPCR signalization is increased in the cells of the subject.

In a specific embodiment, the method further comprises administering to the subject an effective amount of an inhibitor of OPN expression and/ or activity. In another specific embodiment, the inhibitor of OPN activity is an OPN antibody. In another specific embodiment, the inhibitor of OPN expression is a siRNA specific to OPN. In another specific embodiment, the inhibitor of α₅β₁ activity is (a) an antibody that binds specifically to integrin subunit α₅; (b) an antibody that binds specifically to integrin subunit β₁; (c) an antibody that binds specifically to integrin subunits α₅β₁; (d) a molecule that specifically blocks the binding of OPN to α₅β₁ integrin; or (e) any combination of at least two of (a) to (d). In another specific embodiment, said molecule that specifically blocks the binding of OPN to α₅β₁ integrin is a RGD peptide or derivative thereof. In another specific embodiment, said molecule that specifically blocks the binding of OPN to α₅β₁ integrin is a peptide fragment of OPN comprising a RGD motif. In another specific embodiment, the peptide fragment of OPN comprises the amino acid sequence GRGDSWYGLRS (SEQ ID NO: 18). In another specific embodiment, the inhibitor of α₅β₁ expression is a siRNA specific to α₅ or β₁. In a specific embodiment, the method further comprises administering to the subject i) an effective amount of sCD44 or a fragment thereof which specifically binds to OPN; or ii) a stimulator of sCD44 and/or CD44 expression.

In accordance with another aspect of the present invention, there is provided a use of an inhibitor of integrin α₅β₁ expression and/or activity in the preparation of a medicament for inhibiting GiPCR signalization in cells of a subject in need thereof.

In accordance with another aspect of the present invention, there is provided a use of an inhibitor of integrin α₅β₁ expression and/or activity for inhibiting GiPCR signalization in cells of a subject in need thereof.

In accordance with another aspect of the present invention, there is provided an inhibitor of integrin α₅β₁ expression and/or activity for use in inhibiting GiPCR signalization in cells of a subject in need thereof.

In a specific embodiment of the use or the inhibitor, said inhibitor of α₅β₁ activity is (a) an antibody that binds specifically to integrin subunit α₅; (b) an antibody that binds specifically to integrin subunit β₁; (c) an antibody that binds specifically to integrin subunits α₅β₁; (d) a molecule that specifically blocks the binding of OPN to α₅β₁ integrin; or (e) any combination of at least two of (a) to (d). In another specific embodiment of the use or the inhibitor, the molecule that specifically blocks the binding of OPN to α₅β₁ integrin is a RGD peptide or derivative thereof. In another specific embodiment, the molecule that specifically blocks the binding of OPN to α₅β₁ integrin is a peptide fragment of OPN comprising a RGD motif. In another specific embodiment, the peptide fragment of OPN comprises the amino acid sequence GRGDSWYGLRS (SEQ ID NO: 18). In another specific embodiment, the inhibitor of integrin α₅β₁ expression is a siRNA specific to α₅ or β₁.

In accordance with another aspect of the present invention, there is provided a composition comprising the inhibitor of the present invention, and a pharmaceutical carrier.

In another specific embodiment, the composition is for increasing GiPCR signaling in cells of a subject in need thereof.

In specific embodiments of the method, the use, the inhibitor, or the composition of the present invention, the subject is a subject diagnosed with a scoliosis or at risk of developing a scoliosis. In another specific embodiment, the scoliosis is an idiopathic scoliosis. In another specific embodiment, the scoliosis is adolescent idiopathic scoliosis (AIS).

In accordance with another aspect of the present invention, there is provided a method of determining the risk of developing a scoliosis in a subject comprising: (i) providing a cell sample isolated from the subject; (ii) detecting, in the cell sample from the subject, the presence of at least one copy of a CD44 risk allele which introduces a mutation at amino acid position 230 of CD44 or a marker in linkage disequilibrium therewith; and (iii) determining that the subject is at risk of developing a scoliosis when at least one copy of the risk allele or marker in linkage disequilibrium therewith is detected in the cell sample from the subject..

In a specific embodiment, the risk allele comprises SNP rs1467558 and the mutation changes an isoleucine at position 230 of CD44 to a threonine.

In accordance with another aspect of the present invention, there is provided a method of stratifying a subject having scoliosis (*e.g*., idiopathic scoliosis such as adolescent idiopathic scoliosis (AIS)) comprising: (i) providing a cell sample isolated from the subject; (ii) detecting, in the cell sample from the subject, the presence of at least one copy of a CD44 risk allele which introduces a mutation at amino acid position 230 of CD44 or a marker in linkage disequilibrium therewith; and (iii) (a) stratifying the subject into a first AIS subclass when at least one copy of the CD44 risk allele or marker in linkage disequilibrium therewith is detected in the cell sample from the subject; or (b) stratifying the subject into a second AIS subclass when the CD44 risk allele is not detected in the cell sample from the subject.

In a specific embodiment, the mutation changes an isoleucine at position 230 of CD44 to a threonine. In another specific embodiment, the at least one copy of the CD44 risk allele consists of two copies of the CD44 risk allele and the subject is homozygote for the mutation. In another specific embodiment, the sample is a nucleic acid sample. In another specific embodiment, said sample is a protein sample.

In accordance with another aspect of the present invention, there is provided a kit for performing the method as defined in any one of claims 24 to 30 comprising: (i) a nucleic acid probe or primer for detecting a CD44 risk allele comprising a mutation in a codon encoding amino acid 230 of CD44; and/or (ii) a protein ligand which specifically detects a mutation at amino acid 230 of CD44.

In a specific embodiment, the nucleic acid probe or primer comprises a nucleic acid sequence which specifically hybridizes to a nucleic acid encoding a threonine at amino acid position 230 of CD44. In another specific embodiment, the protein ligand is an antibody specific for a CD44 protein comprising a threonine at amino acid position 230.

Other objects, advantages and features of the present invention will become more apparent upon reading of the following non-restrictive description of specific embodiments thereof, given by way of example only with reference to the accompanying drawings.

### BRIEF DESCRIPTION OF THE DRAWINGS

In the appended drawings:

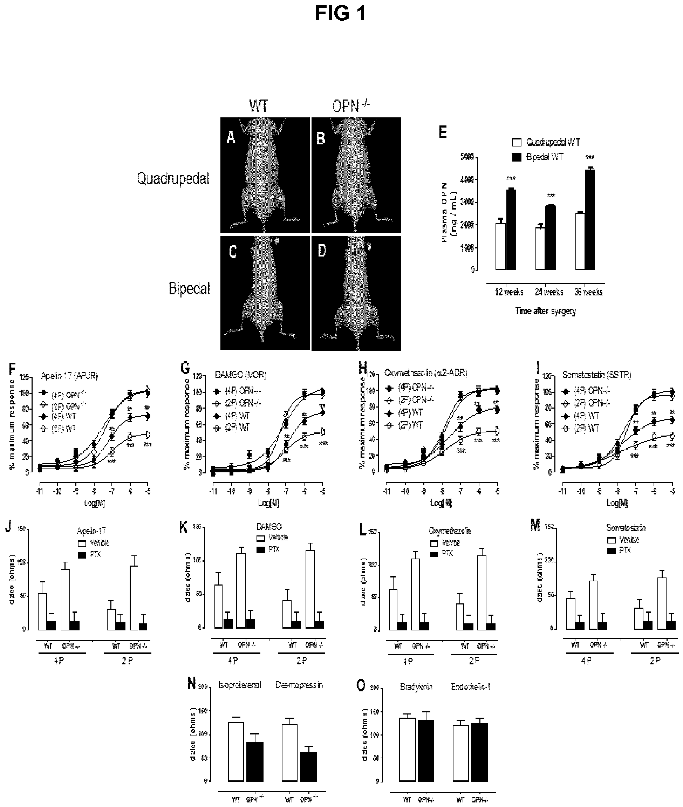

**Figure 1** **shows that genetic deletion of OPN protects bipedal C57BL6 from scoliosis by improving Gi protein-mediated receptor signal transduction.** (A-D) Female C57BI/6 wild-type (WT) and OPN knockout (OPN^{-/-}) mice were amputated from forelimbs and tails at 1 month of age and subjected to bipedal ambulation for 36 weeks to induce scoliosis. X-ray radiographs of the spine as taken at the end of the experiment period. OPN^{-/-} mice did not develop scoliosis in the quadrupedal or even bipedal while WT developed it in the bipedal mice. (E) Plasma OPN was detected in C57BI/6 (WT) quadrupedal and bipedal mice, and the bipedal mice showed higher plasma OPN than quadrupedal WT mice. (F-I) GiPCR signaling was checked in osteoblasts from WT and OPN-/- mice using DAMGO, somatostatin, oxymethazoline and apelin. The response was greater in OPN^{-/-} than WT osteoblasts. On the other hand, bipedal WT mice showed lower response than the quadrupedal WT. (J-M) The pre-treatment of osteoblasts from WT and OPN^{-/-} mice with PTX blocked the Gi coupling for their cognate receptors in these cells, response to each of the previous agonists. This is indicating that these compounds evoked typical CDS response profiles of GiPCR in WT and OPN^{-/-} osteoblasts. (N-O) Isoproterenol and desmopressin were used to activate Gs while bradykinin and endothelin-1 were used to activate Gq through their cognate receptors. Osteoblasts from OPN^{-/-} mice were less responsive to Gs stimulation than those from WT mice, whereas no difference in the Gq stimulation elicited in WT and OPN^{-/-} osteoblasts.

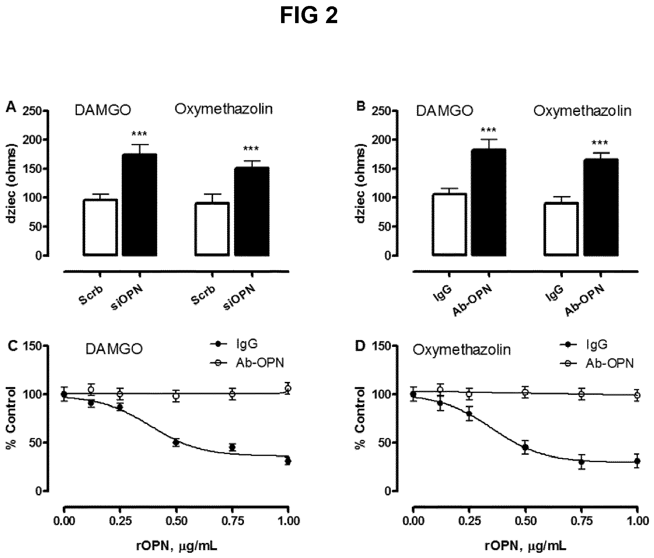

**Figure 2** **shows that extracellular OPN causes Gi protein-coupled receptor signaling dysfunction.** (A) OPN was knockdown by siRNA in MC3T3-E1 osteoblastic cell line and the capacity of GiPCR ligands to induce cell signaling as measured by CDS. Cellular response to DAMGO and oxymethazoline was significantly greater in cells depleted of OPN. (B) OPN, blocked by OPN specific antibody, in MC3T3-E1 osteoblastic cell line gave the same results as in (A). (C-D) MC3T3-E1 osteoblastic cells were treated with exogenous recombinant OPN (rOPN) prior to DAMGO and oxymethazoline stimulation. In each case, rOPN caused a decrease in the integrated response in a concentration-dependent manner, which was prevented by OPN antibody.

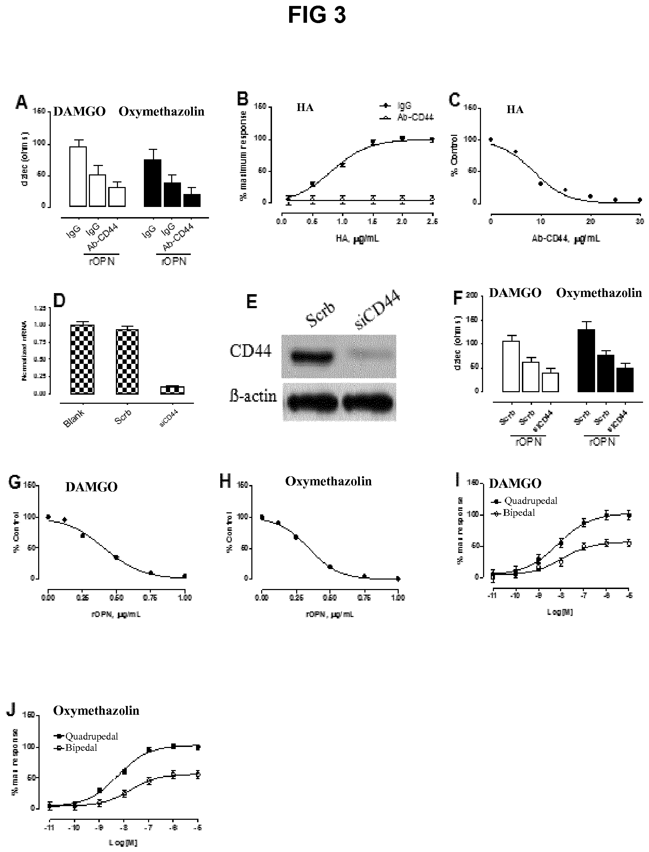

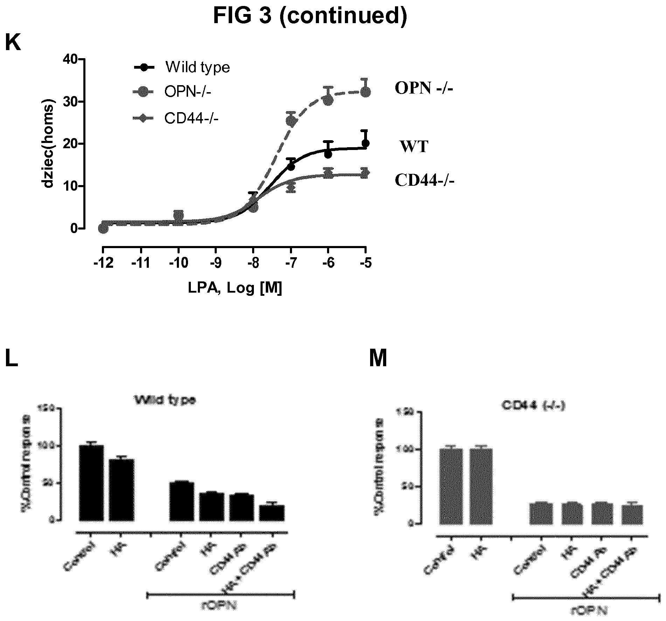

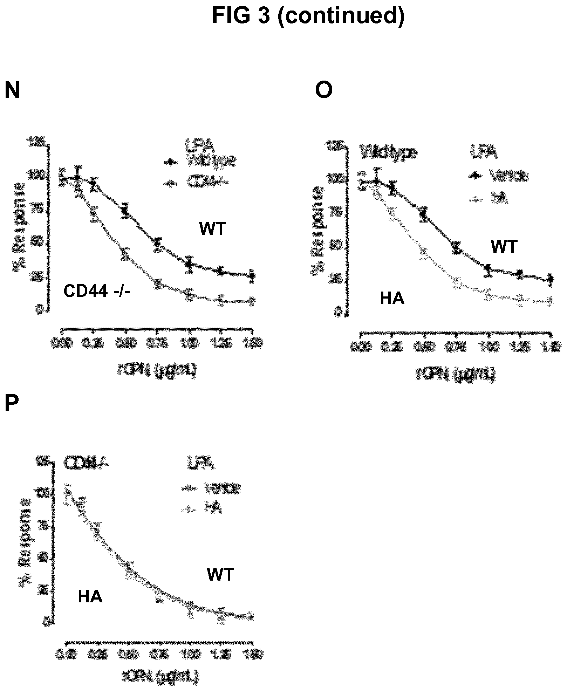

**Figure 3** **shows that CD44 is not involved in the inhibition of GiPCR signaling caused by extracellular OPN but can modulate OPN's effect.** (A) The blockage of CD44 by CD44 specific antibody in MC3T3-E1 osteoblastic cell line and cells treated with rOPN 0.5µg/ml further aggravated GiPCR signaling defect induced by OPN and did not reduce the GiPCR signaling defect cause by OPN. DAMGO and oxymethazoline were used as agonist for GiPCR. (B-C) To validate the activity of the anti-CD44 antibody, the cells pre-treated with IgG control or CD44 antibody were stimulated with increasing concentrations of hyaluronic acid (HA), a high affinity ligand of CD44. The integrated response induced by HA was completely abrogated by pre-treatment with the CD44 antibody. Moreover, the effect of the CD44 antibody on response to HA stimulation was concentration-dependent. (D-E) CD44 was knockdown in MC3T3-E1 osteoblastic cell line by siRNA. The efficiency of siRNA transfection and inhibition of CD44 expression was demonstrated by qPCR (D) and Western blot analysis (E). (F) The knockdown of CD44 led to similar results as (A) and CD44 knockdown did not reduce the GiPCR signaling defect cause by OPN but further aggravated the defect. (G-H) rOPN treatment caused a concentration-dependent decrease in response to both DAMGO and oxymethazoline in osteoblasts from bipedal CD44 knockout (CD44-/-) mice. (I-J) Osteoblasts cells form bipedal (CD44-/-) mice were less responsive to DAMG or oxymethazoline when compared with osteoblasts from quadrupedal (CD44-/-) mice. (K-P) CD44 inhibition and HA intensify the effect of OPN on GiPCR signaling in a dose-dependent manner. (K) In response to LPA stimulation on GiPCR signaling, CD44-/- osteoblasts are less responsive and OPN-/- osteoblasts are more responsive compared to the wild type cells in a dose dependent manner. (L) Treatment with HA, the natural agonist for CD44 receptor, exacerbated the GiPCR signaling defects in wild type osteoblasts, compared to the control. Inhibition of CD44 using HA or an antibody (CD44 fab) in presence of recombinant OPN (rOPN) also exacerbate the signaling defect. The response is most reduced when CD44 is inhibited in both ways (HA+CD44 Fab). (M) The same experiment using CD44-/- osteoblast does not show any sensitivity to HA or anti-CD44 antibody. (N, O and P). Removing the effect of CD44 by using CD44-/- osteoblasts or inhibiting the CD44 receptor by treating the wild type cells with HA has the same effect on GiPCR signaling response in the presence of OPN.

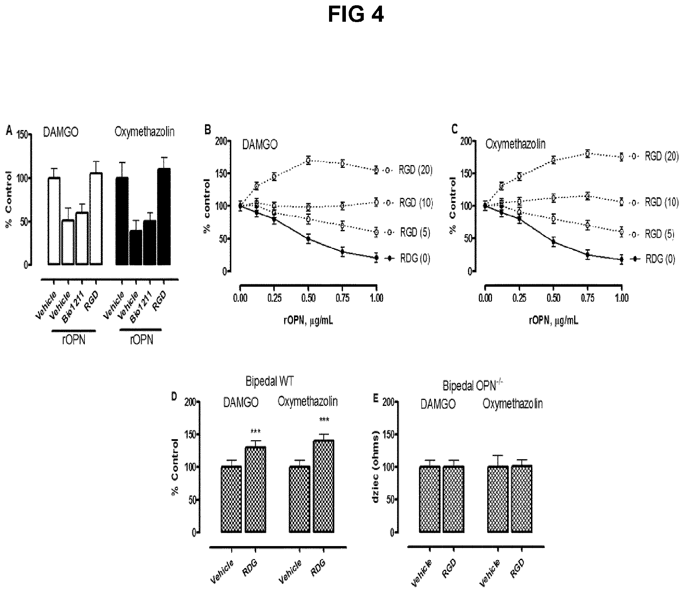

**Figure 4** **shows that RGD-dependent integrins mediate the inhibitory effect of OPN on GiPCR signaling.** (A- B & C) OPN binds to integrins through RGD motif and this was clear when we inhibit the SWYGLR binding motif by bio1211, which selectively inhibits α₄β₁ integrin, the only SVVYGLR-containing integrin present in osteoblasts. Bio1211did not affect the OPN signaling while coincubation of cells with rOPN and RDG peptide completely prevented the inhibitory effect of rOPN on response to DAMGO and oxymethazoline. (D-E) Incubation of osteoblasts from WT mice with high concentrations of RGD demonstrated significant increase of response to DAMGO or oxymethazoline while no change in osteoblasts from OPN^{-/-} mice was observed.

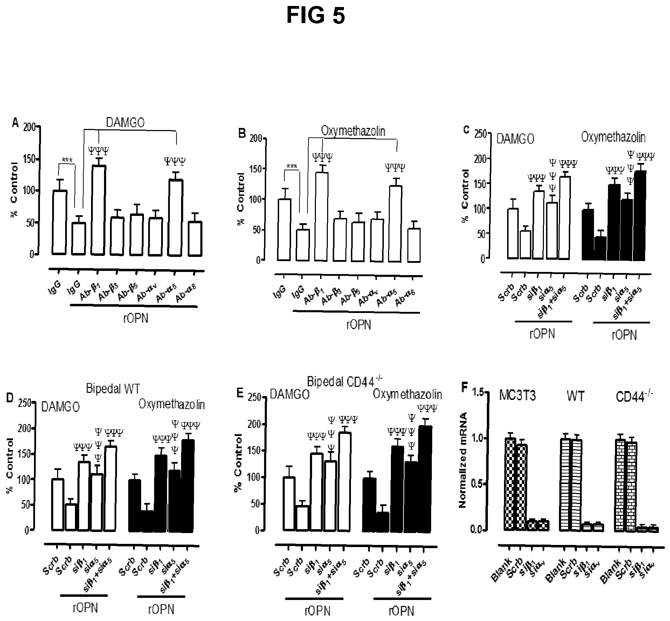

**Figure 5** **shows the identification of integrins involved in the inhibition of GiPCR signaling by OPN.** (A, B) Blockage of the different integrins using specific antibodies and (C) knockdown of these integrins in MC3T3-E1 osteoblastic cells. Only inhibition of α₅ and β₁ integrins in cells reversed the inhibitory action of OPN on GiPCR-signaling. (D-E-F Knockdown of α₅ and β₁ integrins in C57BI/6 bipedal WT and CD44^{-/-} mice reversed the inhibitory effect of rOPN on response to both DAMGO and oxymethazoline.

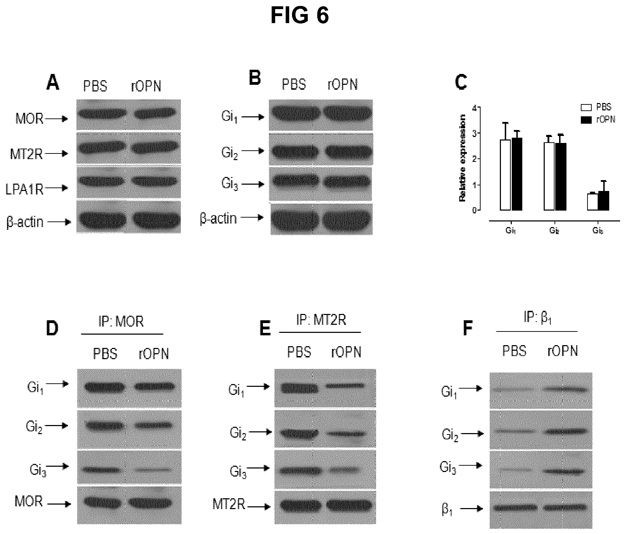

**Figure 6** **shows that OPN reduces the availability of Gi proteins for their cognate receptors. (A, B,** C) MC3T3-E1 osteoblastic cells were treated with PBS or rOPN. Cell lysates were analyzed by Western blot for the expression of three different GPCRs: mu-opioid (MO), lysophosphatidic acid type 1 (LPA1) and melatonin type 2 (MT2) receptors (A) while Gi₁, Gi₂ and Gi₃ proteins isoforms expression was detected by Western blot (B) and qPCR (C). There was no significant effect of rOPN treatment on the quantity of Gi proteins or mRNAs (B, C) or GiPCR proteins (A). (D-E-F) Cells lysates were immunoprecipitated with antibodies against MO, MT2 or β1 integrin receptors, and the presence of Gi proteins in each precipitate was examined by Western blot using antibodies specific for Gi₁, Gi₂ or Gi₃ isoform. The amount of each of these Gi protein isoforms was elevated in the β1 integrin precipitates following rOPN treatment.

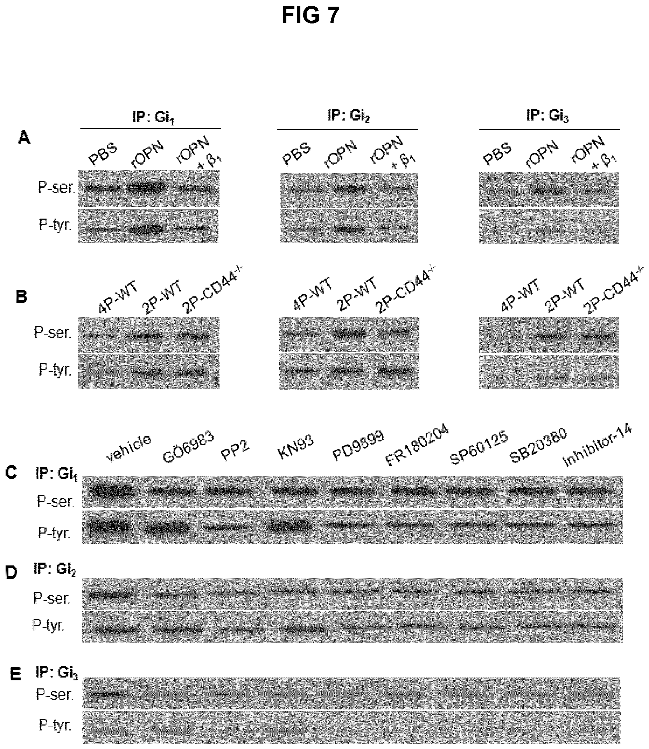

**Figure 7** **shows that OPN enhances the phosphorylation of Gi proteins.** (A) MC3T3-E1 cells were treated with rOPN or pre-treated with antibody against α₅β₁ integrin prior to rOPN treatment and then immunoprecipitation (IP) was performed on cell lysates using antibodies against Gi₁, Gi₂ or Gi₃ protein isoforms, and the presence of phosphorylation revealed by Western blot by anti-phospho serine/threonine or anti-tyrosine antibodies. (B) IP of osteoblasts cell lysates from scoliotic bipedal WT or CD44^{-/-} mice and quadrupedal control mice by antibodies against Gi₁, Gi₂ and Gi₃ proteins followed by Western blot using antibodies against anti-phospho-serine/threonine or an anti-phospho-tyrosine. (C-E) Cell extracts prepared from MC3T3-E1 cells treated with rOPN in the presence or absence of kinases inhibitors followed by IP with antibodies against Gi₁, Gi₂ or Gi₃ protein isoforms, and Western blot using anti-phospho serine/threonine or anti-tyrosine antibodies.

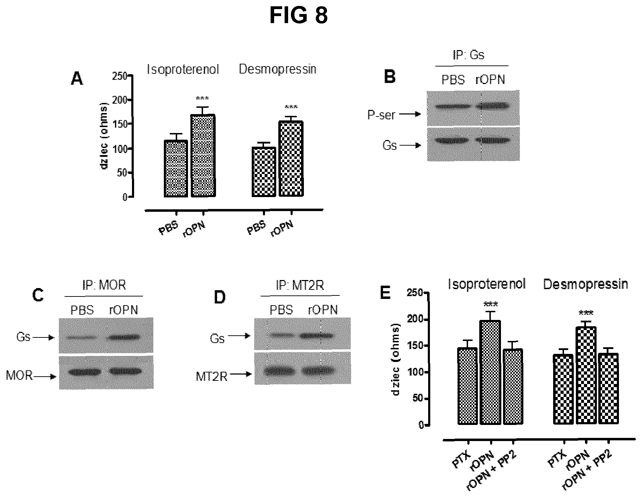

**Figure 8** **shows the effect of OPN on GsPCR proteins.** (A) MC3T3-E1 cells were treated with rOPN or PBS (A) stimulated by isoproterenol or desmopressin or (B) IP by antibodies against Gs followed by Western blot with antibodies against anti-phospho-serine/threonine or (C-D) IP by antibodies against MOR or MT2R followed by Western blot with antibodies against Gs. (E) MC3T3-E1 cells were treated with PTX to mimic disruption action of rOPN and compared to cells treated with rOPN alone or in combination with Src inhibitor (PP2) to prevent tyrosine phosphorylation.

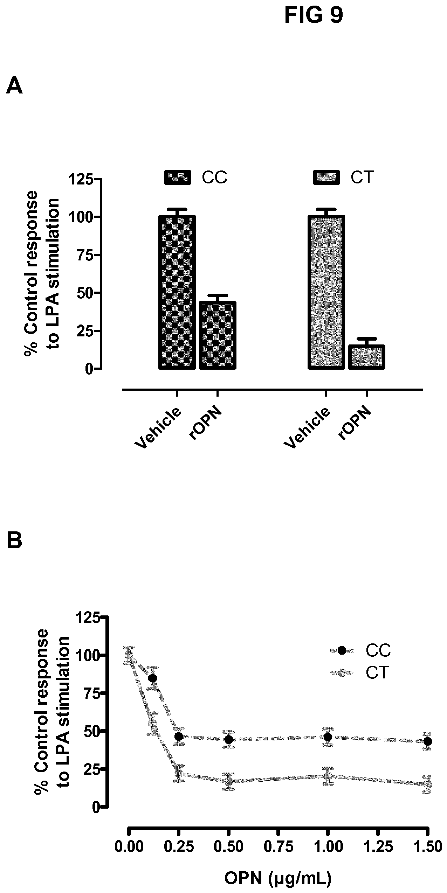

**Figure 9** **shows that the CT SNP in CD44 exacerbates OPN's inhibitory effect on GiPCR signaling.** (A) Osteoblasts from AIS subjects carrying the CT SNP and from healthy subjects were treated with OPN and the effect on GiPCR signaling was assessed following stimulation with LPA. (B) Same as in (A) except that the cell samples were treated with increasing concentrations of OPN. The presence of the CT SNP reduces by about 50% GiPCR signaling.

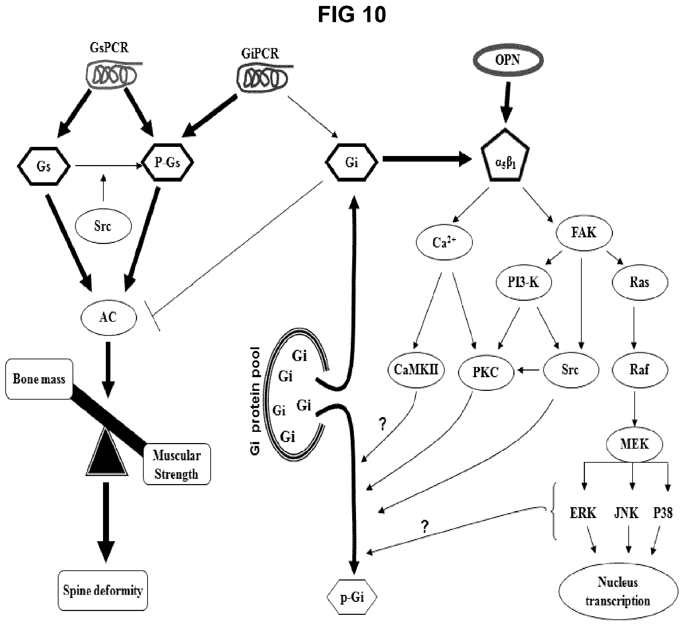

**Figure 10** **shows a schematic representation of the proposed mechanism by which OPN reduces GiPCR signaling and contributes to the development of spinal deformity.** Following OPN binding, α₅β₁ integrin recruits a part of Gi proteins from the common pool, then promotes different signaling pathways leading to the activation of various kinases, which directly or indirectly phosphorylate a part of the remaining Gi protein in the common pool, leading to reduced GiPCR signaling.

### DESCRIPTION OF ILLUSTRATIVE EMBODIMENTS

As used herein the terms "risk of developing scoliosis" refer to a genetic or metabolic predisposition of a subject to develop a scoliosis (i.e. spinal deformity) and/or to develop a more severe scoliosis at a future time (i.e. curve progression). For instance, an increase of the Cobb's angle of a subject (e.g. from 40° to 50°, or from 18° to 25°) is a "development" of scoliosis.

In an embodiment, the above-mentioned subject is a likely candidate for developing a scoliosis, such as idiopathic scoliosis (*e.g*., Infantile Idiopathic Scoliosis, Juvenile Idiopathic Scoliosis or Adolescent Idiopathic Scoliosis (AIS)). As used herein the terms "likely candidate for developing scoliosis" include subjects (*e.g*., children) of which at least one parent has a scoliosis (*e.g*., adolescent idiopathic scoliosis). Among other factors, age (adolescence), gender and other family antecedent are factors that are known to contribute to the risk of developing a scoliosis and are used to a certain degree to assess the risk of developing a scoliosis. In certain subjects, scoliosis develops rapidly over a short period of time to the point of requiring a corrective surgery (often when the deformity reaches a Cobb's angle ≥ 50°). Current courses of action available from the moment a scoliosis such as AIS is diagnosed (when scoliosis is apparent) include observation (when Cobb's angle is around 10-25°), orthopedic devices (when Cobb's angle is around 25-30°), and surgery (over 45°). A more reliable determination of the risk of progression could enable to 1) select an appropriate diet to remove certain food products identified as contributors to scoliosis; 2) select the best therapeutic agent; and/or 3) select the least invasive available treatment such as postural exercises, orthopedic device, or less invasive surgeries or surgeries without fusions (a surgery that does not fuse vertebra and preserves column mobility). The present invention encompasses selecting the most efficient and least invasive known preventive actions or treatments in view of the determined risk of developing scoliosis.

As used herein the term "subject" is meant to refer to any mammal including human, mouse, rat, dog, chicken, cat, pig, monkey, horse, etc. In a particular embodiment, it refers to a human.

A "subject in need thereof" or a "patient" in the context of the present invention is intended to include any subject that will benefit or that is likely to benefit from the modulation (increase or decrease) of OPN's effect on GiPCR signal transduction. In an embodiment, the subject in need thereof is a subject that will benefit or is likely to benefit from an increase in GiPCR signal transduction and thus from (a) an antagonist (inhibitor) of OPN expression and/or activity; (b) an antagonist (inhibitor) of α₅ (e.g., Gene ID 3678, NP_002196) and/or β₁ expression and/or activity or (c) an increase in sCD44 or CD44 expression. In a specific embodiment, the subject in need thereof is a subject having high levels of OPN as compared to a control subject. In a specific embodiment, the high level of OPN corresponds to a level ≥ to about 600 ng/ml, ≥ to about 700 ng/ml, ≥ to about 800 ng/ml, ≥ to about 900 ng/ml or ≥ to about 1000 ng/ml of OPN as measured in a blood sample from the subject. In an embodiment, a subject in need thereof is a subject diagnosed with a scoliosis (*e.g*., AIS). In another embodiment, the subject in need thereof is a subject is likely to develop a scoliosis (*e.g*., AIS).

As used herein the terminology "biological sample" refers to any solid or liquid sample isolated from a living being. In a particular embodiment, it refers to any solid or liquid sample isolated from a human. Without being so limited it includes a biopsy material, blood, tears, saliva, maternal milk, synovial fluid, urine, ear fluid, amniotic fluid and cerebrospinal fluid. In a specific embodiment it refers to a blood sample.

As used herein the terminology "blood sample" is meant to refer to blood, plasma or serum.

As used herein the terminology "control sample" is meant to refer to a sample that does not come from a subject known to have scoliosis or known to be a likely candidate for developing a scoliosis. In methods for determining the risk of developing scoliosis in a subject that is pre-diagnosed with scoliosis, the sample may however also come from the subject under scrutiny at an earlier stage of the disease or disorder. In a specific embodiment, the control sample can come from another subject diagnosed with scoliosis and belonging to the same functional group (e.g., FG1, FG2 or FG3) at an earlier (or later stage) of the disease or disorder.

As used herein the terminology "**control**" is meant to encompass "control sample". In certain embodiments, the term "**control**" also refers to the average or median value obtained following determination of GiPCR signalization in a plurality of samples (*e.g*., samples obtained from several subjects not known to have scoliosis and not known to be a likely candidate for developing scoliosis).

As used herein the term "treating" or "treatment" in reference to scoliosis is meant to refer to at least one of a reduction of Cobb's angle in a preexisting spinal deformity, improvement of column mobility, preservation/maintenance of column mobility, improvement of equilibrium and balance in a specific plan; maintenance/preservation of equilibrium and balance in a specific plan; improvement of functionality in a specific plan, preservation/maintenance of functionality in a specific plan, cosmetic improvement, and combinations of any of the above.

As used herein the term "preventing" or "prevention" in reference to scoliosis is meant to refer to a at least one of a reduction in the progression of a Cobb's angle in a patient having a scoliosis or in an asymptomatic patient, a complete prevention of apparition of a spinal deformity, including changes affecting the rib cage and pelvis in 3D, and a combination of any of the above.

The terms "suppressor" "inhibitor" and "antagonist" are well known in the art and are used herein interchangeably. They include intracellular as well as extracellular inhibitors.

The terms "inhibitor of OPN" or "OPN antagonist" include any compound able to negatively affect (i.e., reduce totally or partially) the expression and/or activity of OPN (e.g., Gene ID 6696, NP_001035147.1 (SEQ ID NO: 25) and NM_001040058 (SEQ ID NO:26)) in cells. In a specific embodiment, the activity of OPN in cells is a reduction in GiPCR signaling. Similarly, the terms "inhibitor of α₅β₁", "inhibitor of α₅" or "inhibitor of β₁" and the like include any compound able to negatively affect the expression and/or activity of α₅ (e.g., Gene ID 3678, NP_002196 (SEQ ID NO:23) and NM_002205.2 (SEQ ID NO: 24)) and/or β₁ (Gene ID 3688, NP_002202 (SEQ ID NO: 21) and NM_002211.3 (SEQ ID NO:22)) in cells. In a particular embodiment, the "activity" of α₅ and/or β₁ in cells is the transduction of the signal leading to the OPN-dependent inhibition of GiPCR signaling.

The terms "inhibitor of OPN expression" or "inhibitor of α₅β₁ expression" include any compound able to negatively affect OPN's, α₅'s and/or β₁'s expression (*i.e*., at the transcriptional and/or translational level) i.e. the level of OPN/ α₅ or β₁ mRNA and/or protein or the stability of the protein. Without being so limited, such inhibitors include RNA interference agents (siRNA, shRNA, miRNA), antisense molecules, and ribozymes. Such RNA interference agents are design to specifically hybridize with their target nucleic acid under suitable conditions and are thus substantially complementary their target nucleic acid.

The terms "inhibitor of OPN activity" or "inhibitor of α₅β₁ activity" refers to any molecules that is able to reduce or block the effect of OPN or α₅β₁ on Gi-mediated signaling. These molecules increase GiPCR signaling in cells by blocking/reducing totally or partially the inhibitory effect induced by OPN and/or α₅β₁ activity. Non-limiting examples of inhibitors of OPN's activity include proteins (*e.g*., dominant negative, inactive variants), peptides, small molecules, anti-OPN antibodies (neutralizing antibodies), antibody fragments, inactive fragments of α₅ and/or β₁ integrins etc. Non-limiting examples of inhibitors of α₅β₁ activity include proteins (*e.g*., dominant negative, inactive variants), peptides (RGD peptides or RGD peptide-derivatives), small molecules, anti α₅ and/or β₁ antibodies (*e.g*., neutralizing antibodies such as Volociximab™ M200), antibody fragments, etc. In an embodiment, the RGD peptide is a peptide fragment of OPN comprising a RGD motif comprising the amino acid sequence GRGDSWYGLRS corresponding to amino acid 158 to 169 of OPN (SEQ ID NO:18). In an embodiment, the OPN fragment comprising the RGD motif comprises amino acids 158 to 162, 158 to 165, 158 to 167, 158 to 170, 158 to 175, 158 to 180, 158 to 185, 158 to 190, 158 to 195, or 158 to 200 of OPN (e.g., SEQ ID NO: 25). In an embodiment, peptide fragment of OPN comprising a RGD motif comprises amino acids 158 to 161, 156 to 161, 154 to 161, 152 to 162, 150 to 162, 148 to 162, 146 to 162, 144 to 162, 140 to 162, 159 to 163, 159 to 164, 159 to 162, 159 to 166, 159 to 167, or 159 to 169 of OPN (e.g., SEQ ID NO: 25).

In an embodiment, the "inhibitor of OPN's activity" is a neutralizing antibody directed against (or specifically binding to) a human OPN polypeptide which inhibits its binding to α₅β₁ (i.e, binding to α₅ and/or β₁ integrin) In an embodiment, the "inhibitor of α₅β₁ activity" is a neutralizing antibody directed against (or specifically binding to) a human α₅β₁ (α₅ and/or β₁) polypeptide which inhibits the binding of OPN to α₅β₁ (i.e, binding to α₅ and/or β1 integrin). In an embodiment, the antibody binds to the RGD domain of OPN. In an embodiment, the antibody is directed against amino acids 159 to 162, 158 to 162, 158 to 165, 158 to 167, 158 to 170, 158 to 175, 158 to 180, 158 to 185, 158 to 190, 158 to 195, or 158 to 200 of OPN (e.g., SEQ ID NO: 25). In an embodiment, the antibody is directed against amino acids 158 to 161, 156 to 161, 154 to 161, 152 to 162, 150 to 162, 148 to 162, 146 to 162, 144 to 162, 140 to 162, 159 to 163, 159 to 164, 159 to 162, 159 to 166, 159 to 167, or 159 to 169 of OPN (e.g., SEQ ID NO: 25).

The term "stimulator" or "enhancer" of sCD44/CD44 expression (e.g., Gene ID 960, NM_000610 (SEQ ID NO: 19), NP_000601.3, (SEQ ID NO: 20)) refers to an agent able to increase the level or expression of CD44, an agent able to increase CD44 secretion. In an embodiment, the stimulator of sCD44/CD44 is an agent able to increase CD44 affinity toward OPN. Without being so limited, the agent can be a protein, a peptide, a small molecule or a nucleotide.

One possible way of decreasing OPN's effect on GiPCR-signaling is to reduce OPN's interaction with the α₅β₁ integrin by, for example, increasing or favoring OPN's binding to CD44. Hyaluronic acid (HA) is known to bind to CD44. As shown herein HA potentiates the effect of OPN, probably by increasing OPN's bioavailability to α₅β₁ (e.g., by sequestering CD44 which is no longer available for binding to OPN). Accordingly, subjects which will benefit from OPN inhibition and increase in GiPCR-signalization should not take HA supplements and/or should decrease their food intake of HA by for example avoiding or decreasing his/her consumption of foods that are particularly rich in HA or that are known to increase HA synthesis. Non-limiting examples of such food include meat and meat organs (e.g., veal, lamb, beef and gizzards, livers, hearts and kidneys), fish, poultry (including meat fish and poultry broths), soy (including soy milk), root vegetables containing starch including potatoes and sweet potatoes. Sweet potatoes particularly rich in HA include satoimo and imoji, two Japanese sweet potatoes.

The present invention also relates to methods for the determination of the level of expression (i.e. transcript (RNA) or translation product (protein)) of OPN, α₅β₁ integrin and/or CD44. As used herein the term α₅β₁ is used herein to refer to α₅β₁ integrin. In specific embodiments, it also includes a method that comprises the determination of the level of expression of one or more other scoliosis markers (see for example WO 2008/119170 to Moreau). The present invention therefore encompasses any known method for such determination including ELISA (Enzyme Linked Immunosorbent Assay), RIA (Radioimmunoassay), immunofluorescence, real time PCR and competitive PCR, Northern blots, nuclease protection, plaque hybridization and slot blots.

The present invention also concerns isolated nucleic acid molecules including probes and primers to detect OPN, α₅β₁ and sCD44/CD44 (and optionally other scoliosis markers). In specific embodiments, the isolated nucleic acid molecules have no more than 300, or no more than 200, or no more than 100, or no more than 90, or no more than 80, or no more than 70, or no more than 60, or no more than 50, or no more than 40 or no more than 30 nucleotides. In specific embodiments, the isolated nucleic acid molecules have at least 17, or at least 18, or at least 19, or at least 20, or at least 30, or at least 40 nucleotides. In other specific embodiments, the isolated nucleic acid molecules have at least 20 and no more than 300 nucleotides. In other specific embodiments, the isolated nucleic acid molecules have at least 20 and no more than 200 nucleotides. In other specific embodiments, the isolated nucleic acid molecules have at least 20 and no more than 100 nucleotides. In other specific embodiments, the isolated nucleic acid molecules have at least 20 and no more than 90 nucleotides. In other specific embodiments, the isolated nucleic acid molecules have at least 20 and no more than 80 nucleotides. In other specific embodiments, the isolated nucleic acid molecules have at least 20 and no more than 70 nucleotides. In other specific embodiments, the isolated nucleic acid molecules have at least 20 and no more than 60 nucleotides. In other specific embodiments, the isolated nucleic acid molecules have at least 20 and no more than 50 nucleotides. In other specific embodiments, the isolated nucleic acid molecules have at least 20 and no more than 40 nucleotides. In other specific embodiments, the isolated nucleic acid molecules have at least 17 and no more than 40 nucleotides. In other specific embodiments, the isolated nucleic acid molecules have at least 20 and no more than 30 nucleotides. In other specific embodiments, the isolated nucleic acid molecules have at least 17 and no more than 30 nucleotides. In other specific embodiments, the isolated nucleic acid molecules have at least 30 and no more than 300 nucleotides. In other specific embodiments, the isolated nucleic acid molecules have at least 30 and no more than 200 nucleotides. In other specific embodiments, the isolated nucleic acid molecules have at least 30 and no more than 100 nucleotides. In other specific embodiments, the isolated nucleic acid molecules have at least 30 and no more than 90 nucleotides. In other specific embodiments, the isolated nucleic acid molecules have at least 30 and no more than 80 nucleotides. In other specific embodiments, the isolated nucleic acid molecules have at least 30 and no more than 70 nucleotides. In other specific embodiments, the isolated nucleic acid molecules have at least 30 and no more than 60 nucleotides. In other specific embodiments, the isolated nucleic acid molecules have at least 30 and no more than 50 nucleotides. In other specific embodiments, the isolated nucleic acid molecules have at least 30 and no more than 40 nucleotides. It should be understood that in real-time PCR, primers also constitute probe without the traditional meaning of this term. Primers or probes appropriate to detect OPN and/or α₅β₁ in the methods of the present invention can be designed with known methods using sequences distributed across their respective nucleotide sequence. The probes and/or primers of the present invention are designed to specifically hybridize with their target nucleic acid (α₅ (SEQ ID NO:24), β₁ (SEQ ID NO:22), CD44 wild type (SEQ ID NO:19) or CD44 risk allele (e.g., comprising 230I→T (SNP (CT)) variant) and/or OPN (SEQ ID NO:26)). In an embodiment, the primers and probes of the present invention are substantially complementary to their target nucleic acid.

Substantially complementary nucleic acids are nucleic acids in which the complement of one molecule is substantially identical to the other molecule. Two nucleic acid or protein sequences are considered substantially identical if, when optimally aligned, they share at least about 70% sequence identity. In alternative embodiments, sequence identity may for example be at least 75%, at least 80%, at least 85%, at least 90%, at least 95%, at least 96%, at least 98% or at least 99%. Optimal alignment of sequences for comparisons of identity may be conducted using a variety of algorithms, such as the local homology algorithm of Smith and Waterman, 1981, Adv. Appl. Math 2: 482, the homology alignment algorithm of Needleman and Wunsch, 1970, J. Mol. Biol. 48:443, the search for similarity method of Pearson and Lipman, and the computerized implementations of these algorithms (such as GAP, BESTFIT, FASTA and TFASTA in the Wisconsin Genetics Software Package, Genetics Computer Group, Madison, WI, U.S.A.). Sequence identity may also be determined using the BLAST algorithm, described in Altschul et al. 1990 (using the published default settings). Software for performing BLAST analysis may be available through the National Center for Biotechnology Information (through the internet at http://www.ncbi.nlm.nih.gov/). The BLAST algorithm involves first identifying high scoring sequence pairs (HSPs) by identifying short words of length W in the query sequence that either match or satisfy some positive-valued threshold score T when aligned with a word of the same length in a database sequence. T is referred to as the neighborhood word score threshold. Initial neighborhood word hits act as seeds for initiating searches to find longer HSPs. The word hits are extended in both directions along each sequence for as far as the cumulative alignment score can be increased. Extension of the word hits in each direction is halted when the following parameters are met: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. One measure of the statistical similarity between two sequences using the BLAST algorithm is the smallest sum probability (P(N)), which provides an indication of the probability by which a match between two nucleotide or amino acid sequences would occur by chance. In alternative embodiments of the invention, nucleotide or amino acid sequences are considered substantially identical if the smallest sum probability in a comparison of the test sequences is less than about 1, preferably less than about 0.1, more preferably less than about 0.01, and most preferably less than about 0.001.

An alternative indication that two nucleic acid sequences are substantially complementary is that the two sequences hybridize to each other under moderately stringent, or preferably stringent, conditions. Hybridization to filter-bound sequences under moderately stringent conditions may, for example, be performed in 0.5 M NaHPO4, 7% sodium dodecyl sulfate (SDS), 1 mM EDTA at 65°C, and washing in 0.2 x SSC/0.1% SDS at 42°C. Alternatively, hybridization to filter-bound sequences under stringent conditions may, for example, be performed in 0.5 M NaHPO4, 7% SDS, 1 mM EDTA at 65°C, and washing in 0.1 x SSC/0.1% SDS at 68°C. Hybridization conditions may be modified in accordance with known methods depending on the sequence of interest. Generally, stringent conditions are selected to be about 5°C lower than the thermal melting point for the specific sequence at a defined ionic strength and pH.

Probes of the invention can be utilized with naturally occurring sugar-phosphate backbones as well as modified backbones including phosphorothioates, dithionates, alkyl phosphonates and α-nucleotides and the like. Modified sugar-phosphate backbones are generally known. Probes of the invention can be constructed of either ribonucleic acid (RNA) or deoxyribonucleic acid (DNA), and preferably of DNA.

The types of detection methods in which probes can be used include Southern blots (DNA detection), dot or slot blots (DNA, RNA), and Northern blots (RNA detection). Although less preferred, labeled proteins could also be used to detect a particular nucleic acid sequence to which it binds. Other detection methods include kits containing probes on a dipstick setup and the like.

As used herein the terms "detectably labeled" refer to a marking of a probe or an antibody in accordance with the presence invention that will allow the detection of OPN and/or α₅β₁ in accordance with the present invention. Although the present invention is not specifically dependent on the use of a label for the detection of a particular nucleic acid sequence, such a label might be beneficial, by increasing the sensitivity of the detection. Furthermore, it enables automation. Probes can be labeled according to numerous well known methods. Non-limiting examples of labels include ³H, ¹⁴C, ³²P, and ³⁵S. Non-limiting examples of detectable markers include ligands, fluorophores, chemiluminescent agents, enzymes, and antibodies. Other detectable markers for use with probes, which can enable an increase in sensitivity of the method of the invention, include biotin and radionucleotides. It will become evident to the person of ordinary skill that the choice of a particular label dictates the manner in which it is bound to the probe.

As commonly known, radioactive nucleotides can be incorporated into probes of the invention by several methods. Non-limiting examples thereof include kinasing the 5' ends of the probes using gamma ³²P ATP and polynucleotide kinase, using the Klenow fragment of Pol I of *E*. *coli* in the presence of radioactive dNTP (e.g. uniformly labeled DNA probe using random oligonucleotide primers in low-melt gels), using the SP6/T7 system to transcribe a DNA segment in the presence of one or more radioactive NTP, and the like.

The present invention also relates to methods of selecting compounds. As used herein the term "compound" is meant to encompass natural, synthetic or semi-synthetic compounds, including without being so limited chemicals, macromolecules, cell or tissue extracts (from plants or animals), nucleic acid molecules, peptides, antibodies and proteins.

The present invention also relates to arrays. As used herein, an "array" is an intentionally created collection of molecules which can be prepared either synthetically or biosynthetically. The molecules in the array can be identical or different from each other. The array can assume a variety of formats, *e.g*., libraries of soluble molecules; libraries of compounds tethered to resin beads, silica chips, or other solid supports.

As used herein "array of nucleic acid molecules" is an intentionally created collection of nucleic acids which can be prepared either synthetically or biosynthetically in a variety of different formats (*e.g*., libraries of soluble molecules; and libraries of oligonucleotides tethered to resin beads, silica chips, or other solid supports). Additionally, the term "array" is meant to include those libraries of nucleic acids which can be prepared by spotting nucleic acids of essentially any length (*e.g*., from 1 to about 1000 nucleotide monomers in length) onto a substrate. The term "nucleic acid" as used herein refers to a polymeric form of nucleotides of any length, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleotide sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

As used herein "solid support", "support", and "substrate" are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations.

Any known nucleic acid arrays can be used in accordance with the present invention. For instance, such arrays include those based on short or longer oligonucleotide probes as well as cDNAs or polymerase chain reaction (PCR) products. Other methods include serial analysis of gene expression (SAGE), differential display, as well as subtractive hybridization methods, differential screening (DS), RNA arbitrarily primer (RAP)-PCR, restriction endonucleolytic analysis of differentially expressed sequences (READS), amplified restriction fragment-length polymorphisms (AFLP).

### Antibodies

The present invention encompasses using antibodies for detecting or determining OPN and/or α₅β₁ levels for instance in the samples of a subject and for including in kits of the present invention. Antibodies that specifically bind to these biological markers can be produced routinely with methods further described below. The present invention also encompasses using antibodies commercially available. Without being so limited antibodies that specifically bind to OPN and/or α₅β₁ include those listed in Table 1 below.

**Table 1: Non-limiting examples of commercially available human OPN Elisa kits.**

| **Company** | **Kit name** | **Catalogue number** | **Sensitivity** |
|---|---|---|---|
| IBL Hambourg | Human Osteopontin ELISA | JP 171 58 | 3,33ng/ml |
| | | | |
| IBL America | Human Osteopontin N-Half Assay Kit-IBL | 27258 | 3,90 pmol/L |
| | | | |
| IBL-America | Human Osteopontin Assay Kit - IBL | 27158 | 3,33ng/ml |
| | | | |
| Assay designs | Osteopontin (human) EIA Kit | 900-142 | 0,11 ng/ml |
| | | | |
| American Research Products, Inc. | Osteopontin, human kit | 17158 | ? |
| | | | |
| R&D Systems | Human Osteopontin (OPN) ELISA Kit | DOST00 | 0.024 ng/mL |
| | | | |
| Promokine | Human Osteopontin ELISA | PK-EL-KA4231 | 3,6ng/ml |
| | | | |
| .Uscnlife | Human Osteopontin,OPN ELISA Kit | E0899h | ? |

**Table 2: Non-limiting examples of commercially available antibodies for OPN (Human, Unconjugated)**

| Company Name | Catalogue Number | Host |
|---|---|---|
| EMD Millipore | AB10910 | rabbit |
| Boster Immunoleader | PA1431 | |
| LifeSpan BioSciences | LS-C63082-100 | mouse |
| LifeSpan BioSciences | LS-B5940-50 | mouse |
| LifeSpan BioSciences | LS-C137501-100 | mouse |
| LifeSpan BioSciences | LS-C31763-100 | rabbit |
| LifeSpan BioSciences | LS-C99283-400 | rabbit |
| LifeSpan BioSciences | LS-C9410-100 | rabbit |
| LifeSpan BioSciences | LS-C122259-20 | rabbit |
| LifeSpan BioSciences | LS-C88774-0.1 | rabbit |
| LifeSpan BioSciences | LS-C136850-100 | rabbit |
| LifeSpan BioSciences | LS-C96393-500 | rabbit |
| LifeSpan BioSciences | LS-C193595-200 | mouse |
| LifeSpan BioSciences | LS-C193596-100 | mouse |
| LifeSpan BioSciences | LS-C63081-100 | mouse |
| LifeSpan BioSciences | LS-C 193597-100 | mouse |
| LifeSpan BioSciences | LS-C169155-100 | mouse |
| LifeSpan BioSciences | LS-C189569-1000 | mouse |
| LifeSpan BioSciences | LS-C189635-1000 | mouse |
| LifeSpan BioSciences | LS-C189636-1000 | mouse |
| LifeSpan BioSciences | LS-C189634-1000 | mouse |
| LifeSpan BioSciences | LS-C73947-500 | mouse |
| LifeSpan BioSciences | LS-C189134-50 | rabbit |
| LifeSpan BioSciences | LS-B5272-250 | rabbit |
| LifeSpan BioSciences | LS-C176152-100 | rabbit |
| LifeSpan BioSciences | LS-C194024-100 | rabbit |
| LifeSpan BioSciences | LS-B5626-50 | rabbit |
| LifeSpan BioSciences | LS-C131159-20 | rabbit |
| LifeSpan BioSciences | LS-B9287-200 | rabbit |
| LifeSpan BioSciences | LS-C73949-200 | rabbit |
| LifeSpan BioSciences | LS-C182368-50 | rabbit |
| LifeSpan BioSciences | LS-B2411-50 | goat |
| LifeSpan BioSciences | LS-B8326-100 | mouse |
| LifeSpan BioSciences | LS-B7193-50 | rabbit |
| LifeSpan BioSciences | LS-B425-50 | rabbit |
| LifeSpan BioSciences | LS-C9413-100 | rabbit |
| LifeSpan BioSciences | LS-B7193-50 | rabbit |
| LifeSpan BioSciences | LS-C9413-100 | rabbit |
| LifeSpan BioSciences | LS-B9080-100 | rabbit |
| LifeSpan BioSciences | LS-C201116-100 | rabbit |
| Boster Immunoleader | PA1431 | |
| antibodies-online | ABIN933617 | mouse |
| antibodies-online | ABIN1381708 | Chicken |
| BACHEM | T-4816.0400 | Rabbit |
| BACHEM | T-4815.0050 | Rabbit |
| Biorbyt | orb12414 | mouse |
| Biorbyt | orb128774 | Rabbit |
| Biorbyt | orb12506 | mouse |
| Biorbyt | orb94522 | Rabbit |
| Biorbyt | orb13123 | Rabbit |
| Biorbyt | orb88187 | goat |
| Biorbyt | orb94961 | mouse |
| Biorbyt | orb86662 | rabbit |
| Biorbyt | orb170816 | mouse |
| Biorbyt | orb175965 | mouse |
| Biorbyt | orb19047 | goat |
| Biorbyt | orb43142 | rabbit |
| Biorbyt | orb120032 | rabbit |
| Biorbyt | orb11192 | rabbit |
| Biorbyt | orb11191 | rabbit |
| antibodies-online | ABIN933617 | mouse |
| BioVision | 5426-100 | mouse |
| BioVision | 5422-100 | mouse |
| BioVision | 5424-100 | mouse |
| BioVision | 5423-100 | mouse |
| BioVision | 5425-100 | mouse |
| BioVision | 5421-100 | mouse |
| Merck Millipore | 04-970 | mouse |
| Merck Millipore | MAB3055 | rabbit |
| Merck Millipore | AB1870 | Rabbit |
| Merck Millipore | AB10910 | Rabbit |
| GenWay Biotech, Inc. | GWB-T00561 | mouse |
| GenWay Biotech, Inc. | GWB-T00557 | mouse |
| GenWay Biotech, Inc. | GWB-T00558 | mouse |
| GenWay Biotech, Inc. | GWB-T00559 | mouse |
| GenWay Biotech, Inc. | GWB-T00560 | mouse |
| GenWay Biotech, Inc. | GWB-3A2E99 | goat |
| GenWay Biotech, Inc. | GWB-23C38D | rabbit |
| GenWay Biotech, Inc. | GWB-295359 | Rabbit |
| GenWay Biotech, Inc. | GWB-806785 | Goat |
| Enzo Life Sciences, Inc. | ADI-905-629-100 | mouse |
| Enzo Life Sciences, Inc. | ADI-905-630-100 | mouse |
| Enzo Life Sciences, Inc. | ADI-905-500-1 | Rabbit |
| Enzo Life Sciences, Inc. | ALX-210-309-R100 | Rabbit |
| GeneTex | GTX28448 | Rabbit |
| GeneTex | GTX37500 | rabbit |
| GeneTex | GTX15489 | rabbit |
| GeneTex | GTX89519 | goat |
| Spring Bioscience | E3282 | rabbit |
| Spring Bioscience | E3280 | rabbit |
| Spring Bioscience | E3281 | rabbit |
| Spring Bioscience | E3284 | rabbit |
| Abbiotec | 251924 | rabbit |
| Abbiotec | 250801 | rabbit |
| MBL International | CY-P1035 | |
| Rockland Immunochemicals, Inc. | 100-401-404 | Rabbit |
| Bioss Inc. | bs-0026R | Rabbit |
| Bioss Inc. | bs-0019R | Rabbit |
| Proteintech Group Inc | 22952-1-AP | Rabbit |

**Table 4: Non-limiting examples of commercially available ELISA Kits for integrin α₅ (ITGA5, Human)**

| Company Name | Catalogue number | Range | Sensitivity |
|---|---|---|---|
| antibodies-online | ABIN417612 | 0.156-10 ng/mL | 0.054ng/mL |
| antibodies-online | ABIN365741 | na | na |
| DLdevelop | DL-ITGa5-Hu | 0.156-10ng/mL | 0.054ng/mL |
| MyBioSource.com | MBS814027 | na | na |
| R&D Systems | DYC3230-2 | 312-20,000 pg/mL | na |
| Biomatik | E91287Hu | 0.156-10ng/mL | 0.054ng/mL |

**Table 5: Non-limiting examples of commercially available Antibodies for α₅ (ITGA5, human)**

| Company Name | Catalogue Number | Host |
|---|---|---|
| Novus Biologicals | NBP1-84576 | rabbit |
| Biorbyt | orb69201 | mouse |
| Abcam | ab72663 | rabbit |
| Acris Antibodies GmbH | BM4033 | mouse |
| Aviva Systems Biology | OAAF05375 | rabbit |
| St John's Laboratory | STJ32097 | mouse |
| GeneTex | GTX86915 | rabbit |
| GeneTex | GTX86905 | rabbit |
| OriGene Technologies | TA311966 | rabbit |
| OriGene Technologies | TA310024 | rat |
| Abbexa | abx15590 | rabbit |
| Abbexa | abx15591 | rabbit |
| Abbiotec | 252937 | mouse |
| Abnova Corporation | MAB10703 | mouse |
| Abnova Corporation | MAB5267 | mouse |
| Bioss Inc. | bs-0567R | rabbit |
| Cell Signaling Technology | 4705S | rabbit |
| Atlas Antibodies | HPA002642 | rabbit |
| GenWay Biotech, Inc. | GWB-MX190A | rabbit |
| GenWay Biotech, Inc. | GWB-D9743E | mouse |
| antibodies-online | ABIN656138 | rabbit |
| antibodies-online | ABIN219716 | rabbit |
| Novus Biologicals | NBP1-71421-0.1mg | rabbit |
| Novus Biologicals | NBP1-71421-0.05mg | rabbit |
| BioLegend | 328009 | mouse |
| BD Biosciences | 610634 | mouse |
| BioLegend | 328002 | mouse |
| BD Biosciences | 610634 | mouse |
| Abcam | ab72665 | rabbit |
| Abcam | ab55988 | rabbit |
| Bioworld Technology | BS7053 | rabbit |
| Santa Cruz Biotechnology, Inc. | sc-166665 | mouse |
| Bioworld Technology | BS7052 | rabbit |
| R&D Systems | AF1864 | goat |
| R&D Systems | FAB1864A | mouse |
| Thermo Scientific Pierce Antibodies | MA5-15568 | mouse |
| Thermo Scientific Pierce Antibodies | MA1-81134 | mouse |
| AbD Serotec (Bio-Rad) | MCA1187 | mouse |
| AbD Serotec (Bio-Rad) | MCA1187T | mouse |
| Life Technologies | 132600 | mouse |
| Proteintech Group Inc | 10569-1-AP | rabbit |
| Raybiotech, Inc. | 119-14178 | mouse |
| Creative Biomart | CAB-3671MH | mouse |
| Merck Millipore | CBL497 | mouse |
| Fitzgerald Industries International | 10R-1984 | mouse |
| EMD Millipore | AB1921 | rabbit |
| EMD Millipore | AB1949 | rabbit |

**Table 6: Non-limiting examples of commercially available ELISA Kits for β1 (ITGB1, human)**

| Company Name | Catalogue number | Range | Sensitivity |
|---|---|---|---|
| antibodies-online | ABIN833710 | na | na |
| Merck Millipore | ECM470 | na | na |
| DLdevelop | DL-ITGb1-Hu | 1.56-100ng/mL | na |
| Biomatik | E91042Hu | 1.56-100ng/mL | 0.64ng/mL |

**Table 7: Non-limiting examples of commercially available antibodies for β₁ ITGB1 (Human, Unconjugated)**

| Company Name | Catalogue number | Host |
|---|---|---|
| Abgent | AM2241b | mouse |
| Biorbyt | orb86390 | rabbit |
| LifeSpan BioSciences | LS-C84969-100 | mouse |
| Novus Biologicals | NB110-55545 | rabbit |
| Abbexa | abx12778 | rabbit |
| Bethyl Laboratories, Inc. | A303-735A | rabbit |
| Abcam | ab5189 | Rabbit |
| St John's Laboratory | STJ60344 | Rabbit |
| Cell Signaling Technology | 4706S | Rabbit |
| Bioss Inc. | bs-0486R | Rabbit |
| Antigenix America Inc. | MA290020 | |
| GenWay Biotech, Inc. | GWB-312F4D | mouse |
| Fitzgerald Industries International | 20R-2722 | rabbit |
| GeneTex | GTX50784 | rabbit |
| Thermo Scientific Pierce Antibodies | MA1-80764 | mouse |
| R&D Systems | AF1778 | goat |
| Abbiotec | 251162 | rabbit |
| Bioworld Technology | BS1817 | rabbit |
| Abgent | AM2241b | mouse |
| Enzo Life Sciences, Inc. | BML-IG6060-0100 | mouse |
| BIOCARE MEDICAL | CME 386 A | rabbit |
| ProSci, Inc | 48-392 | Rabbit |
| eBioscience | 14-0299-82 | mouse |

Both monoclonal and polyclonal antibodies directed to OPN, α₅ and/or β₁ are included within the scope of this invention as they can be produced by well established procedures known to those of skill in the art. Additionally, any secondary antibodies, either monoclonal or polyclonal, directed to the first antibodies would also be included within the scope of this invention.

As used herein, the terms "anti-OPN antibody", anti-α₅ antibody", "anti-β₁ antibody" and "anti-α₅β₁ antibody" or "immunologically specific anti-OPN antibody", immunologically specific anti-α₅ antibody", immunologically specific anti-β₁ antibody" or "immunologically specific anti-α₅β₁ antibody" refers to an antibody that specifically binds to (interacts with) an OPN, α₅, β₁ or α₅β₁ protein, respectively and displays no substantial binding to other naturally occurring proteins other than the ones sharing the same antigenic determinants as the OPN, α₅, β₁ or α₅β₁ protein, respectively. The term antibody or immunoglobulin is used in the broadest sense, and covers monoclonal antibodies (including full length monoclonal antibodies), polyclonal antibodies, multispecific antibodies, and antibody fragments so long as they exhibit the desired biological activity. Antibody fragments comprise a portion of a full length antibody, generally an antigen binding or variable region thereof. Examples of antibody fragments include Fab, Fab', F(ab')₂, and Fv fragments, diabodies, linear antibodies, single-chain antibody molecules, single domain antibodies (*e.g*., from camelids), shark NAR single domain antibodies, and multispecific antibodies formed from antibody fragments. Antibody fragments can also refer to binding moieties comprising CDRs or antigen binding domains including, but not limited to, VH regions (V_{H}, V_{H}-V_{H}), anticalins, PepBodies™, antibody-T-cell epitope fusions (Troybodies) or Peptibodies. Additionally, any secondary antibodies, either monoclonal or polyclonal, directed to the first antibodies would also be included within the scope of this invention.

In general, techniques for preparing antibodies (including monoclonal antibodies and hybridomas) and for detecting antigens using antibodies are well known in the art (Campbell, 1984, In "Monoclonal Antibody Technology: Laboratory Techniques in Biochemistry and Molecular Biology", Elsevier Science Publisher, Amsterdam, The Netherlands) and in Harlow et al., 1988 (in: Antibody A Laboratory Manual, CSH Laboratories). The term antibody encompasses herein polyclonal, monoclonal antibodies and antibody variants such as single-chain antibodies, humanized antibodies, chimeric antibodies and immunologically active fragments of antibodies (e.g. Fab and Fab' fragments) which inhibit or neutralize their respective interaction domains in Hyphen and/or are specific thereto.

Polyclonal antibodies are preferably raised in animals by multiple subcutaneous (sc), intravenous (iv) or intraperitoneal (ip) injections of the relevant antigen with or without an adjuvant. It may be useful to conjugate the relevant antigen to a protein that is immunogenic in the species to be immunized, *e.g*., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals may be immunized against the antigen, immunogenic conjugates, or derivatives by combining the antigen or conjugate (*e.g*., 100 µg for rabbits or 5 µg for mice) with 3 volumes of Freund's complete adjuvant and injecting the solution intradermally at multiple sites. One month later the animals are boosted with the antigen or conjugate (*e.g*., with 1/5 to 1/10 of the original amount used to immunize) in Freund's complete adjuvant by subcutaneous injection at multiple sites. Seven to 14 days later the animals are bled and the serum is assayed for antibody titer. Animals are boosted until the titer plateaus. Preferably, for conjugate immunizations, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies may be made using the hybridoma method first described by Kohler et al., Nature, 256: 495 (1975), or may be made by recombinant DNA methods (*e.g*., U.S. Patent No. 6,204,023). Monoclonal antibodies may also be made using the techniques described in U.S. Patent Nos. 6,025,155 and 6,077,677 as well as U.S. Patent Application Publication Nos. 2002/0160970 and 2003/0083293.

In the hybridoma method, a mouse or other appropriate host animal, such as a rat, hamster or monkey, is immunized (*e.g*., as hereinabove described) to elicit lymphocytes that produce or are capable of producing antibodies that will specifically bind to the antigen used for immunization. Alternatively, lymphocytes may be immunized *in vitro.* Lymphocytes then are fused with myeloma cells using a suitable fusing agent, such as polyethylene glycol, to form a hybridoma cell.

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

As used herein, the term "purified" in the expression "purified antibody" is simply meant to distinguish man-made antibody from an antibody that may naturally be produced by an animal against its own antigens. Hence, raw serum and hybridoma culture medium containing anti-OPN antibody are "purified antibodies" within the meaning of the present invention.

The present invention also encompasses arrays to detect and/or quantify the translation products of OPN and α₅β₁. Such arrays include protein micro- or macroarrays, gel technologies including high-resolution 2D-gel methodologies, possibly coupled with mass spectrometry imaging system at the cellular level such as microscopy combined with a fluorescent labeling system.

The present invention also encompasses methods to screen/select for potential useful therapeutic agents using whole cells assays, the therapeutic compound being able to increase the transcription and/or synthesis of OPN and/or α₅ and/or β₁ or to decrease the transcription or synthesis of CD44/sCD44. Cells for use in such methods includes cells of any source (including in house or commercially available cell lines) and type (any tissue). In house cell lines could be made for instance by immortalizing cells from AIS subjects. In specific embodiments, methods of screening of the invention seek to identify agents that inhibit OPN and/or α₅β₁ expression and/or activity. Useful cell lines for these embodiments include those producing high levels of OPN and/or α₅β₁ or low levels of CD44/sCD44. Non-limiting examples of useful cells include MC3T3-E1 cells and PBMCs.

In a particular embodiment, it includes cells of any cell type derived from a scoliotic patient. (whole cell assay). In specific embodiments, it includes osteoblasts, chondrocytes, myoblasts or blood cells including PBMCs including lymphocytes. As used herein, the term "cell derived from a scoliotic patient" refers to cells isolated directly from scoliotic patients, or immortalized cell lines originating from cells isolated directly from scoliotic patients. In specific embodiments, the cells are paraspinal muscle cells. Such cells may be isolated by a subject through needle biopsies for instance.

The present invention also concerns pharmaceutical compositions for modulating OPN-mediated GiPCR cell signalling and/or for treating or preventing scoliosis in a subject in need thereof (e.g., iodiopathic scoliosis or AIS). Such compositions include agents for increasing or decreasing OPN-mediated GiPCR signalling inhibition in a subject in need thereof. For instance, pharmaceutical compositions of the present invention may comprise OPN, α₅, β₁, and/or α₅β₁ antibodies (i.e., neutralizing antibodies), RGD peptides or antisence/siRNAs against OPN, α₅, β₁, and/or α₅β₁ to decrease OPN and/or α₅β₁ activity. In an embodiment, the pharmaceutical compositions may comprise sCD44 or a stimulator/enhancer of sCD44/CD44 expression. Pharmaceutical compositions can be administered by any suitable routes such as nasally, intravenously, intramuscularly, subcutaneously, sublingually, intrathecally, or intradermally. The route of administration can depend on a variety of factors, such as the environment and therapeutic goals.

### Dosage

Any suitable amount of a pharmaceutical composition can be administered to a subject. The dosages will depend on many factors including the mode of administration. Typically, the amount of anti-scoliosis composition (e.g., agent that decreases OPN and/or α₅β₁) contained within a single dose will be an amount that effectively prevents, delays or reduces scoliosis without inducing significant toxicity "therapeutically effective amount".

The effective amount of the agent that decreases OPN and/or α₅β₁ may also be measured directly. The effective amount may be given daily or weekly or fractions thereof. Typically, a pharmaceutical and/or nutraceutical and/or dietary supplement composition of the invention can be administered in an amount from about 0.001 mg up to about 500 mg per kg of body weight per day (e.g., 10 mg, 50 mg, 100 mg, or 250 mg). Dosages may be provided in either a single or multiple dosage regimen. For example, in some embodiments the effective amount is a dose that ranges from about 1 mg to about 25 grams of the anti-scoliosis preparation per day, about 50 mg to about 10 grams of the anti- scoliosis preparation per day, from about 100 mg to about 5 grams of the anti- scoliosis preparation per day, about 1 gram of the anti- scoliosis preparation per day, about 1 mg to about 25 grams of the anti- scoliosis preparation per week, about 50 mg to about 10 grams of the anti-scoliosis preparation per week, about 100 mg to about 5 grams of the anti- scoliosis preparation every other day, and about 1 gram of the anti- scoliosis preparation once a week.

By way of example, a pharmaceutical (e.g., containing an agent that decreases OPN and/or α₅β₁) composition of the invention can be in the form of a liquid, solution, suspension, pill, capsule, tablet, gelcap, powder, gel, ointment, cream, nebulae, mist, atomized vapor, aerosol, or phytosome. For oral administration, tablets or capsules can be prepared by conventional means with at least one pharmaceutically acceptable excipient such as binding agents, fillers, lubricants, disintegrants, or wetting agents. The tablets can be coated by methods known in the art. Liquid preparations for oral administration can take the form of, for example, solutions, syrups, or suspension, or they can be presented as a dry product for constitution with saline or other suitable liquid vehicle before use. Preparations for oral administration also can be suitably formulated to give controlled release of the active ingredients.

In addition, a pharmaceutical (e.g., containing an agent that decreases OPN and/or α₅β₁) composition of the invention can contain a pharmaceutically acceptable carrier for administration to a mammal, including, without limitation, sterile aqueous or non-aqueous solutions, suspensions, and emulsions. Examples of non-aqueous solvents include, without limitation, propylene glycol, polyethylene glycol, vegetable oils, and injectable organic esters. Aqueous carriers include, without limitation, water, alcohol, saline, and buffered solutions. Pharmaceutically acceptable carriers also can include physiologically acceptable aqueous vehicles (*e.g*., physiological saline) or other known carriers appropriate to specific routes of administration.

An agent that decreases OPN and/or α₅ and/or β₁ expression or activity or an agent that increases sCD44/CD44 level (e.g., binding to OPN) may be incorporated into dosage forms in conjunction with any of the vehicles which are commonly employed in pharmaceutical preparations, e.g. talc, gum arabic, lactose, starch, magnesium stearate, cocoa butter, aqueous or non-aqueous solvents, oils, paraffin derivatives or glycols. Emulsions such as those described in U.S. Pat. No. 5,434,183, may also be used in which vegetable oil (e.g., soybean oil or safflower oil), emulsifying agent (e.g., egg yolk phospholipid) and water are combined with glycerol. Methods for preparing appropriate formulations are well known in the art (see e.g., Remington's Pharmaceutical Sciences, 16th Ed., 1980, A. Oslo Ed., Easton, Pa.).

In cases where parenteral administration is elected as the route of administration, preparations containing agent that decreases OPN and/or α₅β₁ may be provided to patients in combination with pharmaceutically acceptable sterile aqueous or non-aqueous solvents, suspensions or emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medical parenteral vehicles including sodium chloride solution, Ringer's dextrose solution, dextrose plus sodium chloride solution, Ringer's solution containing lactose, or fixed oils. Intravenous vehicles may include fluid and nutrient replenishers, electrolyte replenishers, such as those based upon Ringer's dextrose, and the like.

These are simply guidelines since the actual dose must be carefully selected and titrated by the attending physician based upon clinical factors unique to each patient or by a nutritionist. The optimal daily dose will be determined by methods known in the art and will be influenced by factors such as the age of the patient and other clinically relevant factors. In addition, patients may be taking medications for other diseases or conditions. The other medications may be continued during the time that the agent that decreases OPN and/or α₅β₁ is given to the patient, but it is particularly advisable in such cases to begin with low doses to determine if adverse side effects are experienced.

The present invention also relates to the detection of a CD44 risk allele (e.g., comprising one or more SNPs) in a subject in need thereof. In accordance with the present invention, the CD44 risk allele comprises a SNP located in the coding regions of CD44 and can thus be detected at the genomic, mRNA or protein level. SNP genotyping is the measurement of genetic variations of single nucleotide polymorphisms (SNPs) between members of a species (e.g., humans). A SNP is a single base pair mutation at a specific locus, usually consisting of two alleles (where the rare allele frequency is >1%). SNP genotyping can be performed using methods well-known in the art such as sequencing (e.g., minisequencing, pyrosequencing), hybridization-based methods (using probes complementary to the SNP site which are hybridized under high stringency conditions), enzyme-based methods, Single strand conformation polymorphism (SSCP), Temperature gradient gel electrophoresis, Denaturing high performance liquid chromatography, High-resolution melting of the entire amplicon, using DNA mismatch-binding proteins, or the SNPlexTM platform. Non limiting examples of hybridization-based methods include Dynamic allele-specific hybridization and use of Molecular beacons. Non-limiting examples of enzyme-based methods include, Restriction fragment length polymorphism (RFLP), PCR-based methods (e.g., ARMS), Flap endonuclease (e.g., Invader), primer extension, 5'-nuclease (TaqManTM assay), oligonucleotide ligation assays.

In an embodiment, methods of the present invention comprise methods for i) determining the risk of developing a scoliosis; and ii) methods of stratifying subjects having scoliosis comprising determining the presence of a CD44 risk allele comprising SNP rs1467558 or a mutation/SNP/marker in linkage disequilibrium therewith.

In particular embodiments of the invention, linkage disequilibrium (LD) is defined by a specific quantitative cutoff. As described in detail herein, linkage disequilibrium can be quantitatively determined by measures such as r² and |D'|. As a consequence, certain embodiments of the invention relate to substitute markers in linkage disequilibrium by a measure within a certain range specified by particular values of r² and/or |D'|. In an embodiment, LD is characterized by numerical values for r² of greater than 0.1. In another embodiment, LD is characterized by numerical values for r² of greater than 0.5. In another embodiment, LD is characterized by numerical values for r² of greater than 0.8. In another embodiment, LD is characterized by numerical values for r² of 0.9 or more.

### Linkage Disequilibrium

The natural phenomenon of recombination, which occurs on average once for each chromosomal pair during each meiotic event, represents one way in which nature provides variations in sequence (and biological function by consequence). It has been discovered that recombination does not occur randomly in the genome; rather, there are large variations in the frequency of recombination rates, resulting in small regions of high recombination frequency (also called recombination hotspots) and larger regions of low recombination frequency, which are commonly referred to as Linkage Disequilibrium (LD) blocks (Myers, S. et al., Biochem Soc Trans 34:526-530 (2006); Jeffreys, AJ., et al., Nature Genet 29:217-222 (2001); May, C.A., et al., Nature Genet 31:272-275(2002)).

Linkage Disequilibrium (LD) refers to a non-random assortment of two genetic elements. Thus, the term "linkage disequilibrium" refers to a non-random genetic association between one or more allele(s) of two different polymorphic DNA sequences, that is due to the physical proximity of the two loci. Linkage disequilibrium is present when two DNA segments that are very close to each other on a given chromosome will tend to remain unseparated for several generations with the consequence that alleles of a DNA polymorphism (or marker) in one segment will show a non-random association with the alleles of a different DNA polymorphism (or marker) located in the other DNA segment nearby. This situation is encountered throughout the human genome when two DNA polymorphisms that are very close to each other are studied. Linkage disequilibrium and the use thereof in inheritance studies is well known in the art to which the present invention pertains as exemplified by publications such as Risch and Merikangas, Science 273: 1516-1517 (1996); Maniatis, Methods Mol Biol. 376: 109-21 (2007) and Borecki et al., Adv Genet 60: 51-74 (2008).

For example, if a particular genetic element (*e.g*., an allele of a polymorphic marker, or a haplotype) occurs in a population at a frequency of 0.25 (25%) and another element occurs at a frequency of 0.25 (25%), then the predicted occurrence of a person's having both elements is 0.125 (12.5%), assuming a random distribution of the elements. However, if it is discovered that the two elements occur together at a frequency higher than 0.125, then the elements are said to be in linkage disequilibrium, since they tend to be inherited together at a higher rate than what their independent frequencies of occurrence (*e.g*., allele or haplotype frequencies) would predict. Roughly speaking, LD is generally correlated with the frequency of recombination events between the two elements. Allele or haplotype frequencies can be determined in a population by genotyping individuals in a population and determining the frequency of the occurrence of each allele or haplotype in the population.

Many different measures have been proposed for assessing the strength of linkage disequilibrium (LD). Most capture the strength of association between pairs of biallelic sites. Two important pairwise measures of LD are r² (sometimes denoted Δ) and |D'|. Both measures range from 0 (no disequilibrium) to 1 ('complete' disequilibrium), but their interpretation is slightly different. |D'| is defined in such a way that it is equal to 1 if just two or three of the possible haplotypes are present, and it is <1 if all four possible haplotypes are present. Therefore, a value of |D'| that is <1 indicates that historical recombination may have occurred between two sites (recurrent mutation can also cause |D'| to be <1, but for single nucleotide polymorphisms (SNPs) this is usually regarded as being less likely than recombination). The measure r² represents the statistical correlation between two sites, and takes the value of 1 if only two haplotypes are present.

The r² measure is arguably the most relevant measure for association mapping, because there is a simple inverse relationship between r² and the sample size required to detect association between susceptibility loci and SNPs. These measures are defined for pairs of sites, but for some applications a determination of how strong LD is across an entire region that contains many polymorphic sites might be desirable (*e.g*., testing whether the strength of LD differs significantly among loci or across populations, or whether there is more or less LD in a region than predicted under a particular model).

One approach of measuring LD across a region is to use the measure r, which was developed in population genetics. Roughly speaking, r measures how much recombination would be required under a particular population model to generate the LD that is seen in the data. This type of method can potentially also provide a statistically rigorous approach to the problem of determining whether LD data provide evidence for the presence of recombination hotspots. For the methods and procedures described herein, a significant r² value can be at least 0.1 such as at least 0.1, 0.2, 0.3, 0.4, 0.5, 0.6, 0.7, 0.75, 0.8, 0.85, 0.9, 0.91, 0.92, 0.93, 0.94, 0.95, 0.96, 0.97, 0.98, 0.99 or 1.0. In one preferred embodiment, the significant r² value can be at least 0.8. Alternatively, linkage disequilibrium as described herein, refers to linkage disequilibrium characterized by values of |D'| of at least 0.2, such as 0.3, 0.4, 0.5, 0.6, 0.7, 0.8, 0.85, 0.9, 0.95, 0.96, 0.97, 0.98 or 0.99. Thus, linkage disequilibrium represents a correlation between alleles of distinct markers. It is measured by correlation coefficient or |D'| (r² up to 1.0 and |D'| up to 1.0). Linkage disequilibrium can be determined in a single human population, as defined herein, or it can be determined in a collection of samples comprising individuals from more than one human population. In one embodiment of the invention, LD is determined in a sample from one or more of the HapMap™ populations (Caucasian, African, Japanese, Chinese), as defined (http://www.hapmap.org). In one such embodiment, LD is determined in the CEU population of the HapMap™ III samples.

If all polymorphisms in the genome were identical at the population level, then every single one of them would need to be investigated in association studies. However, due to linkage disequilibrium between polymorphisms, tightly linked polymorphisms are strongly correlated, which reduces the number of polymorphisms that need to be investigated in an association study to observe a significant association. Genomic LD maps have been generated across the genome, and such LD maps have been proposed to serve as framework for mapping disease-genes (Risch, N. and Merkiangas, K, Science 273:1516-1517 (1996); Maniatis, N., et al., Proc Natl Acad Sci USA 99:2228-2233 (2002); Reich, DE et al, Nature 411:199-204 (2001)).

It is now established that many portions of the human genome can be broken into series of discrete haplotype blocks containing a few common haplotypes; for these blocks, linkage disequilibrium data provides little evidence indicating recombination (see, *e.g*., Wall., J.D. and Pritchard, J.K., Nature Reviews Genetics 4:587-597 (2003); Daly, M. et al., Nature Genet. 29:229-232 (2001); Gabriel, S.B. et al., Science 296:2225-2229 (2002); Patil, N. et al., Science 294: 1719-1723 (2001); Dawson, E. et al., Nature 4JS:544-548 (2002); Phillips, M.S. et al., Nature Genet 33:382-387 (2003)).

There are two main methods for defining these haplotype blocks: Blocks can be defined as regions of DNA that have limited haplotype diversity (see, *e.g*., Daly, M. et al., Nature Genet. 29:229-232 (2001); Patil, N. et al., Science 294: 1719-1723 (2001); Dawson, E. et al., Nature 418:544-548 (2002); Zhang, K. et al., Proc. Natl. Acad. Sci. USA 99:7335-7339 (2002)), or as regions between transition zones having extensive historical recombination, identified using linkage disequilibrium (see, *e.g*., Gabriel, S.B. et al., Science 296:2225-2229 (2002); Phillips, M.S. et al., Nature Genet. 33:382-387 (2003); Wang, N. et al., Am. J. Hum. Genet. 7 :1227-1234 (2002); Stumpf, M.P., and Goldstein, D.B., Curr. Biol. 13: 1-8 (2003)). More recently, a fine-scale map of recombination rates and corresponding hotspots across the human genome has been generated (Myers, S., et al., Science 310:321-32324 (2005); Myers, S. et al., Biochem Soc Trans 34:526530 (2006)). The map reveals the enormous variation in recombination across the genome, with recombination rates as high as 10-60 cM/Mb in hotspots, while closer to 0 in intervening regions, which thus represent regions of limited haplotype diversity and high LD. The map can therefore be used to define haplotype blocks/LD blocks as regions flanked by recombination hotspots. As used herein, the terms "haplotype block" or "LD block" includes blocks defined by any of the above described characteristics, or other alternative methods used by the person skilled in the art to define such regions.

Haplotype blocks can be used to map associations between phenotype and haplotype status, using single markers or haplotypes comprising a plurality of markers.

The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks. Thus, the skilled person can readily identify SNPs/variants/markers in linkage disequilibrium with the CD44 risk allele identified herein and can use this marker in linkage disequilibrium to stratify subjects and to determine risk of developing scoliosis.

When the SNP is translated at the protein level, the mutation/variation can be detected using well-known protein detection methods such as ELISA, immunofluorescence, Western blot, sequencing, electrophoresis, HPLC, MS, etc.

The present invention also relates to kits. Without being so limited, it relates to kits for i) increasing GiPCR signaling in cells; ii) stratifying scoliotic subjects, and/or iii) predicting whether a subject is at risk of developing a scoliosis comprising an isolated nucleic acid (e.g., a primer or probe specific for (which is substantially complementary to or hybridizes to α₅ (SEQ ID NO:24), β₁ (SEQ ID NO:22), CD44 (wild type (SEQ ID NO:19) or SNP risk allele (e.g., 230I→T (CT) variant) and/or OPN (SEQ ID NO:26)) a protein or a ligand such as an antibody (for α₅, β₁, CD44 (wild type or SNP risk allele (e.g., 230I→T variant) and/or OPN) in accordance with the present invention as described above. For example, a compartmentalized kit in accordance with the present invention includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the subject sample (DNA genomic nucleic acid, cell sample or blood samples), a container which contains in some kits of the present invention, the probes used in the methods of the present invention, containers which contain enzymes, containers which contain wash reagents, and containers which contain the reagents used to detect the extension products. Kits of the present invention may also contain instructions to use these probes and or antibodies to stratify scoliotic subjects or predict whether a subject is at risk of developing a scoliosis.

The articles "a," "an" and "the" are used herein to refer to one or to more than one (i.e., to at least one) of the grammatical object of the article.

The term "including" and "comprising" are used herein to mean, and re used interchangeably with, the phrases "including but not limited to" and "comprising but not limited to".

The terms "such as" are used herein to mean, and is used interchangeably with, the phrase "such as but not limited to".

The present invention is illustrated in further details by the following non-limiting examples.

### EXAMPLE 1

### Materials and Methods

### Experimental animal models

The Institutional Review Board for the care and handling of animals used in research (CHU Sainte-Justine) has approved the protocol in accordance with the guidelines of the Canadian Council of Animal Care.

Breeding pairs of C57BI/6j mice devoid of either OPN (OPN-null mice) or CD44 (CD44-null mice) were obtained from Dr. Susan Rittling (Rutger University, NJ, USA) and were backcrossed for more than 10 generations in C57BI/6j background to establish our own colonies, while C57BI/6j mice were used as wild-type control mice (Charles-River, Wilmington, MA, USA). C57BI6/6j mouse strain was used because it is naturally deficient in melatonin (Von Gall et al., 2000) and exhibits high circulating OPN levels (Aherrahrou et al., 2004). These mice develop scoliosis when they are maintained in a bipedal state. (Machida et al., 2006). Bipedal surgeries were performed after weaning (5-weeks after birth) by amputation of the forelimbs and tail under anesthesia as reported previously. (Machida et al., 2006). Similarly bipedal C57BI/6j CD44-null mice were generated.

### Derivation of primary osteoblast cultures

Bone specimens from mice were obtained from the spine after euthanasia. Bone fragments were reduced to smaller pieces with a cutter in sterile conditions. The small bone pieces were incubated in αMEM medium containing 10% fetal bovine serum (FBS; certified FBS, Invitrogen, Burlington, ON, Canada) and 1% penicillin/ streptomycin (Invitrogen) at 37°C in 5% CO₂, in a 10-cm² culture dish. After one month, osteoblasts emerging from the bone pieces were separated from the remaining bone fragments by trypsinization. RNA was extracted from the osteoblasts using the TRIzol™ method, (Invitrogen). Expression profiles were studied by qPCR. Transcript expression was assessed with the Stratagene™ Mx3000P (Agilent Technologies, La Jolla, CA).

### Functional evaluation of G proteins

The functional evaluation of Gi, Gs and Gq proteins was assessed by CDS assays using osteoblasts cells derived from C57BI/6j WT and C57BI/6j OPN^{-/-} mice (bipedal and quadrupedal) using CellKey™ apparatus, as previously described (Akoume et al., 2010, and WO 2010/040234 to Moreau et al.).

### Osteopontin enzyme-linked immunosorbent assays

Peripheral blood samples were collected in EDTA-treated tubes and then centrifuged. Plasma samples were derived then aliquoted and reserved frozen at -80°C until thawed and analyzed. The concentrations of plasma OPN were measured by enzyme-linked immunosorbent assays (ELISA) as said by protocols provided by the manufacturer (IBL, Hamburg, Germany). This OPN ELISA kit measures total concentration of both phosphorylated and non-phosphorylated forms of OPN in plasma. All ELISA tests were performed in duplicate and the optical density was measured at 450 nm using an DTX880 microplate reader (Beckman Coulter, USA).

### Quantitative reverse transcription-polymerase chain reaction (qPCR)

Thermo-Script™ reverse transcriptase (Invitrogen) was used to reverse transcribe mRNA into cDNA (1mg total concentration). Several dilutions were tested to choose the concentration that yielded the most efficient amplification. The human primers used were the following:
β-actin forward 5'-GGAAATCGTGCGTGACAT-3' (SEQ ID NO: 1),
β-actin reverse 5'-TCATGATGGAGTTGAAGGTAGTT-3' (SEQ ID NO: 2),
CD44 forward 5'-AGCATCGGATTTGAGACCTG-3' (SEQ ID NO: 3),
CD44 reverse 5'-TGAGTCCACTTGGCTTTCTG-3' (SEQ ID NO: 4),
β1 integrin forward 5'-ATGTGTCAGACCTGCCTTG-3' (SEQ ID NO: 5),
β1 integrin reverse 5'-TTGTCCCGACTTTCTACCTTG-3' (SEQ ID NO: 6),
αv integrin forward 5'-GTCCCCACAGTAGACACATATG-3' (SEQ ID NO: 7),
αv integrin reverse 5'-TCAACTCCTCGCTTTCCATG-3' (SEQ ID NO: 8).

Each amplification was performed in duplicate using 5ml of diluted cDNA, 7.5ml of 3mM primer solution and 12.5ml of 2X QuantiTect™ SYBR Green PCR Master Mix (QIAGEN Inc, Ontario, Canada). All reaction mixes were run on Mx3000P™ system from Stratagene (Agilent Technologies Company, La Jolla, CA) and analyzed with MxPro™ QPCR Software also from Stratagene. Relative quantification was calculated with the delta CT method using β-actin as the endogenous control.

### Cell lines and siRNA transfection

MC3T3-E1 cells were used to check the effect of the knockdown of OPN and its receptors. MC3T3-E1 osteoblasts cells were transiently transfected in serum-free medium, using Lipofectamine™ RNAiMAX reagent (Invitrogen) according to the manufacturer's instructions and functional experiments were performed 48h post transfection. The sequence of RNA oligonucleotides used for the knockdown of different genes are, ssp1 (encodes OPN) (CCA CAG CCA CAA GCA GUC CAG AUU A (SEQ ID NO: 9)), integrin β1 (CCU AAG UCA GCA GUA GGA ACA UUA U (SEQ ID NO: 10)), integrin β3 (CCU CCA GCU CAU UGU UGA UGC UUA U (SEQ ID NO: 11)), integrin α5 (CCG AGU ACC UGA UCA ACC UGG UUC A (SEQ ID NO: 12)), integrin α8 (GAG AUG AAA CUG AAU UCC GAG AUA A (SEQ ID NO: 13)), integrin αv (GAC UGU UGA GUA UGC UCC AUG UAG A (SEQ ID NO: 14 and CD44 (GAA CAA GGA GUC GUC AGA AAC UCC A (SEQ ID NO: 15)). Two other siRNAs against integrin α5 were tested (UCC ACC AUG UCU AUG AGC UCA UCA A (SEQ ID NO:16) and UCA GGA GCA GAU UGC AGA AUC UUA U (SEQ ID NO:17)). Comparable results were obtained will all siRNAs.

### The evaluation of the effect of OPN on GPCR and Gi proteins expression

Gi proteins isoforms expression levels were determined using Western blot technique. MC3T3-E1 cells were treated with PBS or rOPN 0.5µg/ml overnight (R&D systems Inc., Minneapolis, USA). These cells were washed with cold PBS 1x then lysed in RIPA buffer (25 mM Tris.Hcl pH7.4, 150 mM NaCl, 1 % NP-40, 1 % sodium deoxycholate, 0.1 % SDS) added with 5 mM NaVO₄ and protease inhibitor cocktail (Roche molecular Biochemicals, Mannheim, Germany). Immunoblots were performed with primary antibodies directed specifically against Gi₁, Gi₂, Gi₃, Gs, Phosphoserine, integrin β1 (Santa Cruz Biotechnology, Santa Cruz, CA), Mu opioid receptor (MOR) (abcam Toronto, ON, Canada), Lysophosphatidic acid receptor 1 (LPAR1) (assaybiotech Inc., USA), melatonin receptor 2 (MT2) and peroxidase-conjugated secondary antibody. Bands were then visualized using SuperSignal™ chemiluminescent substrate (Pierce, Rockford, IL).

### Effect of OPN on interaction of Gi proteins with GPCR and β1 integrin

MC3T3-E1 cells were treated with PBS or rOPN (05µg/ ml) then the whole cell proteins (1mg) were incubated with anti-MOR (Abcam Toronto, ON, Canada), anti-MT2 or anti-β₁ integrin (Santa Cruz Biotechnology), plus protein G beads for immunoprecipitation (IP) at 4 °C overnight. Purified proteins were loaded on 10 % gel after IP, then, processed for gel transferring to PVDF membrane and 5 % BSA blocking. Membranes were exposed to antibodies specific for Gi₁, Gi₂, Gi₃, MOR, MT2 and β₁ integrin at 4 °C overnight and, subsequently, treated with secondary antibody at room temperature for 1 h. Bands were visualized using SuperSignal™ chemiluminescent (Pierce, Rockford, IL) and quantified by densitometric scanning.

### Whole exome sequencing Identification of SNP in AIS subjects associated with increased risk of spinal deformity progression

A Whole Exome Sequencing (WES) study was performed using Agilent targeted exon capture and Life Technologies SOLiD 5500TM for sequencing. GEMINI1TM was used for SNV (Single Nucleotide Variant) annotations. A SNP (rs1467558) was identified in AIS subjects with more severe AIS, which changes the amino acid isoleucine 230 to a threonine in the CD44 coding sequence.

### Statistical analysis

Data are presented as mean ± SE, and were analyzed by ANOVA or Student's t test using GraphPad™ Prism 4.0 software. Multiple comparisons of means were performed with one-way ANOVA followed by a post-hoc test of Dunnett. Only P values < 0.05 were considered significant.

### EXAMPLE 2

### Genetic deletion of OPN protects bipedal C57BI/6 from scoliosis

The bipedal C57BI/6 mice are widely used as animal model to study the pathophysiological events leading to idiopathic scoliosis. These mice rapidly develop spinal deformity following 40 weeks of bipedal ambulation (Oyama et al., 2006). To determine whether OPN is involved in the development of idiopathic scoliosis, female wild-type (WT) and OPN knockout (OPN^{-/-}) C57BI/6 mice were amputated from forelimbs and tails at 1 month of age and subjected to bipedal ambulation for 36 weeks to induce scoliosis. Representative radiographs of the spine as taken at the end of the experiment period are shown in **Fig. 1**, **A-D**. As expected, scoliosis did not develop in any of the WT or OPN^{-/-} quadrupedal mice. However, lateral curvature was apparent in all bipedal WT mice. The convexity of curve was directed to either side, with no consistent preference. In contrast, analysis of radiographs did not yield evidence of scoliosis in bipedal OPN^{-/-} mice. All bipedal OPN^{-/-} mice had straight spine indistinguishable from the quadrupedal mice. These data strongly emphasize that genetic OPN deletion protects bipedal C57BI/6 mice from scoliosis.

To provide further evidence for this hypothesis, OPN was measured in plasma from bipedal mice at 12, 24 and 36 weeks after surgery (**Fig. 1** **E**), and compared with plasma OPN levels in age-matched quadrupedal mice. Whereas OPN could be detected neither in quadrupedal nor in bipedal OPN^{-/-} mice (data not shown), bipedal WT showed significantly higher plasma OPN than quadrupedal WT mice at all indicated time points. The maximum values were observed at the thirty-sixth postoperative week.

### EXAMPLE 3

### OPN deficiency improves Gi protein-mediated receptor signal transduction

It was next determined whether lack of OPN can influence Gi protein-mediated signaling. For this purpose, osteoblasts from WT and OPN^{-/-} mice were screened for their response to DAMGO, somatostatin, oxymethazoline and apelin to activate opioid, somatostatin, alpha2-adrenergic and APJ receptors, respectively. These receptors are well known to mediate signal transduction through Gi proteins. Results illustrated in **Fig. 1**, **F-I** show that all four compounds caused a concentration-dependent increase of response in osteoblasts from WT and OPN^{-/-} mice. However, in each case, the magnitude of response was greater in OPN^{-/-} than in WT osteoblasts, which suggests that the activation of Gi proteins is facilitated in OPN^{-/-} osteoblasts. Also, while the magnitude of response was similar in osteoblasts from quadrupedal and bipedal OPN^{-/-} mice, a significant difference was observed between the responses from quadrupedal and bipedal WT mice (Data not shown). The extent of response was much lower in osteoblasts from bipedal WT mice.

To corroborate these results with the function of Gi proteins, osteoblasts were treated with pertussis toxin (PTX), which ADP-rybosylates Gi proteins and disrupts their interaction with receptors (Gilman, 1987); (Moss et al., 1984). Results illustrated in **Fig. 1**, **G-M** show that cellular responses to each of the four tested agonists, were largely reduced by PTX pre-treatment in osteoblasts from either phenotype. However, the extent of reduction was higher in OPN^{-/-} osteoblasts. Notably, the differences in the magnitude of response between WT and OPN^{-/-} osteoblasts were completely abrogated by PTX treatment. Collectively, these data indicate that OPN deficiency enhances Gi protein-mediated receptor signal transduction in osteoblasts cells.

To determine whether loss of OPN specifically enhances Gi protein signaling, we examined the effect of loss of OPN on signaling initiated by other G proteins such as Gs and Gq. We used isoproterenol and desmopressin to activate Gs through beta-adrenergic and vasopressin receptors, respectively; while bradykinin and endothelin-1 were used to activate Gq through their cognate receptors. Results in **Fig. 1**, **N and O** show that osteoblasts from OPN^{-/-} mice were less responsive to Gs stimulation than those from WT mice, whereas the Gq stimulation elicited in WT and OPN^{-/-} osteoblasts with comparable magnitude. These findings are strongly indicative that OPN deficiency exclusively improves Gi protein-mediated signaling.

### EXAMPLE 4

### Extracellular OPN disrupts Gi protein-mediated receptor signal transduction

To further investigate the role of OPN in Gi protein-mediated receptor signaling, several lines of experiments were performed using MC3T3-E1 osteoblastic cell line. Since these cells express OPN, was first examined whether depletion of OPN by siRNA influences the capacity of GiPCR ligands to induce cell signaling as measured by CDS in MC3T3-E1 cells. Results illustrated in **Fig. 2** **A** show that the integrated response to DAMGO was significantly greater in cells depleted of OPN. Similar results were obtained when cells were stimulated with oxymethazoline. Interestingly, blockade of secreted OPN, by exposing cells to neutralizing OPN antibody, also increased response to both compounds (**Fig. 2** **B**). These results suggest that the extracellular OPN disrupts GiPCR signaling. To confirm this hypothesis, cells were treated with exogenous recombinant OPN (rOPN) prior to DAMGO and oxymethazoline stimulation. In each case, rOPN caused decrease in the integrated response in a concentration-dependent manner, which was prevented by OPN antibody (**Fig. 2**, **C-D**).

### EXAMPLE 5

### CD44 is not involved in the inhibition of GiPCR signaling by extracellular OPN

OPN is known to function by interacting with a variety of cell surface receptors, including CD44 and many integrins (Weber et al., 1996); (Rangaswami et al., 2006). Since CD44 is considered as a key receptor for OPN, was first explored whether CD44 is responsible for the inhibitory effect of OPN on GiPCR signaling. For this purpose, the interaction between OPN and CD44 was interfered, using CD44 antibody. As shown in **Fig. 3** **A**, OPN reduced the integrated response to DAMGO in cell pre-treated with IgG control or CD44 antibody. Similar results were obtained when cells were stimulated with oxymethazoline (**Fig. 3** **A**), suggesting that the blockade of CD44 did not prevent the GiPCR signaling reduction induced by OPN and even potentiated its effect. To exclude the possibility that the lack of prevention of OPN inhibition by CD44 antibody was due to its inefficacy, the cells pre-treated with IgG control or CD44 antibody were stimulated with increasing concentrations of hyaluronic acid (HA), a high affinity ligand of CD44. As shown in **Fig. 3** **B**, the integrated response induced by HA was completely abrogated by pre-treatment with CD44 antibody. Moreover, the effect of CD44 antibody on response to HA stimulation was concentration-dependent (**Fig. 3** **C**). These data clearly indicate that CD44 antibody was active.

The small interfering RNA (siRNA) approach was further used to knockdown the expression of CD44, and the efficiency of siRNA transfection was demonstrated by quantitative real-time PCR and Western blot analysis (**Fig. 3**, **D and E**). The deletion of CD44 by siRNA did not prevent the inhibitory effect of OPN on response to DAMGO or oxymethazoline (**Fig. 3**, **F**). Consistent with these findings, rOPN treatment caused a concentration-dependent decrease in response to both DAMGO and oxymethazoline in osteoblasts from bipedal CD44 knockout (CD44^{-/-}) mice (**Fig. 3****, G and H**). Moreover, these osteoblasts were less responsive to DAMGO or oxymethazoline when compared with osteoblasts from quadrupedal (CD44^{-/-}) mice (**Fig. 3****, I and J**)**.**

Further experiments in wild type and CD44-/- cells in the presence or absence of HA confirmed that the inhibitory effect of OPN is exacerbated in the absence of CD44 and intensified in the presence of HA, a known CD44 ligand (**Figure 3** **K to P**). CD44 is not the cause of GiPCR signaling defect, rather this effect is due to OPN. However, since CD44 binds to OPN, inhibition of CD44 or its effects (by HA, CD44 antibody, anti-CD44 siRNA or using CD44-/- cells) exacerbates the damaging effects of OPN.

Collectively, these data indicate without ambiguity that CD44 is not involved in the inhibition of GiPCR signaling by OPN and its presence reduces the effect of OPN on GiPCR signaling.

### EXAMPLE 6

### RGD-dependent integrins mediate the inhibitory effect of OPN on GiPCR signaling

Was next examined the possible requirement of integrins. Given that OPN contains an arginine-glycine-aspartate (RGD)-motif that engages a subset of cell surface integrins and a serine-valine-valine-tyrosine-glutamate-leucine-arginine (SVVYGLR)-containing domain that interacts with other integrins, it was of interest to examine whether OPN action required a specific domain-dependent integrin. For this purpose, small synthetic peptides were used to compete with integrin binding of the RGD or SVVYLRG sequences in OPN. BIO1211 was used to selectively inhibit α₄β₁ integrin, the only SVVYLRG-containing integrin present in osteoblasts. As shown in **Fig. 4** **A**, OPN action on GiPCR signaling was not significantly influenced by BIO1211 in MC3T3-E1 cells. Response to DAMGO or oxymethazoline was reduced by rOPN in the absence or presence of BIO1211, suggesting that integrins with SVVYLRG recognition specificity are not entailed in this process. In contrast, coincubation of cells with rOPN and RDG peptide completely prevented the inhibitory effect of rOPN on response to DAMGO and oxymethazoline. **Fig. 4****, B and C** illustrate that this preventive effect of RDG was concentration-dependent. Increasing concentrations of RGD resulted in a progressive attenuation of the inhibitory effect of OPN, reversing the effect of OPN when concentrations went beyond 10µg/ml. Similarly, incubation of osteoblasts from WT mice with high concentrations of RGD demonstrated significant increase of response to DAMGO or oxymethazoline (**Fig. 4**, **D**), suggesting efficient inhibition of the effect of secreted OPN by RGD. Consistent with this view, RGD was without effect when osteoblasts from OPN^{-/-} mice were used (**Fig. 4**, **E**), indicating that the effect of RGD is strictly dependent on the presence of OPN.

Collectively, these data suggest that the inhibitory effect of OPN on GiPCR signaling is mediated by one or more RGD-dependent integrin expressed in osteoblasts.

### EXAMPLE 7

### Identification of integrins involved in the inhibition of GiPCR signaling by OPN

Having established a probable role for RGD-dependent integrins in the inhibitory effect of OPN on GiPCR signaling in osteoblasts, specific integrins were examined to determine whether they could mediate this effect. For this purpose, antibodies were used to selectively neutralize the subunits of αᵥβ₁, αᵥβ₃, αᵥβ₅, α₅β₁, and α₈β₁, the RGD-dependent integrins that have been shown to be expressed in osteoblasts (Hughes et al., 1997); (Gronthos et al., 1997); (Grzesik and Robey, 1994); (Clover et al., 1992); (Moursi et al., 1997); (Pistone et al., 1996). **Fig. 5**, **A** and **B** show that the inhibitory effect of rOPN on response to DAMGO and oxymethazoline was attenuated at different degrees, but not abolished, by β₃, β₅, αᵥ and α₈ antibodies. Conversely, antibodies against α₅ or β₁ reversed the inhibitory effect of rOPN on response to both DAMGO and oxymethazoline. Cells pre-treated with these antibodies showed an increase in the magnitude of the integrated response in the presence of rOPN compared to untreated cells, in sharp contrast to the decrease induced by rOPN in cell pre-treated with IgG. These results suggest that α₅ and β₁ integrins are the primary mediators of the inhibitory effect of OPN on GiPCR signaling.

To further support this hypothesis, the siRNA approach was used. When compared with untreated cells, MC3T3-E1 transfected with scrambled siRNA demonstrated less response, while cells transfected with α₅ or β₁ integrins siRNA demonstrated high response in the presence of rOPN. Cells transfected with both α₅ and β₁ integrin siRNA demonstrated a further increase (**Fig. 5** **C**). Also, silencing of both integrin subunits simultaneously resulted in increased response to DAMGO and oxymethazoline in osteoblasts from bipedal WT and CD44^{-/-} mice (**Fig. 5**, **D and E**). Effective silencing of integrin expression was supported by q-RT-PCR (**Fig. 5****, F**). In all cases, transfection of cells with other integrins resulted in a partial attenuation of the inhibitory effect of OPN on GiPCR signaling (**data not shown**). Taken together, these results are the convincing evidence that α₅β₁ is the main integrin dimmer involved in mediating the inhibitory effect of OPN on GiPCR signaling in osteoblasts.

### EXAMPLE 8

### OPN is without effect on expression of Gi proteins and their cognate receptors

Given that the level of GPCRs is critical for their signaling, it was of interest to examine whether the defective GiPCR signaling induced by OPN is the consequence of a quantitative reduction of these receptors. For this purpose, MC3T3-E1 cells were treated with PBS or rOPN and the cell lysates were analyzed by Western blot for the expression of three different GPCRs; including mu-opioid (MOR), lysophosphatidic acid type 1 (LPA1R) and melatonin type 2 (MT2R) receptors. As showed in **Fig. 6** **A**, all three receptors were immunodetected in MC3T3-E1 cells and the level of each receptor was not modified by rOPN treatment. Consequently, the effect of rOPN on the expression of Gi proteins was examined. Relative to PBS-treated cells, there was no significant effect of rOPN treatment on the quantity of Gi proteins as determined by immunoblotting using antibodies against Gi₁, Gi₂ and Gi₃ isoforms (**Fig. 6** **B**). The qPCR analysis also revealed no significant difference in the mRNA expression of any of these isoforms between PBS- and rOPN-treated cells (**Fig. 6** **C**). These results exclude the possibility that rOPN reduces the expression of receptors or Gi proteins.

### Example 9

### OPN reduces the availability of Gi proteins for their cognate receptors

Gi proteins have been shown to be recruited by β₁ integrins via an adaptor molecule and to mediate integrin signaling (Green et al., 1999). It is notable that when different receptors signal through the same subfamily of G proteins, they share the same limited pool of G proteins. Therefore, if OPN enhances the recruitment of Gi proteins in the β₁ integrin complex, reduction in the amount of Gi proteins in GiPCR complex would be expected. To evaluate this possibility, cells lysates were immunoprecipitated with antibodies against MO, MT2 or β₁ integrin receptors, and the presence of Gi proteins in each precipitate was examined by Western blot using antibodies specific for Gi₁, Gi₂ or Gi₃ isoform. **Fig. 6** **D**, **E and F** shows that the relative amount of Gi₁ isoform immunoprecipitated with MO or MT2 receptor was reduced in rOPN-treated cells compared to PBS-treated cells. Similar observations were noted for Gi₂ and Gi₃ isoforms. In contrast, the amount of each of these Gi protein isoforms was elevated in the β₁ integrin precipitates following rOPN treatment. These results suggest that OPN causes a kidnapping of Gi proteins and reduces their availability for GiPCRs.

### Example 10

### OPN enhances the phosphorylation of Gi proteins

Given that the defective GiPCR signaling associated with idiopathic scoliosis has causatively been related to increased phosphorylation of Gi proteins (Moreau et al., 2004), it was of interest to examined whether OPN influences the phosphorylation status of Gi proteins. Accordingly, MC3T3-E1 cells were treated with rOPN, then cell lysates were immunoprecipitated with antibodies against Gi₁, Gi₂ or Gi₃ protein isoforms, and the phosphorylation level was examined by Western blot using anti-phospho serine/threonine or anti-tyrosine antibodies. Results revealed the presence of phosphorylated serine and tyrosine residues in Gi₁ precipitates obtained from cells treated with PBS or rOPN. However, band density was higher in OPN-treated cells. Similar observations were noted in Gi₂ and Gi₃ precipitates (**Fig. 7** **A**). These results show that OPN enhances the phosphorylation of Gi proteins at serine and tyrosine residues.

To support the involvement of α₅β₁ integrin in this process, cells were pre-treated with antibody against α₅β₁ integrin prior to rOPN treatment. Western blot analysis revealed that the Gi phosphorylation induced by rOPN treatment was attenuated by anti-α₅β₁, integrin blocking antibody (**Fig. 7** **A**).

To further associate these findings with the decreased GiPCR signaling in scoliosis, Gi proteins were examined directly to determine whether they undergo increased phosphorylation in osteoblasts from scoliotic mice. The immunoprecipitated of Gi₁, Gi₂ and Gi₃ proteins were probed with an anti-phospho-serine/threonine or an anti-phospho-tyrosine specific antibody and it was observed that the level of phosphorylation of each Gi isoform was significantly greater in osteoblasts from scoliotic bipedal WT or CD44^{-/-} mice compared with those from quadrupedal control mice (**Fig. 7B**). Importantly, phosphorylation levels were attenuated by anti-α₅β₁ integrin blocking antibody (data not shown). These results provide convincing evidence that GiPCR signaling in scoliotic mice is associated with Gi protein phosphorylation induced by OPN via α₅β₁ integrin engagement.

### Example 11

### Phosphorylation of Gi proteins induced by OPN involves various kinases

To identify which kinases are involved in the phosphorylation of Gi proteins induced by OPN, FAK, MEK, ERK1/2, P38, JNK, PI3K, Src, PKC and CaMKII were pharmacologically inhibited. Cell extracts prepared from MC3T3-E1 that had been treated with rOPN in the presence or absence of an inhibitor specific for each kinase were subjected to immunoprecipitation and Western blot analysis. Results in **Fig. 7C-E** show that an inhibitor of PKC (Gö6983) attenuated level of serine phosphorylation in the Gi protein precipitates but was without effect on tyrosine phosphorylation levels. However, both serine and tyrosine phosphorylation were attenuated by inhibitors of FAK (FAK inhibitor-14), Scr (PP2), CaMKII (KN93), PI3K (wortmannin-data not shown), MEK (PD98059), ERK1/2 (FR180204), JNK (SP60125) and P38 (SB203580). These results indicate that various kinases are directly or indirectly involved in the phosphorylation of Gi proteins induced by OPN.

### Example 12

### The effects of OPN on Gi and Gs proteins favour GsPCR signaling

Several reports indicate that inhibition of Gi proteins disrupts signal from GiPCR and enhances GsPCR signaling (Itoh et al., 1984); (Katada et al., 1985); (Wesslau and Smith, 1992). On the other hand, it has been demonstrated that the tyrosine phosphorylation of Gs protein by Src also enhances GsPCR signaling, presumably by increasing activity of Gs protein and its association with receptor (Hausdorff et al., 1992); (Chakrabarti and Gintzler, 2007).

Taken into account these considerations and having observed that genetic deletion of OPN inversely influenced response to Gi and Gs stimulation (**Fig. 1**), it was of interest to examine the effect of OPN on the functional and phosphorylation status of Gs protein and on its interaction with receptors. For this purpose, cells were subjected to isoproterenol and desmopressin in the presence of rOPN or PBS. As expected, response to isoproterenol was higher in rOPN than in PBS-treated cells. Similar results were obtained when cells were stimulated with desmopressin, indicating that OPN increases response to GsPCR stimulation (**Fig. 8** **A**). Interestingly, immunoprecipitates of Gs proteins probed with anti-tyrosine antibody exhibited higher intensity in rOPN-treated cells compared to PBS-treated cells (**Fig. 8** **B**). More interestingly, rOPN treatment enhances the presence of Gs proteins in the immunoprecipitates of MT2 and MO receptors (**Fig. 8C-D**).

Collectively, these results demonstrate that OPN enhances not only the GsPCR signaling, but also the tyrosine phosphorylation of Gs protein and its association with receptors.

Assuming that inhibition of Gi protein and phosphorylation of Gs protein are responsible for the increased GsPCR signaling associated with rOPN, the next experiments aimed to determine the contribution of each parameter. For this purpose, cells were treated with PTX to mimic disruption action of rOPN and compared to cells treated with rOPN alone or in combination with Src inhibitor (PP2) to prevent tyrosine phosphorylation. Results in **Fig. 8** **E** show that rOPN-treated cells were more responsive to isoproterenol stimulation than PTX-treated cells by 30 %. This modest difference was abolished by Src inhibitor (PP2). Similar observations were noted when cells were stimulated with desmopressin. These results indicate that Gs phosphorylation contributes only part of the stimulatory effect of rOPN on GsPCR signaling and that disruption of the inhibitory signal from GiPCR represents the main cause of this phenomenon.

### Example 13:

### Identification of a CT SNP in AIS subjects associated with increased risk of spinal deformity progression and effect on GiPCR signaling

A Whole Exome Sequencing (WES) study was performed on cell samples from AIS subjects and a SNP (rs1467558) was identified in AIS subjects with severe AIS, which changes the amino acid isoleucine 230 to threonine in the CD44 coding sequence.

Next, the effect of the CT SNP on GiPCR signaling was assessed. Osteoblasts from severe AIS patients (who underwent surgery) carrying the CT SNP and osteoblasts from healthy controls with wild type CD44 (CC) were stimulated with LPA in the presence of recombinant OPN (rOPN). The GiPCR defective signaling effect in response to the same rOPN treatment was reduced 50% more in mutated CD44 (CT) osteoblasts as compared to in wild type CD44 (CC) osteoblasts (**Fig. 9** **A**). Comparable results have been obtained using different concentrations of OPN. The CT mutation in CD44 thus increases the sensitivity of scoliotic patients to the damaging effects of OPN on GiPCR signaling and thereby contributes the more severe phenotype observed in these subjects.

On the basis of findings presented herein and the available evidences, and without being so limited, a hypothetical scenario schematized in **Fig. 9**, explains the mechanism by which OPN reduces GiPCR signaling and contributes to the development of spinal deformity. According to the proposed mechanism, upon OPN binding, α₅β₁ integrin recruits a part of Gi proteins from the common pool, then promotes different signaling pathways leading to the activation of various kinases, which directly or indirectly phosphorylate a part of the remaining Gi proteins in the common pool. These events simultaneously cause depletion of pool and inactivation of different isoforms, leading to the reduction in the amount of the functional Gi proteins necessary for the efficient GiPCR signaling. This would reduce the inhibitory control on Gi protein and favour GsPCR signaling, which is exacerbated by phosphorylation of Gs protein by Src. The exaggerated GsPCR signaling increases bone formation (Hsiao et al., 2008) and reduce myoblast proliferation (Marchal et al., 1995), leading to an imbalance between bone mass and muscular strength around the spine. Thus, spine will be throwing out of the balance and the lateral curvature will appear. Since signal transduction through Gs proteins enhances OPN mRNA and protein levels (Nagao et al., 2011), the exaggerated GsPCR signaling would sustain the high production of OPN and amplify events driving the development of spinal deformity. Although additional mechanisms could be operable, the contribution of GsPCR signaling is further supported by the addition of spinal deformity to the constellation of orthopaedic problems commonly associated with Fibrous Dysphasia of bone, an uncommon disease caused by congenital mutation in Gs protein (Leet et al., 2004).

Results presented herein demonstrate that absence of CD44 expression potentiates the effect of OPN and further reduces GiPCR signalization in cells (**Figure 3**). Furthermore, HA was shown to potentiate this effect. CD44 is known to bind to OPN and HA. Finally, in AIS subjects with severe scoliosis (without being bound to any particular theory), CD44 could act by sequestering a portion of the OPN pool available to interact with the integrin receptor. Under circumstances where less CD44 is available to bind to OPN (e.g., under circumstances where expression levels of CD44 are reduced, where OPN's affinity for CD44 is reduced or where binding to OPN is blocked (e.g., by an antibody specific for CD44 or by another ligand such as HA)), bioavailability of OPN to α₅β₁ is increased and GiPCR signalization is further reduced. Conversely, increasing CD44/sCD44 expression (availability to bind to OPN) could contribute to reduce OPN's binding to integrins and increase GiPCR signalization in cells.

The scope of the claims should not be limited by the preferred embodiments set forth in the examples but should be given the broadest interpretation consistent with the description as a whole.

### REFERENCES:

1. Kane, W. J. (1977) Scoliosis prevalence: a call for a statement of terms. Clinical orthopaedics and related research, 43-46.
2. Dickson, R. A. (1992) The etiology and pathogenesis of idiopathic scoliosis. Acta orthopaedica Belgica 58 Suppl 1, 21-25.
3. Machida, M. (1999) Cause of idiopathic scoliosis. Spine 24, 2576-2583.
4. Burwell, R. G. (2003) Aetiology of idiopathic scoliosis: current concepts. Pediatric rehabilitation 6, 137-170.
5. Aherrahrou, Z., S.B. Axtner, P.M. Kaczmarek, A. Jurat, S. Korff, L.C. Doehring, D. Weichenhan, H.A. Katus, and B.T. Ivandic. 2004. A locus on chromosome 7 determines dramatic up-regulation of osteopontin in dystrophic cardiac calcification in mice. The American journal of pathology 164:1379-1387.
6. Akoume, M.Y., B. Azeddine, I. Turgeon, A. Franco, H. Labelle, B. Poitras, C.H. Rivard, G. Grimard, J. Ouellet, S. Parent, and A. Moreau. 2010. Cell-based screening test for idiopathic scoliosis using cellular dielectric spectroscopy. Spine (Phila Pa 1976) 35:E601-608.
7. Azeddine, B., K. Letellier, S. Wang da, F. Moldovan, and A. Moreau. 2007. Molecular determinants of melatonin signaling dysfunction in adolescent idiopathic scoliosis. Clinical orthopaedics and related research 462:45-52.
8. Berg, K.A., G. Zardeneta, K.M. Hargreaves, W.P. Clarke, and S.B. Milam. 2007. Integrins regulate opioid receptor signaling in trigeminal ganglion neurons. Neuroscience 144:889-897.
9. Brown, E.J., and W.A. Frazier. 2001. Integrin-associated protein (CD47) and its ligands. Trends Cell Biol 11:130-135.
10. Bushfield, M., B.E. Lavan, and M.D. Houslay. 1991. Okadaic acid identifies a phosphorylation/dephosphorylation cycle controlling the inhibitory guanine-nucleotide-binding regulatory protein Gi2. The Biochemical journal 274 (Pt 2):317-321.
11. Chakrabarti, S., and A.R. Gintzler. 2007. Phosphorylation of Galphas influences its association with the micro-opioid receptor and is modulated by long-term morphine exposure. Molecular pharmacology 72:753-760.
12. Chiba, S., M.M. Rashid, H. Okamoto, H. Shiraiwa, S. Kon, M. Maeda, M. Murakami, M. Inobe, A. Kitabatake, and A.F. Chambers. 1999. The role of osteopontin in the development of granulomatous lesions in lung. Microbiology and immunology 44:319-332.
13. Clover, J., R.A. Dodds, and M. Gowen. 1992. Integrin subunit expression by human osteoblasts and osteoclasts in situ and in culture. Journal of cell science 103 (Pt 1):267-271.
14. Denhardt, D., E. Burger, C. Kazanecki, S. Krishna, C. Semeins, and J. Klein-Nulend. 2001a. Osteopontin-deficient bone cells are defective in their ability to produce NO in response to pulsatile fluid flow. Biochemical and biophysical research communications 288:448-453.
15. Denhardt, D.T., C.M. Giachelli, and S.R. Rittling. 2001b. Role of osteopontin in cellular signaling and toxicant injury. Annual review of pharmacology and toxicology 41:723-749.
16. Denhardt, D.T., and X. Guo. 1993. Osteopontin: a protein with diverse functions. FASEB journal : official publication of the Federation of American Societies for Experimental Biology 7:1475-1482.
17. Denhardt, D.T., M. Noda, A.W. O'Regan, D. Pavlin, and J.S. Berman. 2001c. Osteopontin as a means to cope with environmental insults: regulation of inflammation, tissue remodeling, and cell survival. The Journal of clinical investigation 107:1055-1061.
18. Fitzpatrick, L., A. Severson, W. Edwards, and R. Ingram. 1994. Diffuse calcification in human coronary arteries. Association of osteopontin with atherosclerosis. Journal of Clinical Investigation 94:1597.
19. Giachelli, C.M., N. Bae, M. Almeida, D.T. Denhardt, C.E. Alpers, and S.M. Schwartz. 1993. Osteopontin is elevated during neointima formation in rat arteries and is a novel component of human atherosclerotic plaques. The Journal of clinical investigation 92:1686-1696.
20. Giachelli, C.M., L. Liaw, C.E. Murry, S.M. Schwartz, and M. Almeida. 1995. Osteopontin expression in cardiovascular diseases. Annals of the New York Academy of Sciences 760:109-126.
21. Giachelli, C.M., R. Pichler, D. Lombardi, D.T. Denhardt, C.E. Alpers, S.M. Schwartz, and R.J. Johnson. 1994. Osteopontin expression in angiotensin II-induced tubulointerstitial nephritis. Kidney international 45:515-524.
22. Gilman, A.G. 1987. G proteins: transducers of receptor-generated signals. Annual review of biochemistry 56:615-649.
23. Green, J.M., A. Zhelesnyak, J. Chung, F.P. Lindberg, M. Sarfati, W.A. Frazier, and E.J. Brown. 1999. Role of cholesterol in formation and function of a signaling complex involving alphavbeta3, integrin-associated protein (CD47), and heterotrimeric G proteins. The Journal of cell biology 146:673-682.
24. Gronthos, S., K. Stewart, S.E. Graves, S. Hay, and P.J. Simmons. 1997. Integrin Expression and Function on Human Osteoblast-like Cells. Journal of Bone and Mineral Research 12:1189-1197.
25. Grzesik, W.J., and P.G. Robey. 1994. Bone matrix RGD glycoproteins: immunolocalization and interaction with human primary osteoblastic bone cells in vitro. Journal of bone and mineral research: the official journal of the American Society for Bone and Mineral Research 9:487-496.
26. Hausdorff, W.P., J.A. Pitcher, D.K. Luttrell, M.E. Linder, H. Kurose, S.J. Parsons, M.G. Caron, and R.J. Lefkowitz. 1992. Tyrosine phosphorylation of G protein alpha subunits by pp60c-src. Proceedings of the National Academy of Sciences of the United States of America 89:5720-5724.
27. Hsiao, E.C., B.M. Boudignon, W.C. Chang, M. Bencsik, J. Peng, T.D. Nguyen, C. Manalac, B.P. Halloran, B.R. Conklin, and R.A. Nissenson. 2008. Osteoblast expression of an engineered Gs-coupled receptor dramatically increases bone mass. Proceedings of the National Academy of Sciences 105:1209-1214.
28. Hughes, P.E., M.W. Renshaw, M. Pfaff, J. Forsyth, V.M. Keivens, M.A. Schwartz, and M.H. Ginsberg. 1997. Suppression of integrin activation: a novel function of a Ras/Raf-initiated MAP kinase pathway. Cell 88:521-530.
29. Illario, M., V. Amideo, A. Casamassima, M. Andreucci, T. di Matola, C. Miele, G. Rossi, G. Fenzi, and M. Vitale. 2003. Integrin-dependent cell growth and survival are mediated by different signals in thyroid cells. The Journal of clinical endocrinology and metabolism 88:260-269.
30. Itoh, H., F. Okajima, and M. Ui. 1984. Conversion of adrenergic mechanism from an alpha- to a beta-type during primary culture of rat hepatocytes. Accompanying decreases in the function of the inhibitory guanine nucleotide regulatory component of adenylate cyclase identified as the substrate of islet-activating protein. The Journal of biological chemistry 259:15464-15473.
31. Katada, T., A.G. Gilman, Y. Watanabe, S. Bauer, and K.H. Jakobs. 1985. Protein kinase C phosphorylates the inhibitory guanine-nucleotide-binding regulatory component and apparently suppresses its function in hormonal inhibition of adenylate cyclase. European journal of biochemistry / FEBS 151:431-437.
32. Katagiri, Y.U., J. Sleeman, H. Fujii, P. Herrlich, H. Hotta, K. Tanaka, S. Chikuma, H. Yagita, K. Okumura, M. Murakami, I. Saiki, A.F. Chambers, and T. Uede. 1999. CD44 variants but not CD44s cooperate with beta1-containing integrins to permit cells to bind to osteopontin independently of arginine-glycine-aspartic acid, thereby stimulating cell motility and chemotaxis. Cancer research 59:219-226.
33. Leet, A.I., E. Magur, J.S. Lee, S. Wientroub, P.G. Robey, and M.T. Collins. 2004. Fibrous dysplasia in the spine: prevalence of lesions and association with scoliosis. The Journal of Bone & Joint Surgery 86:531-537.
34. Liaw, L., D.E. Birk, C.B. Ballas, J.S. Whitsitt, J.M. Davidson, and B.L. Hogan. 1998. Altered wound healing in mice lacking a functional osteopontin gene (spp1). The Journal of clinical investigation 101:1468-1478.
35. Machida, M., J. Dubousset, T. Yamada, J. Kimura, M. Saito, T. Shiraishi, and M. Yamagishi. 2006. Experimental scoliosis in melatonin-deficient C57BL/6J mice without pinealectomy. Journal of pineal research 41:1-7.
36. Machida, M., I. Murai, Y. Miyashita, J. Dubousset, T. Yamada, and J. Kimura. 1999. Pathogenesis of idiopathic scoliosis. Experimental study in rats. Spine (Phila Pa 1976) 24:1985-1989.
37. Marchal, S., I. Cassar-Malek, J.P. Magaud, J.P. Rouault, C. Wrutniak, and G. Cabello. 1995. Stimulation of avian myoblast differentiation by triiodothyronine: possible involvement of the cAMP pathway. Experimental cell research 220:1-10.
38. Moreau, A., S. Forget, B. Azeddine, D. Angeloni, F. Fraschini, H. Labelle, B. Poitras, C.-H. Rivard, and G. Grimard. 2004. Melatonin signaling dysfunction in adolescent idiopathic scoliosis. Spine 29:1772-1781.
39. Moss, J., P. Bruni, J.A. Hsia, S.C. Tsai, P.A. Watkins, J.L. Halpern, D.L. Burns, Y. Kanaho, P.P. Chang, E.L. Hewlett, and et al. 1984. Pertussis toxin-catalyzed ADP-ribosylation: effects on the coupling of inhibitory receptors to the adenylate cyclase system. Journal of receptor research 4:459-474.
40. Moursi, A.M., R.K. Globus, and C.H. Damsky. 1997. Interactions between integrin receptors and fibronectin are required for calvarial osteoblast differentiation in vitro. Journal of cell science 110 (Pt 18):2187 -2196.
41. Murry, C.E., C.M. Giachelli, S.M. Schwartz, and R. Vracko. 1994. Macrophages express osteopontin during repair of myocardial necrosis. The American journal of pathology 145:1450-1462.
42. Murthy, K.S., J.R. Grider, and G.M. Makhlouf. 2000. Heterologous desensitization of response mediated by selective PKC-dependent phosphorylation of G(i-1) and G(i-2). American journal of physiology. Cell physiology 279:C925-934.
43. Nagao, M., T.N. Feinstein, Y. Ezura, T. Hayata, T. Notomi, Y. Saita, R. Hanyu, H. Hemmi, Y. Izu, S. Takeda, K. Wang, S. Rittling, T. Nakamoto, K. Kaneko, H. Kurosawa, G. Karsenty, D.T. Denhardt, J.P. Vilardaga, and M. Noda. 2011. Sympathetic control of bone mass regulated by osteopontin. Proceedings of the National Academy of Sciences of the United States of America 108:17767-17772.
44. O'Brien, K.D., J. Kuusisto, D.D. Reichenbach, M. Ferguson, C. Giachelli, C.E. Alpers, and C.M. Otto. 1995. Osteopontin is expressed in human aortic valvular lesions. Circulation 92:2163-2168.
45. Oates, A., R. Barraclough, and P. Rudland. 1997. The role of osteopontin in tumorigenesis and metastasis. Invasion & metastasis 17:1.
46. Ophascharoensuk, V., C.M. Giachelli, K. Gordon, J. Hughes, R. Pichler, P. Brown, L. Liaw, R. Schmidt, S.J. Shankland, C.E. Alpers, W.G. Couser, and R.J. Johnson. 1999. Obstructive uropathy in the mouse: role of osteopontin in interstitial fibrosis and apoptosis. Kidney international 56:571-580.
47. Oyama, J., I. Murai, K. Kanazawa, and M. Machida. 2006. Bipedal ambulation induces experimental scoliosis in C57BL/6J mice with reduced plasma and pineal melatonin levels. Journal of pineal research 40:219-224.
48. Pistone, M., C. Sanguineti, A. Federici, F. Sanguineti, P. Defilippi, F. Santolini, G. Querze, P.C. Marchisio, and P. Manduca. 1996. Integrin synthesis and utilization in cultured human osteoblasts. Cell biology international 20:471-479.
49. Rangaswami, H., A. Bulbule, and G.C. Kundu. 2006. Osteopontin: role in cell signaling and cancer progression. Trends in cell biology 16:79-87.
50. Reinholt, F.P., K. Hultenby, A. Oldberg, and D. Heinegard. 1990. Osteopontin--a possible anchor of osteoclasts to bone. Proceedings of the National Academy of Sciences of the United States of America 87:4473-4475.
51. Shangguan, Y., K.E. Hall, R.R. Neubig, and J.W. Wiley. 2003. Diabetic neuropathy: inhibitory G protein dysfunction involves PKC-dependent phosphorylation of Goalpha. Journal of neurochemistry 86:1006-1014.
52. Sodek, J., B. Ganss, and M. McKee. 2000. Osteopontin. Critical Reviews in Oral Biology & Medicine 11:279-303.
53. Strassheim, D., and C.C. Malbon. 1994. Phosphorylation of Gi alpha 2 attenuates inhibitory adenylyl cyclase in neuroblastoma/glioma hybrid (NG-108-15) cells. The Journal of biological chemistry 269:14307 -14313.
54. Von Gall, C., A. Lewy, C. Schomerus, B. Vivien-Roels, P. Pevét, H.W. Korf, and J.H. Stehle. 2000. Transcription factor dynamics and neuroendocrine signalling in the mouse pineal gland: a comparative analysis of melatonin-deficient C57BL mice and melatonin-proficient C3H mice. European Journal of Neuroscience 12:964-972.
55. Weber, G.F., S. Ashkar, M.J. Glimcher, and H. Cantor. 1996. Receptor-ligand interaction between CD44 and osteopontin (Eta-1). Science 271:509-512.
56. Wesslau, C., and U. Smith. 1992. The inhibitory GTP-binding protein (Gi) regulates the agonistic property of beta-adrenergic ligands in isolated rat adipocytes. Evidence for a priming effect of cyclic AMP. The Biochemical journal 288 (Pt 1):41-46.
57. Letellier K, Azeddine B, Parent S, Labelle H, Rompré PH, Moreau A, Moldovan F. Estrogen cross-talk with the melatonin signaling pathway in human osteoblasts derived from adolescent idiopathic scoliosis patients. J Pineal Res. 2008 Nov;45(4):383-93.
58. Verdonk E, Johnson K, McGuinness R, Leung G, Chen YW, Tang HR, Michelotti JM, Liu VF. Cellular dielectric spectroscopy: a label-free comprehensive platform for functional evaluation of endogenous receptors.Assay Drug Dev Technol. 2006 Oct;4(5):609-19.

## Claims

1. An inhibitor of integrin α₅β₁ expression and/or activity for use in increasing GiPCR signalization in the cells of a subject in need thereof.

2. The inhibitor for use of claim 1, for further use with an inhibitor of osteopontin (OPN) expression and/ or activity.

3. The inhibitor for use of claim 2, wherein the inhibitor of OPN activity is an OPN antibody.

4. The inhibitor for use of claim 2, wherein the inhibitor of OPN expression is a siRNA specific to OPN.

5. The inhibitor for use of any one of claims 1-4, wherein the inhibitor of α₅β₁ activity is (a) an antibody that binds specifically to integrin subunit α₅; (b) an antibody that binds specifically to integrin subunit β₁ (c) an antibody that binds specifically to integrin subunits α₅β₁; (d) a molecule that specifically blocks the binding of OPN to α₅β₁ integrin; or (e) any combination of at least two of (a) to (d).

6. The inhibitor for use of claim 5, wherein said molecule (d) that specifically blocks the binding of OPN to α₅β₁ integrin is a RGD peptide or derivative thereof.

7. The inhibitor for use of claim 5, wherein said (d) molecule that specifically blocks the binding of OPN to α₅β₁ integrin is a peptide fragment of OPN comprising a RGD motif.

8. The inhibitor for use of claim 7, wherein the peptide fragment of OPN comprises the amino acid sequence GRGDSWYGLRS (SEQ ID NO: 18).

9. The inhibitor for use of any one of claims 1-4, wherein the inhibitor of α₅β₁ expression is a siRNA specific to α₅ or β₁.

10. The inhibitor for use of any one of claims 1-9, further comprising administering to the subject i) an effective amount of sCD44 or a fragment thereof which specifically binds to OPN; or ii) a stimulator of sCD44 and/or CD44 expression.

11. A composition comprising the inhibitor as defined in any one of claims 1 to 9, and a pharmaceutical carrier.

12. The composition for use of claim 11, for increasing GiPCR signaling in cells of a subject in need thereof.

13. The inhibitor for use of any one of claims 1-9, or the composition of claim 11, wherein the subject is a subject diagnosed with a scoliosis or at risk of developing a scoliosis.

14. The inhibitor for use, or composition of claim 13, wherein the scoliosis is an idiopathic scoliosis.

15. The inhibitor for use, or composition of claim 13, wherein the scoliosis is adolescent idiopathic scoliosis (AIS).


SEQUENCE LISTING

<110>  CHU Ste-Justine
       Moreau, Alain
       Akoume Ndong, Marie-Yvonne

<120>  A METHOD OF INCREASING GIPCR SIGNALISATION IN THE CELLS OF A
       SCOLIOTIC SUBJECT

<130>  765/14033.131

<140>  N.D.; Divisional of EP14814185.6
<141>  2014-06-17

<150>  EP 14814185.6
<151>  2014-06-17

<150>  PCT/CA2014/050569
<151>  2014-06-17

<150>  US 61/835,926
<151>  2013-06-17

<160>  26

<170>  PatentIn version 3.5

<210>  1
<211>  18
<212>  DNA
<213>  Artificial sequence

<220>
<223>  Synthetic Construct

<400>  1
ggaaatcgtg cgtgacat                                                    18


<210>  2
<211>  23
<212>  DNA
<213>  Artificial sequence

<220>
<223>  Synthetic Construct

<400>  2
tcatgatgga gttgaaggta gtt                                              23


<210>  3
<211>  20
<212>  DNA
<213>  Artificial sequence

<220>
<223>  Synthetic Construct

<400>  3
agcatcggat ttgagacctg                                                  20

<210>  4
<211>  20
<212>  DNA
<213>  Artificial sequence

<220>
<223>  Synthetic Construct

<400>  4
tgagtccact tggctttctg                                                    20


<210>  5
<211>  19
<212>  DNA
<213>  Artificial sequence

<220>
<223>  Synthetic Construct

<400>  5
atgtgtcaga cctgccttg                                                     19


<210>  6
<211>  21
<212>  DNA
<213>  Artificial sequence

<220>
<223>  Synthetic Construct

<400>  6
ttgtcccgac tttctacctt g                                                  21


<210>  7
<211>  22
<212>  DNA
<213>  Artificial sequence

<220>
<223>  Synthetic Construct

<400>  7
gtccccacag tagacacata tg                                                 22


<210>  8
<211>  20
<212>  DNA
<213>  Artificial sequence

<220>
<223>  Synthetic Construct

<400>  8
tcaactcctc gctttccatg                                                    20


<210>  9
<211>  25
<212>  RNA

<213>  Artificial sequence

<220>
<223>  Synthetic Construct

<400>  9
ccuaagucag caguaggaac auuau                                          25


<210>  10
<211>  25
<212>  RNA
<213>  Artificial sequence

<220>
<223>  Synthetic Construct

<400>  10
ccuaagucag caguaggaac auuau                                          25


<210>  11
<211>  25
<212>  RNA
<213>  Artificial sequence

<220>
<223>  Synthetic Construct

<400>  11
ccuccagcuc auuguugaug cuuau                                          25


<210>  12
<211>  25
<212>  RNA
<213>  Artificial sequence

<220>
<223>  Synthetic Construct

<400>  12
ccgaguaccu gaucaaccug guuca                                          25


<210>  13
<211>  25
<212>  RNA
<213>  Artificial sequence

<220>
<223>  Synthetic Construct

<400>  13
gagaugaaac ugaauuccga gauaa                                          25


<210>  14
<211>  25
<212>  RNA
<213>  Artificial sequence

<220>

<223>  Synthetic Construct

<400>  14
gacuguugag uaugcuccau guaga                                          25


<210>  15
<211>  25
<212>  RNA
<213>  Artificial sequence

<220>
<223>  Synthetic Construct

<400>  15
gaacaaggag ucgucagaaa cucca                                          25


<210>  16
<211>  25
<212>  RNA
<213>  Artificial sequence

<220>
<223>  Synthetic Construct

<400>  16
uccaccaugu cuaugagcuc aucaa                                          25


<210>  17
<211>  25
<212>  RNA
<213>  Artificial sequence

<220>
<223>  Synthetic Construct

<400>  17
ucaggagcag auugcagaau cuuau                                          25


<210>  18
<211>  12
<212>  PRT
<213>  Artificial sequence

<220>
<223>  Synthetic Construct

<400>  18

Gly Arg Gly Asp Ser Val Val Tyr Gly Leu Arg Ser
1               5                   10


<210>  19
<211>  5748
<212>  DNA
<213>  homo sapiens

<400>  19
gagaagaaag ccagtgcgtc tctgggcgca ggggccagtg gggctcggag gcacaggcac      60

cccgcgacac tccaggttcc ccgacccacg tccctggcag ccccgattat ttacagcctc 120

agcagagcac ggggcggggg cagaggggcc cgcccgggag ggctgctact tcttaaaacc 180

tctgcgggct gcttagtcac agcccccctt gcttgggtgt gtccttcgct cgctccctcc 240

ctccgtctta ggtcactgtt ttcaacctcg aataaaaact gcagccaact tccgaggcag 300

cctcattgcc cagcggaccc cagcctctgc caggttcggt ccgccatcct cgtcccgtcc 360

tccgccggcc cctgccccgc gcccagggat cctccagctc ctttcgcccg cgccctccgt 420

tcgctccgga caccatggac aagttttggt ggcacgcagc ctggggactc tgcctcgtgc 480

cgctgagcct ggcgcagatc gatttgaata taacctgccg ctttgcaggt gtattccacg 540

tggagaaaaa tggtcgctac agcatctctc ggacggaggc cgctgacctc tgcaaggctt 600

tcaatagcac cttgcccaca atggcccaga tggagaaagc tctgagcatc ggatttgaga 660

cctgcaggta tgggttcata gaagggcacg tggtgattcc ccggatccac cccaactcca 720

tctgtgcagc aaacaacaca ggggtgtaca tcctcacatc caacacctcc cagtatgaca 780

catattgctt caatgcttca gctccacctg aagaagattg tacatcagtc acagacctgc 840

ccaatgcctt tgatggacca attaccataa ctattgttaa ccgtgatggc acccgctatg 900

tccagaaagg agaatacaga acgaatcctg aagacatcta ccccagcaac cctactgatg 960

atgacgtgag cagcggctcc tccagtgaaa ggagcagcac ttcaggaggt tacatctttt 1020

acaccttttc tactgtacac cccatcccag acgaagacag tccctggatc accgacagca 1080

cagacagaat ccctgctacc actttgatga gcactagtgc tacagcaact gagacagcaa 1140

ccaagaggca agaaacctgg gattggtttt catggttgtt tctaccatca gagtcaaaga 1200

atcatcttca cacaacaaca caaatggctg gtacgtcttc aaataccatc tcagcaggct 1260

gggagccaaa tgaagaaaat gaagatgaaa gagacagaca cctcagtttt tctggatcag 1320

gcattgatga tgatgaagat tttatctcca gcaccatttc aaccacacca cgggcttttg 1380

accacacaaa acagaaccag gactggaccc agtggaaccc aagccattca aatccggaag 1440

tgctacttca gacaaccaca aggatgactg atgtagacag aaatggcacc actgcttatg 1500

aaggaaactg gaacccagaa gcacaccctc ccctcattca ccatgagcat catgaggaag 1560

aagagacccc acattctaca agcacaatcc aggcaactcc tagtagtaca acggaagaaa 1620

cagctaccca gaaggaacag tggtttggca acagatggca tgagggatat cgccaaacac 1680

ccaaagaaga ctcccattcg acaacaggga cagctgcagc ctcagctcat accagccatc 1740

caatgcaagg aaggacaaca ccaagcccag aggacagttc ctggactgat ttcttcaacc 1800

caatctcaca ccccatggga cgaggtcatc aagcaggaag aaggatggat atggactcca 1860

gtcatagtat aacgcttcag cctactgcaa atccaaacac aggtttggtg gaagatttgg 1920

acaggacagg acctctttca atgacaacgc agcagagtaa ttctcagagc ttctctacat 1980
cacatgaagg cttggaagaa gataaagacc atccaacaac ttctactctg acatcaagca 2040
ataggaatga tgtcacaggt ggaagaagag acccaaatca ttctgaaggc tcaactactt 2100
tactggaagg ttatacctct cattacccac acacgaagga aagcaggacc ttcatcccag 2160
tgacctcagc taagactggg tcctttggag ttactgcagt tactgttgga gattccaact 2220
ctaatgtcaa tcgttcctta tcaggagacc aagacacatt ccaccccagt ggggggtccc 2280
ataccactca tggatctgaa tcagatggac actcacatgg gagtcaagaa ggtggagcaa 2340
acacaacctc tggtcctata aggacacccc aaattccaga atggctgatc atcttggcat 2400
ccctcttggc cttggctttg attcttgcag tttgcattgc agtcaacagt cgaagaaggt 2460
gtgggcagaa gaaaaagcta gtgatcaaca gtggcaatgg agctgtggag gacagaaagc 2520
caagtggact caacggagag gccagcaagt ctcaggaaat ggtgcatttg gtgaacaagg 2580
agtcgtcaga aactccagac cagtttatga cagctgatga gacaaggaac ctgcagaatg 2640
tggacatgaa gattggggtg taacacctac accattatct tggaaagaaa caaccgttgg 2700
aaacataacc attacaggga gctgggacac ttaacagatg caatgtgcta ctgattgttt 2760
cattgcgaat cttttttagc ataaaatttt ctactctttt tgttttttgt gttttgttct 2820
ttaaagtcag gtccaatttg taaaaacagc attgctttct gaaattaggg cccaattaat 2880
aatcagcaag aatttgatcg ttccagttcc cacttggagg cctttcatcc ctcgggtgtg 2940
ctatggatgg cttctaacaa aaactacaca tatgtattcc tgatcgccaa cctttccccc 3000
accagctaag gacatttccc agggttaata gggcctggtc cctgggagga aatttgaatg 3060
ggtccatttt gcccttccat agcctaatcc ctgggcattg ctttccactg aggttggggg 3120
ttggggtgta ctagttacac atcttcaaca gaccccctct agaaattttt cagatgcttc 3180
tgggagacac ccaaagggtg aagctattta tctgtagtaa actatttatc tgtgtttttg 3240
aaatattaaa ccctggatca gtcctttgat cagtataatt ttttaaagtt actttgtcag 3300
aggcacaaaa gggtttaaac tgattcataa taaatatctg tacttcttcg atcttcacct 3360
tttgtgctgt gattcttcag tttctaaacc agcactgtct gggtccctac aatgtatcag 3420
gaagagctga gaatggtaag gagactcttc taagtcttca tctcagagac cctgagttcc 3480
cactcagacc cactcagcca aatctcatgg aagaccaagg agggcagcac tgtttttgtt 3540
ttttgttttt tgtttttttt ttttgacact gtccaaaggt tttccatcct gtcctggaat 3600
cagagttgga agctgaggag cttcagcctc ttttatggtt taatggccac ctgttctctc 3660
ctgtgaaagg ctttgcaaag tcacattaag tttgcatgac ctgttatccc tggggcccta 3720
tttcatagag gctggcccta ttagtgattt ccaaaaacaa tatggaagtg ccttttgatg 3780
tcttacaata agagaagaag ccaatggaaa tgaaagagat tggcaaaggg gaaggatgat 3840

gccatgtaga tcctgtttga catttttatg gctgtatttg taaacttaaa cacaccagtg 3900

tctgttcttg atgcagttgc tatttaggat gagttaagtg cctggggagt ccctcaaaag 3960

gttaaaggga ttcccatcat tggaatctta tcaccagata ggcaagttta tgaccaaaca 4020

agagagtact ggctttatcc tctaacctca tattttctcc cacttggcaa gtcctttgtg 4080

gcatttattc atcagtcagg gtgtccgatt ggtcctagaa cttccaaagg ctgcttgtca 4140

tagaagccat tgcatctata aagcaacggc tcctgttaaa tggtatctcc tttctgaggc 4200

tcctactaaa agtcatttgt tacctaaact tatgtgctta acaggcaatg cttctcagac 4260

cacaaagcag aaagaagaag aaaagctcct gactaaatca gggctgggct tagacagagt 4320

tgatctgtag aatatcttta aaggagagat gtcaactttc tgcactattc ccagcctctg 4380

ctcctccctg tctaccctct cccctccctc tctccctcca cttcacccca caatcttgaa 4440

aaacttcctt tctcttctgt gaacatcatt ggccagatcc attttcagtg gtctggattt 4500

ctttttattt tcttttcaac ttgaaagaaa ctggacatta ggccactatg tgttgttact 4560

gccactagtg ttcaagtgcc tcttgttttc ccagagattt cctgggtctg ccagaggccc 4620

agacaggctc actcaagctc tttaactgaa aagcaacaag ccactccagg acaaggttca 4680

aaatggttac aacagcctct acctgtcgcc ccagggagaa aggggtagtg atacaagtct 4740

catagccaga gatggttttc cactccttct agatattccc aaaaagaggc tgagacagga 4800

ggttattttc aattttattt tggaattaaa tacttttttc cctttattac tgttgtagtc 4860

cctcacttgg atatacctct gttttcacga tagaaataag ggaggtctag agcttctatt 4920

ccttggccat tgtcaacgga gagctggcca agtcttcaca aacccttgca acattgcctg 4980

aagtttatgg aataagatgt attctcactc ccttgatctc aagggcgtaa ctctggaagc 5040

acagcttgac tacacgtcat ttttaccaat gattttcagg tgacctgggc taagtcattt 5100

aaactgggtc tttataaaag taaaaggcca acatttaatt attttgcaaa gcaacctaag 5160

agctaaagat gtaatttttc ttgcaattgt aaatcttttg tgtctcctga agacttccct 5220

taaaattagc tctgagtgaa aaatcaaaag agacaaaaga catcttcgaa tccatatttc 5280

aagcctggta gaattggctt ttctagcaga acctttccaa aagttttata ttgagattca 5340

taacaacacc aagaattgat tttgtagcca acattcattc aatactgtta tatcagagga 5400

gtaggagaga ggaaacattt gacttatctg gaaaagcaaa atgtacttaa gaataagaat 5460

aacatggtcc attcaccttt atgttataga tatgtctttg tgtaaatcat ttgttttgag 5520

ttttcaaaga atagcccatt gttcattctt gtgctgtaca atgaccactg ttattgttac 5580

tttgactttt cagagcacac ccttcctctg gtttttgtat atttattgat ggatcaataa 5640

taatgaggaa agcatgatat gtatattgct gagttgaaag cacttattgg aaaatattaa 5700

aaggctaaca ttaaaagact aaaggaaaca gaaaaaaaaa aaaaaaaa 5748

<210> 20
<211> 742
<212> PRT
<213> homo sapiens

<400> 20
   Met Asp Lys Phe Trp Trp His Ala Ala Trp Gly Leu Cys Leu Val Pro
   1 5 10 15
   Leu Ser Leu Ala Gln Ile Asp Leu Asn Ile Thr Cys Arg Phe Ala Gly
   20 25 30
   Val Phe His Val Glu Lys Asn Gly Arg Tyr Ser Ile Ser Arg Thr Glu
   35 40 45
   Ala Ala Asp Leu Cys Lys Ala Phe Asn Ser Thr Leu Pro Thr Met Ala
   50 55 60
   Gln Met Glu Lys Ala Leu Ser Ile Gly Phe Glu Thr Cys Arg Tyr Gly
   65 70 75 80
   Phe Ile Glu Gly His Val Val Ile Pro Arg Ile His Pro Asn Ser Ile
   85 90 95
   Cys Ala Ala Asn Asn Thr Gly Val Tyr Ile Leu Thr Ser Asn Thr Ser
   100 105 110
   Gln Tyr Asp Thr Tyr Cys Phe Asn Ala Ser Ala Pro Pro Glu Glu Asp
   115 120 125
   Cys Thr Ser Val Thr Asp Leu Pro Asn Ala Phe Asp Gly Pro Ile Thr
   130 135 140
   Ile Thr Ile Val Asn Arg Asp Gly Thr Arg Tyr Val Gln Lys Gly Glu
   145 150 155 160
   Tyr Arg Thr Asn Pro Glu Asp Ile Tyr Pro Ser Asn Pro Thr Asp Asp
   165 170 175
   Asp Val Ser Ser Gly Ser Ser Ser Glu Arg Ser Ser Thr Ser Gly Gly
   180 185 190
   Tyr Ile Phe Tyr Thr Phe Ser Thr Val His Pro Ile Pro Asp Glu Asp
   195 200 205
   Ser Pro Trp Ile Thr Asp Ser Thr Asp Arg Ile Pro Ala Thr Thr Leu
   210 215 220
   Met Ser Thr Ser Ala Thr Ala Thr Glu Thr Ala Thr Lys Arg Gln Glu
   225 230 235 240
   Thr Trp Asp Trp Phe Ser Trp Leu Phe Leu Pro Ser Glu Ser Lys Asn
   245 250 255
   His Leu His Thr Thr Thr Gln Met Ala Gly Thr Ser Ser Asn Thr Ile
   260 265 270
   Ser Ala Gly Trp Glu Pro Asn Glu Glu Asn Glu Asp Glu Arg Asp Arg
   275 280 285
   His Leu Ser Phe Ser Gly Ser Gly Ile Asp Asp Asp Glu Asp Phe Ile
   290 295 300
   Ser Ser Thr Ile Ser Thr Thr Pro Arg Ala Phe Asp His Thr Lys Gln
   305 310 315 320
   Asn Gln Asp Trp Thr Gln Trp Asn Pro Ser His Ser Asn Pro Glu Val
   325 330 335
   Leu Leu Gln Thr Thr Thr Arg Met Thr Asp Val Asp Arg Asn Gly Thr
   340 345 350
   Thr Ala Tyr Glu Gly Asn Trp Asn Pro Glu Ala His Pro Pro Leu Ile
   355 360 365
   His His Glu His His Glu Glu Glu Glu Thr Pro His Ser Thr Ser Thr
   370 375 380
   Ile Gln Ala Thr Pro Ser Ser Thr Thr Glu Glu Thr Ala Thr Gln Lys
   385 390 395 400
   Glu Gln Trp Phe Gly Asn Arg Trp His Glu Gly Tyr Arg Gln Thr Pro
   405 410 415
   Lys Glu Asp Ser His Ser Thr Thr Gly Thr Ala Ala Ala Ser Ala His
   420 425 430
   Thr Ser His Pro Met Gln Gly Arg Thr Thr Pro Ser Pro Glu Asp Ser
   435 440 445
   Ser Trp Thr Asp Phe Phe Asn Pro Ile Ser His Pro Met Gly Arg Gly
   450 455 460
   His Gln Ala Gly Arg Arg Met Asp Met Asp Ser Ser His Ser Ile Thr
   465                 470                 475                 480
   Leu Gln Pro Thr Ala Asn Pro Asn Thr Gly Leu Val Glu Asp Leu Asp
                   485                 490                 495
   Arg Thr Gly Pro Leu Ser Met Thr Thr Gln Gln Ser Asn Ser Gln Ser
               500                 505                 510
   Phe Ser Thr Ser His Glu Gly Leu Glu Glu Asp Lys Asp His Pro Thr
           515                 520                 525
   Thr Ser Thr Leu Thr Ser Ser Asn Arg Asn Asp Val Thr Gly Gly Arg
       530                 535                 540
   Arg Asp Pro Asn His Ser Glu Gly Ser Thr Thr Leu Leu Glu Gly Tyr
   545                 550                 555                 560
   Thr Ser His Tyr Pro His Thr Lys Glu Ser Arg Thr Phe Ile Pro Val
                   565                 570                 575
   Thr Ser Ala Lys Thr Gly Ser Phe Gly Val Thr Ala Val Thr Val Gly
               580                 585                 590
   Asp Ser Asn Ser Asn Val Asn Arg Ser Leu Ser Gly Asp Gln Asp Thr
           595                 600                 605
   Phe His Pro Ser Gly Gly Ser His Thr Thr His Gly Ser Glu Ser Asp
       610                 615                 620
   Gly His Ser His Gly Ser Gln Glu Gly Gly Ala Asn Thr Thr Ser Gly
   625                 630                 635                 640
   Pro Ile Arg Thr Pro Gln Ile Pro Glu Trp Leu Ile Ile Leu Ala Ser
                   645                 650                 655
   Leu Leu Ala Leu Ala Leu Ile Leu Ala Val Cys Ile Ala Val Asn Ser
               660                 665                 670
   Arg Arg Arg Cys Gly Gln Lys Lys Lys Leu Val Ile Asn Ser Gly Asn
           675                 680                 685
   Gly Ala Val Glu Asp Arg Lys Pro Ser Gly Leu Asn Gly Glu Ala Ser
       690                 695                 700
   Lys Ser Gln Glu Met Val His Leu Val Asn Lys Glu Ser Ser Glu Thr
   705                 710                 715                 720
   Pro Asp Gln Phe Met Thr Ala Asp Glu Thr Arg Asn Leu Gln Asn Val
                   725                 730                 735
   Asp Met Lys Ile Gly Val
               740

<210>  21
<211>  798
<212>  PRT
<213>  homo sapiens

<400>  21

Met Asn Leu Gln Pro Ile Phe Trp Ile Gly Leu Ile Ser Ser Val Cys
1               5                   10                  15

Cys Val Phe Ala Gln Thr Asp Glu Asn Arg Cys Leu Lys Ala Asn Ala
            20                  25                  30

Lys Ser Cys Gly Glu Cys Ile Gln Ala Gly Pro Asn Cys Gly Trp Cys
        35                  40                  45

Thr Asn Ser Thr Phe Leu Gln Glu Gly Met Pro Thr Ser Ala Arg Cys
    50                  55                  60

Asp Asp Leu Glu Ala Leu Lys Lys Lys Gly Cys Pro Pro Asp Asp Ile
65                  70                  75                  80

Glu Asn Pro Arg Gly Ser Lys Asp Ile Lys Lys Asn Lys Asn Val Thr
                85                  90                  95

Asn Arg Ser Lys Gly Thr Ala Glu Lys Leu Lys Pro Glu Asp Ile Thr
            100                 105                 110

Gln Ile Gln Pro Gln Gln Leu Val Leu Arg Leu Arg Ser Gly Glu Pro
        115                 120                 125

Gln Thr Phe Thr Leu Lys Phe Lys Arg Ala Glu Asp Tyr Pro Ile Asp
    130                 135                 140

Leu Tyr Tyr Leu Met Asp Leu Ser Tyr Ser Met Lys Asp Asp Leu Glu
145                 150                 155                 160

Asn Val Lys Ser Leu Gly Thr Asp Leu Met Asn Glu Met Arg Arg Ile
                165                 170                 175

Thr Ser Asp Phe Arg Ile Gly Phe Gly Ser Phe Val Glu Lys Thr Val
            180                 185                 190

Met Pro Tyr Ile Ser Thr Thr Pro Ala Lys Leu Arg Asn Pro Cys Thr
        195                 200                 205

Ser Glu Gln Asn Cys Thr Ser Pro Phe Ser Tyr Lys Asn Val Leu Ser
    210                 215                 220

Leu Thr Asn Lys Gly Glu Val Phe Asn Glu Leu Val Gly Lys Gln Arg
225                 230                 235                 240

Ile Ser Gly Asn Leu Asp Ser Pro Glu Gly Gly Phe Asp Ala Ile Met
                245                 250                 255

Gln Val Ala Val Cys Gly Ser Leu Ile Gly Trp Arg Asn Val Thr Arg
            260                 265                 270

Leu Leu Val Phe Ser Thr Asp Ala Gly Phe His Phe Ala Gly Asp Gly
        275                 280                 285

Lys Leu Gly Gly Ile Val Leu Pro Asn Asp Gly Gln Cys His Leu Glu
    290                 295                 300

Asn Asn Met Tyr Thr Met Ser His Tyr Tyr Asp Tyr Pro Ser Ile Ala
305                 310                 315                 320

His Leu Val Gln Lys Leu Ser Glu Asn Asn Ile Gln Thr Ile Phe Ala
                325                 330                 335

Val Thr Glu Glu Phe Gln Pro Val Tyr Lys Glu Leu Lys Asn Leu Ile
            340                 345                 350

Pro Lys Ser Ala Val Gly Thr Leu Ser Ala Asn Ser Ser Asn Val Ile
        355                 360                 365

Gln Leu Ile Ile Asp Ala Tyr Asn Ser Leu Ser Ser Glu Val Ile Leu
    370                 375                 380

Glu Asn Gly Lys Leu Ser Glu Gly Val Thr Ile Ser Tyr Lys Ser Tyr
385                 390                 395                 400

Cys Lys Asn Gly Val Asn Gly Thr Gly Glu Asn Gly Arg Lys Cys Ser
                405                 410                 415

Asn Ile Ser Ile Gly Asp Glu Val Gln Phe Glu Ile Ser Ile Thr Ser
            420                 425                 430

Asn Lys Cys Pro Lys Lys Asp Ser Asp Ser Phe Lys Ile Arg Pro Leu
        435                 440                 445

Gly Phe Thr Glu Glu Val Glu Val Ile Leu Gln Tyr Ile Cys Glu Cys
    450                 455                 460

Glu Cys Gln Ser Glu Gly Ile Pro Glu Ser Pro Lys Cys His Glu Gly
465                 470                 475                 480

Asn Gly Thr Phe Glu Cys Gly Ala Cys Arg Cys Asn Glu Gly Arg Val
                485                 490                 495

Gly Arg His Cys Glu Cys Ser Thr Asp Glu Val Asn Ser Glu Asp Met
            500                 505                 510

Asp Ala Tyr Cys Arg Lys Glu Asn Ser Ser Glu Ile Cys Ser Asn Asn
        515                 520                 525

Gly Glu Cys Val Cys Gly Gln Cys Val Cys Arg Lys Arg Asp Asn Thr
    530                 535                 540

Asn Glu Ile Tyr Ser Gly Lys Phe Cys Glu Cys Asp Asn Phe Asn Cys
545                 550                 555                 560

Asp Arg Ser Asn Gly Leu Ile Cys Gly Gly Asn Gly Val Cys Lys Cys
                565                 570                 575

Arg Val Cys Glu Cys Asn Pro Asn Tyr Thr Gly Ser Ala Cys Asp Cys
            580                 585                 590

Ser Leu Asp Thr Ser Thr Cys Glu Ala Ser Asn Gly Gln Ile Cys Asn
        595                 600                 605

Gly Arg Gly Ile Cys Glu Cys Gly Val Cys Lys Cys Thr Asp Pro Lys
    610                 615                 620

Phe Gln Gly Gln Thr Cys Glu Met Cys Gln Thr Cys Leu Gly Val Cys
625                 630                 635                 640

Ala Glu His Lys Glu Cys Val Gln Cys Arg Ala Phe Asn Lys Gly Glu
                645                 650                 655

Lys Lys Asp Thr Cys Thr Gln Glu Cys Ser Tyr Phe Asn Ile Thr Lys
            660                 665                 670

Val Glu Ser Arg Asp Lys Leu Pro Gln Pro Val Gln Pro Asp Pro Val
        675                 680                 685

Ser His Cys Lys Glu Lys Asp Val Asp Asp Cys Trp Phe Tyr Phe Thr

690                 695                 700

Tyr Ser Val Asn Gly Asn Asn Glu Val Met Val His Val Val Glu Asn
705                 710                 715                 720

Pro Glu Cys Pro Thr Gly Pro Asp Ile Ile Pro Ile Val Ala Gly Val
                725                 730                 735

Val Ala Gly Ile Val Leu Ile Gly Leu Ala Leu Leu Leu Ile Trp Lys
            740                 745                 750

Leu Leu Met Ile Ile His Asp Arg Arg Glu Phe Ala Lys Phe Glu Lys
        755                 760                 765

Glu Lys Met Asn Ala Lys Trp Asp Thr Gly Glu Asn Pro Ile Tyr Lys
    770                 775                 780

Ser Ala Val Thr Thr Val Val Asn Pro Lys Tyr Glu Gly Lys
785                 790                 795

<210>  22
<211>  3879
<212>  DNA
<213>  homo sapiens

<400>  22
atcagacgcg cagaggaggc ggggccgcgg ctggtttcct gccggggggc ggctctgggc      60

cgccgagtcc cctcctcccg cccctgagga ggaggagccg ccgccacccg ccgcgcccga     120

cacccgggag gccccgccag cccgcgggag aggcccagcg ggagtcgcgg aacagcaggc     180

ccgagcccac cgcgccgggc cccggacgcc gcgcggaaaa gatgaattta caaccaattt     240

tctggattgg actgatcagt tcagtttgct gtgtgtttgc tcaaacagat gaaaatagat     300

gtttaaaagc aaatgccaaa tcatgtggag aatgtataca agcagggcca aattgtgggt     360

ggtgcacaaa ttcaacattt ttacaggaag gaatgcctac ttctgcacga tgtgatgatt     420

tagaagcctt aaaaaagaag ggttgccctc cagatgacat agaaaatccc agaggctcca     480

aagatataaa gaaaaataaa aatgtaacca accgtagcaa aggaacagca gagaagctca     540

agccagagga tattactcag atccaaccac agcagttggt tttgcgatta agatcagggg     600

agccacagac atttacatta aaattcaaga gagctgaaga ctatcccatt gacctctact     660

accttatgga cctgtcttac tcaatgaaag acgatttgga gaatgtaaaa agtcttggaa     720

cagatctgat gaatgaaatg aggaggatta cttcggactt cagaattgga tttggctcat     780

ttgtggaaaa gactgtgatg ccttacatta gcacaacacc agctaagctc aggaaccctt     840

gcacaagtga acagaactgc accagcccat ttagctacaa aaatgtgctc agtcttacta     900

ataaaggaga agtatttaat gaacttgttg gaaaacagcg catatctgga aatttggatt 960
ctccagaagg tggtttcgat gccatcatgc aagttgcagt ttgtggatca ctgattggct 1020
ggaggaatgt tacacggctg ctggtgtttt ccacagatgc cgggtttcac tttgctggag 1080
atgggaaact tggtggcatt gttttaccaa atgatggaca atgtcacctg gaaaataata 1140
tgtacacaat gagccattat tatgattatc cttctattgc tcaccttgtc cagaaactga 1200
gtgaaaataa tattcagaca atttttgcag ttactgaaga atttcagcct gtttacaagg 1260
agctgaaaaa cttgatccct aagtcagcag taggaacatt atctgcaaat tctagcaatg 1320
taattcagtt gatcattgat gcatacaatt ccctttcctc agaagtcatt ttggaaaacg 1380
gcaaattgtc agaaggcgta acaataagtt acaaatctta ctgcaagaac ggggtgaatg 1440
gaacagggga aaatggaaga aaatgttcca atatttccat tggagatgag gttcaatttg 1500
aaattagcat aacttcaaat aagtgtccaa aaaaggattc tgacagcttt aaaattaggc 1560
ctctgggctt tacggaggaa gtagaggtta ttcttcagta catctgtgaa tgtgaatgcc 1620
aaagcgaagg catccctgaa agtcccaagt gtcatgaagg aaatgggaca tttgagtgtg 1680
gcgcgtgcag gtgcaatgaa gggcgtgttg gtagacattg tgaatgcagc acagatgaag 1740
ttaacagtga agacatggat gcttactgca ggaaagaaaa cagttcagaa atctgcagta 1800
acaatggaga gtgcgtctgc ggacagtgtg tttgtaggaa gagggataat acaaatgaaa 1860
tttattctgg caaattctgc gagtgtgata atttcaactg tgatagatcc aatggcttaa 1920
tttgtggagg aaatggtgtt tgcaagtgtc gtgtgtgtga gtgcaacccc aactacactg 1980
gcagtgcatg tgactgttct ttggatacta gtacttgtga agccagcaac ggacagatct 2040
gcaatggccg gggcatctgc gagtgtggtg tctgtaagtg tacagatccg aagtttcaag 2100
ggcaaacgtg tgagatgtgt cagacctgcc ttggtgtctg tgctgagcat aaagaatgtg 2160
ttcagtgcag agccttcaat aaaggagaaa agaaagacac atgcacacag gaatgttcct 2220
attttaacat taccaaggta gaaagtcggg acaaattacc ccagccggtc caacctgatc 2280
ctgtgtccca ttgtaaggag aaggatgttg acgactgttg gttctatttt acgtattcag 2340
tgaatgggaa caacgaggtc atggttcatg ttgtggagaa tccagagtgt cccactggtc 2400
cagacatcat tccaattgta gctggtgtgg ttgctggaat tgttcttatt ggccttgcat 2460
tactgctgat atggaagctt ttaatgataa ttcatgacag aagggagttt gctaaatttg 2520
aaaaggagaa aatgaatgcc aaatgggaca cgggtgaaaa tcctatttat aagagtgccg 2580
taacaactgt ggtcaatccg aagtatgagg gaaaatgagt actgcccgtg caaatcccac 2640
aacactgaat gcaaagtagc aatttccata gtcacagtta ggtagcttta gggcaatatt 2700
gccatggttt tactcatgtg caggttttga aaatgtacaa tatgtataat ttttaaaatg 2760
ttttattatt ttgaaaataa tgttgtaatt catgccaggg actgacaaaa gacttgagac 2820

aggatggtta ctcttgtcag ctaaggtcac attgtgcctt tttgaccttt tcttcctgga 2880

ctattgaaat caagcttatt ggattaagtg atatttctat agcgattgaa agggcaatag 2940

ttaaagtaat gagcatgatg agagtttctg ttaatcatgt attaaaactg atttttagct 3000

ttacaaatat gtcagtttgc agttatgcag aatccaaagt aaatgtcctg ctagctagtt 3060

aaggattgtt ttaaatctgt tattttgcta tttgcctgtt agacatgact gatgacatat 3120

ctgaaagaca agtatgttga gagttgctgg tgtaaaatac gtttgaaata gttgatctac 3180

aaaggccatg ggaaaaattc agagagttag gaaggaaaaa ccaatagctt taaaacctgt 3240

gtgccatttt aagagttact taatgtttgg taacttttat gccttcactt tacaaattca 3300

agccttagat aaaagaaccg agcaattttc tgctaaaaag tccttgattt agcactattt 3360

acatacaggc catactttac aaagtatttg ctgaatgggg accttttgag ttgaatttat 3420

tttattattt ttattttgtt taatgtctgg tgctttctgt cacctcttct aatcttttaa 3480

tgtatttgtt tgcaattttg gggtaagact ttttttatga gtactttttc tttgaagttt 3540

tagcggtcaa tttgcctttt taatgaacat gtgaagttat actgtggcta tgcaacagct 3600

ctcacctacg cgagtcttac tttgagttag tgccataaca gaccactgta tgtttacttc 3660

tcaccatttg agttgcccat cttgtttcac actagtcaca ttcttgtttt aagtgccttt 3720

agttttaaca gttcactttt tacagtgcta tttactgaag ttatttatta aatatgccta 3780

aaatacttaa atcggatgtc ttgactctga tgtattttat caggttgtgt gcatgaaatt 3840

tttatagatt aaagaagttg aggaaaagca aaaaaaaaa 3879

<210> 23
<211> 1049
<212> PRT
<213> homo sapiens

<400> 23

Met Gly Ser Arg Thr Pro Glu Ser Pro Leu His Ala Val Gln Leu Arg
1               5                   10                  15

Trp Gly Pro Arg Arg Arg Pro Pro Leu Leu Pro Leu Leu Leu Leu Leu
            20                  25                  30

Leu Pro Pro Pro Pro Arg Val Gly Gly Phe Asn Leu Asp Ala Glu Ala
        35                  40                  45

Pro Ala Val Leu Ser Gly Pro Pro Gly Ser Phe Phe Gly Phe Ser Val
    50                  55                  60

Glu Phe Tyr Arg Pro Gly Thr Asp Gly Val Ser Val Leu Val Gly Ala
65                  70                  75                  80

Pro Lys Ala Asn Thr Ser Gln Pro Gly Val Leu Gln Gly Gly Ala Val
                85                  90                  95

Tyr Leu Cys Pro Trp Gly Ala Ser Pro Thr Gln Cys Thr Pro Ile Glu
            100                 105                 110

Phe Asp Ser Lys Gly Ser Arg Leu Leu Glu Ser Ser Leu Ser Ser Ser
        115                 120                 125

Glu Gly Glu Glu Pro Val Glu Tyr Lys Ser Leu Gln Trp Phe Gly Ala
    130                 135                 140

Thr Val Arg Ala His Gly Ser Ser Ile Leu Ala Cys Ala Pro Leu Tyr
145                 150                 155                 160

Ser Trp Arg Thr Glu Lys Glu Pro Leu Ser Asp Pro Val Gly Thr Cys
                165                 170                 175

Tyr Leu Ser Thr Asp Asn Phe Thr Arg Ile Leu Glu Tyr Ala Pro Cys
            180                 185                 190

Arg Ser Asp Phe Ser Trp Ala Ala Gly Gln Gly Tyr Cys Gln Gly Gly
        195                 200                 205

Phe Ser Ala Glu Phe Thr Lys Thr Gly Arg Val Val Leu Gly Gly Pro
    210                 215                 220

Gly Ser Tyr Phe Trp Gln Gly Gln Ile Leu Ser Ala Thr Gln Glu Gln
225                 230                 235                 240

Ile Ala Glu Ser Tyr Tyr Pro Glu Tyr Leu Ile Asn Leu Val Gln Gly
                245                 250                 255

Gln Leu Gln Thr Arg Gln Ala Ser Ser Ile Tyr Asp Asp Ser Tyr Leu
            260                 265                 270

Gly Tyr Ser Val Ala Val Gly Glu Phe Ser Gly Asp Asp Thr Glu Asp
        275                 280                 285

Phe Val Ala Gly Val Pro Lys Gly Asn Leu Thr Tyr Gly Tyr Val Thr
    290                 295                 300

Ile Leu Asn Gly Ser Asp Ile Arg Ser Leu Tyr Asn Phe Ser Gly Glu
305                 310                 315                 320

Gln Met Ala Ser Tyr Phe Gly Tyr Ala Val Ala Ala Thr Asp Val Asn

                325                 330                 335

Gly Asp Gly Leu Asp Asp Leu Leu Val Gly Ala Pro Leu Leu Met Asp
            340                 345                 350

Arg Thr Pro Asp Gly Arg Pro Gln Glu Val Gly Arg Val Tyr Val Tyr
        355                 360                 365

Leu Gln His Pro Ala Gly Ile Glu Pro Thr Pro Thr Leu Thr Leu Thr
    370                 375                 380

Gly His Asp Glu Phe Gly Arg Phe Gly Ser Ser Leu Thr Pro Leu Gly
385                 390                 395                 400

Asp Leu Asp Gln Asp Gly Tyr Asn Asp Val Ala Ile Gly Ala Pro Phe
                405                 410                 415

Gly Gly Glu Thr Gln Gln Gly Val Val Phe Val Phe Pro Gly Gly Pro
            420                 425                 430

Gly Gly Leu Gly Ser Lys Pro Ser Gln Val Leu Gln Pro Leu Trp Ala
        435                 440                 445

Ala Ser His Thr Pro Asp Phe Phe Gly Ser Ala Leu Arg Gly Gly Arg
    450                 455                 460

Asp Leu Asp Gly Asn Gly Tyr Pro Asp Leu Ile Val Gly Ser Phe Gly
465                 470                 475                 480

Val Asp Lys Ala Val Val Tyr Arg Gly Arg Pro Ile Val Ser Ala Ser
                485                 490                 495

Ala Ser Leu Thr Ile Phe Pro Ala Met Phe Asn Pro Glu Glu Arg Ser
            500                 505                 510

Cys Ser Leu Glu Gly Asn Pro Val Ala Cys Ile Asn Leu Ser Phe Cys
        515                 520                 525

Leu Asn Ala Ser Gly Lys His Val Ala Asp Ser Ile Gly Phe Thr Val
    530                 535                 540

Glu Leu Gln Leu Asp Trp Gln Lys Gln Lys Gly Gly Val Arg Arg Ala
545                 550                 555                 560

Leu Phe Leu Ala Ser Arg Gln Ala Thr Leu Thr Gln Thr Leu Leu Ile
                565                 570                 575

Gln Asn Gly Ala Arg Glu Asp Cys Arg Glu Met Lys Ile Tyr Leu Arg
            580                 585                 590

Asn Glu Ser Glu Phe Arg Asp Lys Leu Ser Pro Ile His Ile Ala Leu
        595                 600                 605

Asn Phe Ser Leu Asp Pro Gln Ala Pro Val Asp Ser His Gly Leu Arg
    610                 615                 620

Pro Ala Leu His Tyr Gln Ser Lys Ser Arg Ile Glu Asp Lys Ala Gln
625                 630                 635                 640

Ile Leu Leu Asp Cys Gly Glu Asp Asn Ile Cys Val Pro Asp Leu Gln
                645                 650                 655

Leu Glu Val Phe Gly Glu Gln Asn His Val Tyr Leu Gly Asp Lys Asn
            660                 665                 670

Ala Leu Asn Leu Thr Phe His Ala Gln Asn Val Gly Glu Gly Gly Ala
        675                 680                 685

Tyr Glu Ala Glu Leu Arg Val Thr Ala Pro Pro Glu Ala Glu Tyr Ser
    690                 695                 700

Gly Leu Val Arg His Pro Gly Asn Phe Ser Ser Leu Ser Cys Asp Tyr
705                 710                 715                 720

Phe Ala Val Asn Gln Ser Arg Leu Leu Val Cys Asp Leu Gly Asn Pro
                725                 730                 735

Met Lys Ala Gly Ala Ser Leu Trp Gly Gly Leu Arg Phe Thr Val Pro
            740                 745                 750

His Leu Arg Asp Thr Lys Lys Thr Ile Gln Phe Asp Phe Gln Ile Leu
        755                 760                 765

Ser Lys Asn Leu Asn Asn Ser Gln Ser Asp Val Val Ser Phe Arg Leu
    770                 775                 780

Ser Val Glu Ala Gln Ala Gln Val Thr Leu Asn Gly Val Ser Lys Pro
785                 790                 795                 800

Glu Ala Val Leu Phe Pro Val Ser Asp Trp His Pro Arg Asp Gln Pro
                805                 810                 815

Gln Lys Glu Glu Asp Leu Gly Pro Ala Val His His Val Tyr Glu Leu
            820                 825                 830

Ile Asn Gln Gly Pro Ser Ser Ile Ser Gln Gly Val Leu Glu Leu Ser
        835                 840                 845

Cys Pro Gln Ala Leu Glu Gly Gln Gln Leu Leu Tyr Val Thr Arg Val
    850                 855                 860

Thr Gly Leu Asn Cys Thr Thr Asn His Pro Ile Asn Pro Lys Gly Leu
865                 870                 875                 880

Glu Leu Asp Pro Glu Gly Ser Leu His His Gln Gln Lys Arg Glu Ala
                885                 890                 895

Pro Ser Arg Ser Ser Ala Ser Ser Gly Pro Gln Ile Leu Lys Cys Pro
            900                 905                 910

Glu Ala Glu Cys Phe Arg Leu Arg Cys Glu Leu Gly Pro Leu His Gln
        915                 920                 925

Gln Glu Ser Gln Ser Leu Gln Leu His Phe Arg Val Trp Ala Lys Thr
    930                 935                 940

Phe Leu Gln Arg Glu His Gln Pro Phe Ser Leu Gln Cys Glu Ala Val
945                 950                 955                 960

Tyr Lys Ala Leu Lys Met Pro Tyr Arg Ile Leu Pro Arg Gln Leu Pro
                965                 970                 975

Gln Lys Glu Arg Gln Val Ala Thr Ala Val Gln Trp Thr Lys Ala Glu
            980                 985                 990

Gly Ser Tyr Gly Val Pro Leu Trp Ile Ile Ile Leu Ala Ile Leu Phe
        995                 1000                1005

Gly Leu Leu Leu Leu Gly Leu Leu Ile Tyr Ile Leu Tyr Lys Leu
    1010                1015                1020

Gly Phe Phe Lys Arg Ser Leu Pro Tyr Gly Thr Ala Met Glu Lys
    1025                1030                1035

Ala Gln Leu Lys Pro Pro Ala Thr Ser Asp Ala
    1040                1045

<210> 24
<211> 4267
<212> DNA
<213> homo sapiens

<400> 24
   attcgcctct gggaggttta ggaagcggct ccgggtcggt ggccccagga cagggaagag 60
   cgggcgctat ggggagccgg acgccagagt cccctctcca cgccgtgcag ctgcgctggg 120
   gcccccggcg ccgacccccg ctgctgccgc tgctgttgct gctgctgccg ccgccaccca 180
   gggtcggggg cttcaactta gacgcggagg ccccagcagt actctcgggg cccccgggct 240
   ccttcttcgg attctcagtg gagttttacc ggccgggaac agacggggtc agtgtgctgg 300
   tgggagcacc caaggctaat accagccagc caggagtgct gcagggtggt gctgtctacc 360
   tctgtccttg gggtgccagc cccacacagt gcacccccat tgaatttgac agcaaaggct 420
   ctcggctcct ggagtcctca ctgtccagct cagagggaga ggagcctgtg gagtacaagt 480
   ccttgcagtg gttcggggca acagttcgag cccatggctc ctccatcttg gcatgcgctc 540
   cactgtacag ctggcgcaca gagaaggagc cactgagcga ccccgtgggc acctgctacc 600
   tctccacaga taacttcacc cgaattctgg agtatgcacc ctgccgctca gatttcagct 660
   gggcagcagg acagggttac tgccaaggag gcttcagtgc cgagttcacc aagactggcc 720
   gtgtggtttt aggtggacca ggaagctatt tctggcaagg ccagatcctg tctgccactc 780
   aggagcagat tgcagaatct tattaccccg agtacctgat caacctggtt caggggcagc 840
   tgcagactcg ccaggccagt tccatctatg atgacagcta cctaggatac tctgtggctg 900
   ttggtgaatt cagtggtgat gacacagaag actttgttgc tggtgtgccc aaagggaacc 960
   tcacttacgg ctatgtcacc atccttaatg gctcagacat tcgatccctc tacaacttct 1020
   caggggaaca gatggcctcc tactttggct atgcagtggc cgccacagac gtcaatgggg 1080
   acgggctgga tgacttgctg gtgggggcac ccctgctcat ggatcggacc cctgacgggc 1140
   ggcctcagga ggtgggcagg gtctacgtct acctgcagca cccagccggc atagagccca 1200
   cgcccaccct taccctcact ggccatgatg agtttggccg atttggcagc tccttgaccc 1260
   ccctggggga cctggaccag gatggctaca atgatgtggc catcggggct ccctttggtg 1320
   gggagaccca gcagggagta gtgtttgtat ttcctggggg cccaggaggg ctgggctcta 1380
   agccttccca ggttctgcag cccctgtggg cagccagcca caccccagac ttctttggct 1440
   ctgcccttcg aggaggccga gacctggatg gcaatggata tcctgatctg attgtggggt 1500
   cctttggtgt ggacaaggct gtggtataca ggggccgccc catcgtgtcc gctagtgcct 1560
   ccctcaccat cttccccgcc atgttcaacc cagaggagcg gagctgcagc ttagagggga 1620
   accctgtggc ctgcatcaac cttagcttct gcctcaatgc ttctggaaaa cacgttgctg 1680
   actccattgg tttcacagtg gaacttcagc tggactggca gaagcagaag ggaggggtac 1740
   ggcgggcact gttcctggcc tccaggcagg caaccctgac ccagaccctg ctcatccaga 1800
   atggggctcg agaggattgc agagagatga agatctacct caggaacgag tcagaatttc 1860
   gagacaaact ctcgccgatt cacatcgctc tcaacttctc cttggacccc caagccccag 1920
   tggacagcca cggcctcagg ccagccctac attatcagag caagagccgg atagaggaca 1980
   aggctcagat cttgctggac tgtggagaag acaacatctg tgtgcctgac ctgcagctgg 2040
   aagtgtttgg ggagcagaac catgtgtacc tgggtgacaa gaatgccctg aacctcactt 2100
   tccatgccca gaatgtgggt gagggtggcg cctatgaggc tgagcttcgg gtcaccgccc 2160
   ctccagaggc tgagtactca ggactcgtca gacacccagg gaacttctcc agcctgagct 2220
   gtgactactt tgccgtgaac cagagccgcc tgctggtgtg tgacctgggc aaccccatga 2280
   aggcaggagc cagtctgtgg ggtggccttc ggtttacagt ccctcatctc cgggacacta 2340
   agaaaaccat ccagtttgac ttccagatcc tcagcaagaa tctcaacaac tcgcaaagcg 2400
   acgtggtttc ctttcggctc tccgtggagg ctcaggccca ggtcaccctg aacggtgtct 2460
   ccaagcctga ggcagtgcta ttcccagtaa gcgactggca tccccgagac cagcctcaga 2520
   aggaggagga cctgggacct gctgtccacc atgtctatga gctcatcaac caaggcccca 2580
   gctccattag ccagggtgtg ctggaactca gctgtcccca ggctctggaa ggtcagcagc 2640
   tcctatatgt gaccagagtt acgggactca actgcaccac caatcacccc attaacccaa 2700
   agggcctgga gttggatccc gagggttccc tgcaccacca gcaaaaacgg gaagctccaa 2760
   gccgcagctc tgcttcctcg ggacctcaga tcctgaaatg cccggaggct gagtgtttca 2820
   ggctgcgctg tgagctcggg cccctgcacc aacaagagag ccaaagtctg cagttgcatt 2880
   tccgagtctg ggccaagact ttcttgcagc gggagcacca gccatttagc ctgcagtgtg 2940
   aggctgtgta caaagccctg aagatgccct accgaatcct gcctcggcag ctgccccaaa 3000
   aagagcgtca ggtggccaca gctgtgcaat ggaccaaggc agaaggcagc tatggcgtcc 3060
   cactgtggat catcatccta gccatcctgt ttggcctcct gctcctaggt ctactcatct 3120
   acatcctcta caagcttgga ttcttcaaac gctccctccc atatggcacc gccatggaaa 3180
   aagctcagct caagcctcca gccacctctg atgcctgagt cctcccaatt tcagactccc 3240
   attcctgaag aaccagtccc cccaccctca ttctactgaa aaggaggggt ctgggtactt 3300
   cttgaaggtg ctgacggcca gggagaagct cctctcccca gcccagagac atacttgaag 3360
   ggccagagcc aggggggtga ggagctgggg atccctcccc cccatgcact gtgaaggacc 3420
   cttgtttaca catacctctt tcatggatgg gggaactcag atccagggac agaggcccca 3480
   gcctccctga agcctttgca ttttggagag tttcctgaaa caacttggaa agataactag 3540
   gaaatccatt cacagttctt tgggccagac atgccacaag gacttcctgt ccagctccaa 3600
   cctgcaaaga tctgtcctca gccttgccag agatccaaaa gaagccccca gctaagaacc 3660
   tggaacttgg ggagttaaga cctggcagct ctggacagcc ccaccctggt gggccaacaa 3720
   agaacactaa ctatgcatgg tgccccagga ccagctcagg acagatgcca cacaaggata 3780
   gatgctggcc cagggcccag agcccagctc caaggggaat cagaactcaa atggggccag     3840
   atccagcctg gggtctggag ttgatctgga acccagactc agacattggc acctaatcca     3900
   ggcagatcca ggactatatt tgggcctgct ccagacctga tcctggaggc ccagttcacc     3960
   ctgatttagg agaagccagg aatttcccag gaccctgaag gggccatgat ggcaacagat     4020
   ctggaacctc agcctggcca gacacaggcc ctccctgttc cccagagaaa ggggagccca     4080
   ctgtcctggg cctgcagaat ttgggttctg cctgccagct gcactgatgc tgcccctcat     4140
   ctctctgccc aacccttccc tcaccttggc accagacacc caggacttat ttaaactctg     4200
   ttgcaagtgc aataaatctg acccagtgcc cccactgacc agaactagaa aaaaaaaaaa     4260
   aaaaaaa                                                              4267

<210>  25
<211>  314
<212>  PRT
<213>  homo sapiens

<400>  25

Met Arg Ile Ala Val Ile Cys Phe Cys Leu Leu Gly Ile Thr Cys Ala
1               5                   10                  15

Ile Pro Val Lys Gln Ala Asp Ser Gly Ser Ser Glu Glu Lys Gln Leu
            20                  25                  30

Tyr Asn Lys Tyr Pro Asp Ala Val Ala Thr Trp Leu Asn Pro Asp Pro
        35                  40                  45

Ser Gln Lys Gln Asn Leu Leu Ala Pro Gln Asn Ala Val Ser Ser Glu
    50                  55                  60

Glu Thr Asn Asp Phe Lys Gln Glu Thr Leu Pro Ser Lys Ser Asn Glu
65                  70                  75                  80

Ser His Asp His Met Asp Asp Met Asp Asp Glu Asp Asp Asp Asp His
                85                  90                  95

Val Asp Ser Gln Asp Ser Ile Asp Ser Asn Asp Ser Asp Asp Val Asp
            100                 105                 110

Asp Thr Asp Asp Ser His Gln Ser Asp Glu Ser His His Ser Asp Glu
        115                 120                 125

Ser Asp Glu Leu Val Thr Asp Phe Pro Thr Asp Leu Pro Ala Thr Glu
    130                 135                 140

Val Phe Thr Pro Val Val Pro Thr Val Asp Thr Tyr Asp Gly Arg Gly

145                 150                 155                 160

Asp Ser Val Val Tyr Gly Leu Arg Ser Lys Ser Lys Lys Phe Arg Arg
                165                 170                 175

Pro Asp Ile Gln Tyr Pro Asp Ala Thr Asp Glu Asp Ile Thr Ser His
            180                 185                 190

Met Glu Ser Glu Glu Leu Asn Gly Ala Tyr Lys Ala Ile Pro Val Ala
        195                 200                 205

Gln Asp Leu Asn Ala Pro Ser Asp Trp Asp Ser Arg Gly Lys Asp Ser
    210                 215                 220

Tyr Glu Thr Ser Gln Leu Asp Asp Gln Ser Ala Glu Thr His Ser His
225                 230                 235                 240

Lys Gln Ser Arg Leu Tyr Lys Arg Lys Ala Asn Asp Glu Ser Asn Glu
                245                 250                 255

His Ser Asp Val Ile Asp Ser Gln Glu Leu Ser Lys Val Ser Arg Glu
            260                 265                 270

Phe His Ser His Glu Phe His Ser His Glu Asp Met Leu Val Val Asp
        275                 280                 285

Pro Lys Ser Lys Glu Glu Asp Lys His Leu Lys Phe Arg Ile Ser His
    290                 295                 300

Glu Leu Asp Ser Ala Ser Ser Glu Val Asn
305                 310

<210>  26
<211>  1641
<212>  DNA
<213>  homo sapiens

<400>  26
ctccctgtgt tggtggagga tgtctgcagc agcatttaaa ttctgggagg gcttggttgt      60

cagcagcagc aggaggaggc agagcacagc atcgtcggga ccagactcgt ctcaggccag     120

ttgcagcctt ctcagccaaa cgccgaccaa ggaaaactca ctaccatgag aattgcagtg     180

atttgctttt gcctcctagg catcacctgt gccataccag ttaaacaggc tgattctgga     240

agttctgagg aaaagcagct ttacaacaaa tacccagatg ctgtggccac atggctaaac     300

cctgacccat ctcagaagca gaatctccta gccccacaga atgctgtgtc ctctgaagaa     360

accaatgact ttaaacaaga gacccttcca agtaagtcca acgaaagcca tgaccacatg     420

gatgatatgg atgatgaaga tgatgatgac catgtggaca gccaggactc cattgactcg 480

aacgactctg atgatgtaga tgacactgat gattctcacc agtctgatga gtctcaccat 540

tctgatgaat ctgatgaact ggtcactgat tttcccacgg acctgccagc aaccgaagtt 600

ttcactccag ttgtccccac agtagacaca tatgatggcc gaggtgatag tgtggtttat 660

ggactgaggt caaaatctaa gaagtttcgc agacctgaca tccagtaccc tgatgctaca 720

gacgaggaca tcacctcaca catggaaagc gaggagttga atggtgcata caaggccatc 780

cccgttgccc aggacctgaa cgcgccttct gattgggaca gccgtgggaa ggacagttat 840

gaaacgagtc agctggatga ccagagtgct gaaacccaca gccacaagca gtccagatta 900

tataagcgga aagccaatga tgagagcaat gagcattccg atgtgattga tagtcaggaa 960

ctttccaaag tcagccgtga attccacagc catgaatttc acagccatga agatatgctg 1020

gttgtagacc ccaaaagtaa ggaagaagat aaacacctga aatttcgtat ttctcatgaa 1080

ttagatagtg catcttctga ggtcaattaa aaggagaaaa aatacaattt ctcactttgc 1140

atttagtcaa aagaaaaaat gctttatagc aaaatgaaag agaacatgaa atgcttcttt 1200

ctcagtttat tggttgaatg tgtatctatt tgagtctgga aataactaat gtgtttgata 1260

attagtttag tttgtggctt catggaaact ccctgtaaac taaaagcttc agggttatgt 1320

ctatgttcat tctatagaag aaatgcaaac tatcactgta ttttaatatt tgttattctc 1380

tcatgaatag aaatttatgt agaagcaaac aaaatacttt tacccactta aaaagagaat 1440

ataacatttt atgtcactat aatcttttgt tttttaagtt agtgtatatt ttgttgtgat 1500

tatctttttg tggtgtgaat aaatctttta tcttgaatgt aataagaatt tggtggtgtc 1560

aattgcttat ttgttttccc acggttgtcc agcaattaat aaaacataac cttttttact 1620

gcctaaaaaa aaaaaaaaaa a 1641